# EUROPEAN PATENT APPLICATION

(11) **EP 4 410 842 A1**
(43) Date of publication of application: **07.08.2024**
(21) Application number: 22882927.1
(22) Date of filing: 20.10.2022
(51) Int. Cl.: C07K 16/46, C12N 15/62, A61K 39/395, A61P 35/00

(54) **FUSION POLYPEPTIDE AND USE THEREOF**

(30) Priority: 21.10.2021 CN 202111225215
(71) Applicant: Adlai Nortye Biopharma Co., Ltd., Hangzhou, Zhejiang 311100 (CN)
(72) Inventor: HE, Nanhai, Hangzhou, Zhejiang 311100 (CN); WANG, Youping, Hangzhou, Zhejiang 311100 (CN); YANG, Wenjing, Hangzhou, Zhejiang 311100 (CN); CHEN, Wanwan, Hangzhou, Zhejiang 311100 (CN)
(74) Representative: Office Freylinger
(86) International application number: PCT/CN2022/126301
(87) International publication number: WO 2023/066322

(57) **Abstract**

The present application relates to a fusion polypeptide and its use. Specifically, the fusion polypeptide of the present application can block PD-1/PD-L1 signaling and/or can have the function of activating an immune response. The application also provides the use of the polypeptide.

## Description

### Technical field

This application relates to the field of biomedicine, in particular to a fusion polypeptide and its use.

### Background

Immune checkpoints are a class of immune-related molecules with inhibitory signaling molecules, which mainly realize their functions by regulating the immune response in peripheral tissues, including participating in immune defense, immune tolerance and immune tissue damage, etc., and targeting immune checkpoints related treatment method is to regulate the immune response, including the innate immune response and the adaptive immune response, to achieve the intervention of the immune system and achieve the purpose of treating the disease. Tumor cells can use immune checkpoint inhibitory signaling pathways to achieve their role in immune evasion. The binding of programmed death receptor-1 (programmed death-1 (PD-1)) to its ligand programmed death receptor protein ligand-1 (PD-L1) is an important event in the suppression of anti-tumor immune responses, and PD -L1 is expressed in a variety of tumor cells, including colon cancer, lung cancer, ovarian cancer and a variety of myeloma, and the expression of PD-L1 has an important relationship with the prognosis of various cancers. The binding of PD-L1 and PD-1 can lead to T cell apoptosis, immune signal suppression, cell depletion and secretion of immunosuppressive factors and other effects, so T cells involved in tumor infiltration function ineffective response, and then assist tumor cells to evade the surveillance of the immune system.

At present, anti-PD-L1 blocking antibodies on the market include Atezolizumab developed by Roche, Avelumab jointly developed by Merck KGaA/Pfizer, and Durvalumab developed by AstraZeneca; The blocking antibodies targeting PD-1 on the market include Nivolumab developed by Bristol-Myers Squibb (BMS), Permbrolizumab developed by Merck, Camrelizumab developed by Hengrui Medicine, and Tislelizumab developed by BeiGene and Sintilimab developed by Innovent Biologics; although these antibodies have shown the effect of tumor treatment, their average treatment effective rate is only about 20%, and a considerable number of tumor patients do not respond to reatment with anti-PD-L1 antibody and anti-PD-1 antibody. Therefore, enhancing the effectiveness of tumor therapy is still a major problem that needs to be solved urgently in tumor therapy.

### Contents of the invention

Due to the current limitation of the effectiveness of PD-1/PD-L1 pathway blockade in regulating T cell activation to treat tumors, this application proposes the development of new agents that can synergize with PD-1/PD-L1 pathway blockade to enhance T cell activation treatment options for clinical oncology. The development of new agents that can synergistically block PD-1/PD-L1 pathway, enhance T cell activation, and/or improve antigen-presenting cell activation for the clinical treatment of tumors has the potential to bring more benefits to more tumor patients.

The application provides a fusion polypeptide, the fusion polypeptide of the application may comprise (i) an antibody-associated antigen binding fragment derived from an anti-programmed death receptor protein ligand-1 (programmed death-1 (PD-L1)) antibody/anti-programmed death receptor protein-1 (programmed death-1 (PD-1)), or/and (ii) immunoglobulin Fc domain, or/and (iii) fusion polypeptides of CD80 extracellular domain (ECD), or/and (iv) fusion polypeptides composed of LAG3 extracellular domains, and the application provides polynucleotides for expressing the fusion polypeptides; the application provides methods of using the fusion polypeptides for inducing and/or enhancing immunity and methods of treating diseases such as cancer.

The application provides a class of dual and/or multifunctional drugs that simultaneously interfere with, inhibit or block the PD-1/PD-L1 signal transduction pathway and/or co-stimulate the activation of antigen-presenting cells and/or co-stimulate the activation of T cells. The fusion polypeptide can effectively stimulate T cells to enhance immune response and/or stimulate antigen-presenting cells, and can have a potential tumor-killing effect, so it can be used in the treatment of diseases such as tumors caused by the suppression of T cell functions, especially in patients with tumors that are unresponsive or weakly responsive to anti-PD-1 antibodies and/or anti-PD-L1 antibodies to improve efficacy.

In one aspect, the application provides a fusion polypeptide, the fusion polypeptide comprises a first domain and a second domain, the first domain comprises HCDR3 of an antibody heavy chain, and the antibody heavy chain comprises the amino acid sequence shown in SEQ ID NO: 121, the second domain can activate the immune response.

In one embodiment of the fusion polypeptide, the first domain comprises HCDR2 of an antibody heavy chain, and the antibody heavy chain comprises the amino acid sequence shown in SEQ ID NO: 121.

In one embodiment of the fusion polypeptide, the first domain comprises HCDR1 of an antibody heavy chain, and the antibody heavy chain comprises the amino acid sequence shown in SEQ ID NO: 121.

In one embodiment of the fusion polypeptide, the first domain comprises the heavy chain variable region VH of an antibody heavy chain, and the antibody heavy chain comprises the amino acid sequence shown in SEQ ID NO: 121.

In one embodiment of the fusion polypeptide, the first domain comprises a heavy chain, and the heavy chain comprises the amino acid sequence shown in SEQ ID NO: 121.

In one embodiment of the fusion polypeptide, the first domain comprises LCDR3 of an antibody light chain, and the antibody light chain comprises the amino acid sequence shown in SEQ ID NO: 122.

In one embodiment of the fusion polypeptide, the first domain comprises LCDR2 of an antibody light chain, and the antibody light chain comprises the amino acid sequence shown in SEQ ID NO: 122.

In one embodiment of the fusion polypeptide, the first domain comprises LCDR1 of an antibody light chain, and the antibody light chain comprises the amino acid sequence shown in SEQ ID NO: 122.

In one embodiment of the fusion polypeptide, the first domain comprises the light chain variable region VL of the antibody light chain, and the antibody light chain comprises the amino acid sequence shown in SEQ ID NO: 122.

In one embodiment of the fusion polypeptide, the first domain comprises a light chain comprising the amino acid sequence shown in SEQ ID NO: 122.

In one embodiment of the fusion polypeptide, the first domain comprises an antibody or antigen-binding fragment thereof.

In one embodiment of the fusion polypeptide, the antibody is selected from the group consisting of recombinant antibodies, single domain antibodies, heavy chain antibodies, chimeric antibodies and bispecific antibodies.

In one embodiment of the fusion polypeptide, the antigen-binding fragment is selected from one or more of the group consisting of Fab, Fab', Fv fragment, F(ab')2, F(ab)2, scFv, di - scFv, VHH and dAb.

In one embodiment of the fusion polypeptide, the second domain is selected from the group consisting of CD80 or a functionally active fragment thereof, and LAG3 or a functionally active fragment thereof.

In one embodiment of the fusion polypeptide, the second domain is selected from the group consisting of human-derived CD80 or its functionally active fragment, mouse-derived CD80 or its functionally active fragment, human-derived LAG3 or its functionally active fragment, and mouse-derived LAG3 or functionally active fragments thereof.

In one embodiment of the fusion polypeptide, the second domain is capable of binding to CD28.

In one embodiment of the fusion polypeptide, the second domain comprises the extracellular domain of CD80 or a functionally active fragment thereof.

In one embodiment of the fusion polypeptide, the second domain comprises the amino acid sequence shown in SEQ ID NO:2.

In one embodiment of the fusion polypeptide, the second domain is capable of **binding** MHCII molecules on antigen-presenting cells.

In one embodiment of the fusion polypeptide, the second domain comprises the extracellular domain of LAG3 or a functionally active fragment thereof.

In one embodiment of the fusion polypeptide, the second domain comprises the amino acid sequence shown in SEQ ID NO: 11.

In one embodiment of the fusion polypeptide, the second domain comprises IgD1, IgD2, IgD3 and/or IgD4 of LAG3 or a functionally active fragment thereof.

In one embodiment of the fusion polypeptide, the second domain comprises an amino acid sequence selected from the following group consisting of SEQ ID NO: 13, SEQ ID NO: 14, SEQ ID NO: 15, SEQ ID NO: 16, SEQ ID NO:17, SEQ ID NO:18, SEQ ID NO:19, and SEQ ID NO: 12.

In one embodiment of the fusion polypeptide, the first domain is directly or indirectly linked to the second domain.

In one embodiment of the fusion polypeptide, the first domain comprises an antibody heavy chain, and the antibody heavy chain of the first domain is directly or indirectly linked to the second domain.

In one embodiment of the fusion polypeptide, the first domain comprises an antibody light chain, and the antibody light chain of the first domain is directly or indirectly linked to the second domain.

In one embodiment of the fusion polypeptide, the first domain is directly or indirectly connected to the N-terminus of the second domain.

In one embodiment of the fusion polypeptide, the first domain is directly or indirectly linked to the C-terminus of the second domain.

In one embodiment of the fusion polypeptide, the indirect link comprises link via a linker.

In one embodiment of the fusion polypeptide, the linker comprises an amino acid sequence selected from the group consisting of: SEQ ID NO: 22, SEQ ID NO: 23, SEQ ID NO: 24, SEQ ID NO: 25, and SEQ ID NO: 26.

In one embodiment of the fusion polypeptide, the fusion polypeptide comprises an amino acid sequence selected from the following groups: SEQ ID NO: 27-42, SEQ ID NO: 130, SEQ ID NO: 132, SEQ ID NO: 135, SEQ ID NO: 137, SEQ ID NO: 139, SEQ ID NO: 147, SEQ ID NO: 149, SEQ ID NO: 153, SEQ ID NO: 155, SEQ ID NO: 159, SEQ ID NO: 161, SEQ ID NO: 168, SEQ ID NO: 170, SEQ ID NO: 172, SEQ ID NO: 174, and SEQ ID NO: 176.

In another aspect, the present application provides a fusion polypeptide comprising a first domain and a second domain, the first domain can block PD-1/PD-L1 signaling, and the second domain comprises LAG3 or a functionally active fragment thereof.

In one embodiment of the fusion polypeptide, the first domain comprises HCDR3 of an antibody heavy chain, and the antibody heavy chain comprises an amino acid sequence selected from the group consisting of: SEQ ID NO: 3-9, and SEQ ID NO :121.

In one embodiment of the fusion polypeptide, the first domain comprises HCDR2 of an antibody heavy chain, and the antibody heavy chain comprises an amino acid sequence selected from the group consisting of: SEQ ID NO: 3-9, and SEQ ID NO :121.

In one embodiment of the fusion polypeptide, the first domain comprises HCDR1 of an antibody heavy chain, and the antibody heavy chain comprises an amino acid sequence selected from the group consisting of: SEQ ID NO: 3-9, and SEQ ID NO :121.

In one embodiment of the fusion polypeptide, the first domain comprises the heavy chain variable region VH of an antibody heavy chain, and the antibody heavy chain comprises an amino acid sequence selected from the group consisting of: SEQ ID NO: 3-9 , and SEQ ID NO:121.

In one embodiment of the fusion polypeptide, the first domain comprises an antibody heavy chain, and the antibody heavy chain comprises an amino acid sequence selected from the group consisting of: SEQ ID NO: 3-9, and SEQ ID NO: 121.

In one embodiment of the fusion polypeptide, the first domain comprises LCDR3 of an antibody light chain, and the antibody light chain comprises an amino acid sequence selected from the group consisting of: SEQ ID NO: 162-167, and SEQ ID NO : 122.

In one embodiment of the fusion polypeptide, the first domain comprises LCDR2 of an antibody light chain, and the antibody light chain comprises an amino acid sequence selected from the group consisting of: SEQ ID NO: 162-167, and SEQ ID NO : 122.

In one embodiment of the fusion polypeptide, the first domain comprises LCDR1 of an antibody light chain, and the antibody light chain comprises an amino acid sequence selected from the group consisting of: SEQ ID NO: 162-167, and SEQ ID NO : 122.

In one embodiment of the fusion polypeptide, the first domain comprises the light chain variable region VL of an antibody light chain, and the antibody light chain comprises an amino acid sequence selected from the group consisting of SEQ ID NO: 162-167, and SEQ ID NO: 122.

In one embodiment of the fusion polypeptide, the first domain comprises an antibody light chain, and the antibody light chain comprises an amino acid sequence selected from the group consisting of: SEQ ID NO: 162-167, and SEQ ID NO: 122.

In one embodiment of the fusion polypeptide, the first domain can bind to PD-L1 and/or PD-1.

In one embodiment of the fusion polypeptide, the first domain comprises an antibody or antigen-binding fragment thereof.

In one embodiment of the fusion polypeptide, the antibody is selected from the group consisting of recombinant antibodies, single domain antibodies, heavy chain antibodies, chimeric antibodies and bispecific antibodies.

In one embodiment of the fusion polypeptide, the antigen-binding fragment is selected from one or more of the group consisting of: Fab, Fab', Fv fragment, F(ab')2, F(ab)2, scFv, di - scFv, VHH and dAb.

In one embodiment of the fusion polypeptide, the second domain is selected from the group consisting of human-derived LAG3 or a functionally active fragment thereof and mouse-derived LAG3 or a functionally active fragment thereof.

In one embodiment of the fusion polypeptide, the second domain is capable of **binding** MHCII molecules on antigen-presenting cells.

In one embodiment of the fusion polypeptide, the second domain comprises the extracellular domain of LAG3 or a functionally active fragment thereof.

In one embodiment of the fusion polypeptide, the second domain comprises the amino acid sequence shown in SEQ ID NO: 11.

In one embodiment of the fusion polypeptide, the second domain comprises IgD1, IgD2, IgD3 and/or IgD4 of LAG3 or a functionally active fragment thereof.

In one embodiment of the fusion polypeptide, the second domain comprises an amino acid sequence selected from the following group: SEQ ID NO: 13, SEQ ID NO: 14, SEQ ID NO: 15, SEQ ID NO: 16, SEQ ID NO: 17, SEQ ID NO: 18, SEQ ID NO: 19, and SEQ ID NO: 12.

In one embodiment of the fusion polypeptide, the first domain is directly or indirectly linked to the second domain.

In one embodiment of the fusion polypeptide, the first domain comprises an antibody heavy chain, and the antibody heavy chain of the first domain is directly or indirectly linked to the second domain.

In one embodiment of the fusion polypeptide, the first domain comprises an antibody light chain, and the antibody light chain of the first domain is directly or indirectly linked to the second domain.

In one embodiment of the fusion polypeptide, the first domain is directly or indirectly connected to the N-terminus of the second domain.

In one embodiment of the fusion polypeptide, the first domain is directly or indirectly linked to the C-terminus of the second domain.

In one embodiment of the fusion polypeptide, the indirect link comprises link through a linker.

In one embodiment of the fusion polypeptide, the linker comprises an amino acid sequence selected from the group consisting of: SEQ ID NO: 22, SEQ ID NO: 23, SEQ ID NO: 24, SEQ ID NO: 25, and SEQ ID NO: 26.

In one embodiment of the fusion polypeptide, the fusion polypeptide comprises an amino acid sequence selected from the group consisting of: SEQ ID NO: 28-30, SEQ ID NO: 32, SEQ ID NO: 34-42, SEQ ID NO: 44-46, SEQ ID NO: 48, SEQ ID NO: 50-58, SEQ ID NO: 60-62, SEQ ID NO: 64, SEQ ID NO: 66-74, SEQ ID NO: 76-78, SEQ ID NO: 80, SEQ ID NO: 82-90, SEQ ID NO: 92-94, SEQ ID NO: 96-104, SEQ ID NO: 106-108, SEQ ID NO: 110, SEQ ID NO: 112-120, SEQ ID NO: 135, SEQ ID NO: 137, SEQ ID NO: 139, SEQ ID NO: 147, SEQ ID NO: 149, SEQ ID NO: 153, SEQ ID NO: 155, SEQ ID NO: 161, SEQ ID NO: 168, SEQ ID NO: 170, SEQ ID NO: 172, SEQ ID NO: 174, and SEQ ID NO: 176.

In another aspect, the present application provides a fusion polypeptide comprising a first domain, a second domain and a third domain, and the first domain can block PD-1/PD-L1 signaling, the second domain comprises LAG3 or a functionally active fragment thereof, and the third domain is capable of activating an immune response.

In one embodiment of the fusion polypeptide, the first domain comprises HCDR3 of an antibody heavy chain, and the antibody heavy chain comprises an amino acid sequence selected from the group consisting of: SEQ ID NO: 3-9, and SEQ ID NO :121.

In one embodiment of the fusion polypeptide, the first domain comprises HCDR2 of an antibody heavy chain, and the antibody heavy chain comprises an amino acid sequence selected from the group consisting of: SEQ ID NO: 3-9, and SEQ ID NO :121.

In one embodiment of the fusion polypeptide, the first domain comprises HCDR1 of an antibody heavy chain, and the antibody heavy chain comprises an amino acid sequence selected from the group consisting of: SEQ ID NO: 3-9, and SEQ ID NO :121.

In one embodiment of the fusion polypeptide, the first domain comprises the heavy chain variable region VH of an antibody heavy chain, and the antibody heavy chain comprises an amino acid sequence selected from the group consisting of: SEQ ID NO: 3-9, and SEQ ID NO:121.

In one embodiment of the fusion polypeptide, the first domain comprises an antibody heavy chain, and the antibody heavy chain comprises an amino acid sequence selected from the group consisting of: SEQ ID NO: 3-9, and SEQ ID NO: 121.

In one embodiment of the fusion polypeptide, the first domain comprises LCDR3 of an antibody light chain, and the antibody light chain comprises an amino acid sequence selected from the group consisting of: SEQ ID NO: 162-167, and SEQ ID NO : 122.

In one embodiment of the fusion polypeptide, the first domain comprises LCDR2 of an antibody light chain, and the antibody light chain comprises an amino acid sequence selected from the group consisting of: SEQ ID NO: 162-167, and SEQ ID NO : 122.

In one embodiment of the fusion polypeptide, the first domain comprises LCDR1 of an antibody light chain, and the antibody light chain comprises an amino acid sequence selected from the group consisting of: SEQ ID NO: 162-167, and SEQ ID NO :122.

In one embodiment of the fusion polypeptide, the first domain comprises the light chain variable region VL of an antibody light chain, and the antibody light chain comprises an amino acid sequence selected from the group consisting of: SEQ ID NO: 162-167, and SEQ ID NO: 122.

In one embodiment of the fusion polypeptide, the first domain comprises an antibody light chain, and the antibody light chain comprises an amino acid sequence selected from the group consisting of: SEQ ID NO: 162-167, and SEQ ID NO: 122.

In one embodiment of the fusion polypeptide, the first domain can bind to PD-L1 and/or PD-1.

In one embodiment of the fusion polypeptide, said first domain comprises an antibody or antigen-binding fragment thereof.

In one embodiment of the fusion polypeptide, the antibody is selected from the group consisting of recombinant antibodies, single domain antibodies, heavy chain antibodies, chimeric antibodies and bispecific antibodies.

In one embodiment of the fusion polypeptide, the antigen-binding fragment is selected from one or more of the group consisting of: Fab, Fab', Fv fragment, F(ab')2, F(ab)2, scFv, di - scFv, VHH and dAb.

In one embodiment of the fusion polypeptide, the second domain is selected from the group consisting of human-derived LAG3 or a functionally active fragment thereof and mouse-derived LAG3 or a functionally active fragment thereof.

In one embodiment of the fusion polypeptide, the second domain is capable of **binding** MHCII molecules on antigen-presenting cells.

In one embodiment of the fusion polypeptide, the second domain comprises the extracellular domain of LAG3 or a functionally active fragment thereof.

In one embodiment of the fusion polypeptide, the second domain comprises the amino acid sequence shown in SEQ ID NO: 11.

In one embodiment of the fusion polypeptide, the second domain comprises IgD1, IgD2, IgD3 and/or IgD4 of LAG3 or a functionally active fragment thereof.

In one embodiment of the fusion polypeptide, the second domain comprises an amino acid sequence selected from **the following group:** SEQ ID NO: 13, SEQ ID NO: 14, SEQ ID NO: 15, SEQ ID NO: 16, SEQ ID NO: 17, SEQ ID NO: 18, SEQ ID NO: 19, and SEQ ID NO: 12.

In one embodiment of the fusion polypeptide, the third domain comprises CD80 or a functionally active fragment thereof.

In one embodiment of the fusion polypeptide, the third domain is selected from the group consisting of human-derived CD80 or a functionally active fragment thereof and mouse-derived CD80 or a functionally active fragment thereof.

In one embodiment of the fusion polypeptide, the third domain is capable of **binding** CD28.

In one embodiment of the fusion polypeptide, the third domain comprises the extracellular domain of CD80 or a functionally active fragment thereof.

In one embodiment of the fusion polypeptide, the third domain comprises the amino acid sequence shown in SEQ ID NO:2.

In one embodiment of the fusion polypeptide, the first domain is directly or indirectly linked to the second domain.

In one embodiment of the fusion polypeptide, the first domain comprises an antibody heavy chain, and the antibody heavy chain of the first domain is directly or indirectly linked to the second domain.

In one embodiment of the fusion polypeptide, the first domain comprises an antibody light chain, and the antibody light chain of the first domain is directly or indirectly linked to the second domain.

In one embodiment of the fusion polypeptide, the first domain is directly or indirectly linked to the third domain.

In one embodiment of the fusion polypeptide, the first domain comprises an antibody heavy chain, and the antibody heavy chain of the first domain is directly or indirectly linked to the third domain.

In one embodiment of the fusion polypeptide, the first domain comprises an antibody light chain, and the antibody light chain of the first domain is directly or indirectly linked to the third domain.

In one embodiment of the fusion polypeptide, the second domain is directly or indirectly linked to the third domain.

In one embodiment of the fusion polypeptide, the first domain is directly or indirectly connected to the N-terminus of the second domain.

In one embodiment of the fusion polypeptide, the first domain is directly or indirectly linked to the C-terminus of the second domain.

In one embodiment of the fusion polypeptide, the first domain is directly or indirectly linked to the N-terminus of the third domain.

In one embodiment of the fusion polypeptide, the first domain is directly or indirectly linked to the C-terminus of the third domain.

In one embodiment of the fusion polypeptide, the second domain is directly or indirectly linked to the N-terminus of the third domain.

In one embodiment of the fusion polypeptide, the second domain is directly or indirectly linked to the C-terminus of the third domain.

In one embodiment of the fusion polypeptide, the first domain is directly or indirectly linked to the N-terminus of the second domain, and the second domain is directly or indirectly linked to the N-terminus of the third domain.

In one embodiment of the fusion polypeptide, the first domain is directly or indirectly linked to the N-terminus of the third domain, and the third domain is directly or indirectly linked to the N-terminus of the second domain.

In one embodiment of the fusion polypeptide, the first domain is directly or indirectly linked to the C-terminal of the second domain, and the first domain is directly or indirectly linked to the N-terminal of the third domain.

In one embodiment of the fusion polypeptide, the first domain is directly or indirectly linked to the C-terminal of the third domain, and the first domain is directly or indirectly linked to the N-terminal of the second domain.

In one embodiment of the fusion polypeptide, the indirect link comprises link via a linker.

In one embodiment of the fusion polypeptide, the linker comprises an amino acid sequence selected from the group consisting of: SEQ ID NO: 22, SEQ ID NO: 23, SEQ ID NO: 24, SEQ ID NO: 25, and SEQ ID NO: 26.

In one embodiment of the fusion polypeptide, the fusion polypeptide comprises an amino acid sequence selected from the group consisiting of: SEQ ID NO: 35-42, SEQ ID NO: 51-58, SEQ ID NO: 67-74, SEQ ID NO: 83-90, SEQ ID NO: 97-104, SEQ ID NO: 113-120, SEQ ID NO: 147, SEQ ID NO: 149, SEQ ID NO: 153, SEQ ID NO: 155, SEQ ID NO: 168, SEQ ID NO:170, SEQ ID NO:172, SEQ ID NO:174, and SEQ ID NO:176.

In another aspect, the present application provides an immunoconjugate comprising the fusion polypeptide described in the present application.

In another aspect, the present application provides a nucleic acid molecule encoding the fusion polypeptide described in the present application.

In another aspect, the present application provides a vector comprising the nucleic acid molecule described in the present application.

In another aspect, the present application provides a cell comprising and/or expressing the fusion polypeptide described in the present application, the immunoconjugate described in the present application, the nucleic acid molecule described in the present application, and/or the vector described in the present application.

In another aspect, the present application provides a composition comprising the fusion polypeptide described in the present application, the immunoconjugate described in the present application, the nucleic acid molecule described in the present application, the carrier described in the present application, and/ or the cells described in the present application, and optionally a pharmaceutically acceptable carrier.

In another aspect, the present application provides a method for preparing the fusion polypeptide described in the present application, which comprises culturing the cells described in the present application under conditions enabling the expression of the fusion polypeptide.

In another aspect, the present application provides a method for blocking the interaction between PD-L1 protein and PD-1, which includes administering an effective amount of the fusion polypeptide described in the present application, the immunoconjugate described in the present application, the nucleic acid molecule described herein, the vector described herein, the cell described herein, and/or the composition described herein.

In another aspect, the present application provides a method for stimulating antigen-presenting cells and/or activating the action of T cells, which includes administering an effective amount of the fusion polypeptide described in the present application, the immunoconjugate described in the present application, the nucleic acid molecule described in the application, the vector described in the application, the cell described in the application, and/or the composition described in the application.

According to the method described in the present application, the stimulating antigen-presenting cells includes being selected from the following group: increasing the expression of co-stimulatory molecules in the antigen-presenting cells, causing the morphological changes and maturation of the antigen-presenting cells, and causing the antigen-presenting cells to secrete chemokines. Improve and enhance the phagocytic ability of antigen-presenting cells.

In another aspect, the present application provides a method for inhibiting the growth and/or proliferation of tumors or tumor cells, comprising administering an effective amount of the fusion polypeptide described in this application, the immunoconjugate described in this application, the nucleic acid molecule described herein, the vector described in this application, the cells described in this application, and/or the composition described in this application.

In another aspect, the present application provides the use of the fusion polypeptide described in the present application, the immunoconjugate described in the present application, the nucleic acid molecule described in the present application, the carrier described in the present application, the cell described in the present application, and/or the composition described in this application in the preparation of medicaments, wherein the medicaments can be used to prevent, improve and/or treat tumors.

According to the use described in the application, the tumor includes solid tumors and hematological tumors.

According to the purposes described in the application, the tumor is selected from the group consisting of colon tumor, breast tumor, lung tumor, stomach tumor, melanoma, head and neck tumor, lymphoma, nasopharyngeal tumor, cervical tumor, esophagus tumor, kidney tumor, squamous cell carcinoma of the skin, endometrial tumors, liver tumors, bladder tumors, urothelial tumors and skin tumors.

In another aspect, the present application provides the fusion polypeptide described in the present application, the immunoconjugate described in the present application, the nucleic acid molecule described in the present application, the carrier described in the present application, the cell described in the present application, and/or the composition described in the present application, which is used for preventing, improving and/or treating tumors.

In another aspect, the present application provides the fusion polypeptide described in the present application, the immunoconjugate described in the present application, the nucleic acid molecule described in the present application, the carrier described in the present application, the cell described in the present application, and/or Or the composition described in the present application, which is used for preventing, improving and/or treating tumors, wherein the tumors include solid tumors and hematological tumors.

In another aspect, the present application provides the fusion polypeptide described in the present application, the immunoconjugate described in the present application, the nucleic acid molecule described in the present application, the carrier described in the present application, the cell described in the present application, and/or Or the composition described in the application, it is used for preventing, improving and/or treating tumor, wherein said tumor is selected from following group: colon tumor, mammary gland tumor, lung tumor, stomach tumor, melanoma, head and neck tumor, lymphoma , nasopharyngeal tumors, cervical tumors, esophageal tumors, kidney tumors, skin squamous cell carcinoma, endometrial tumors, liver tumors, bladder tumors, urothelial tumors and skin tumors.

In another aspect, the present application provides a method for preventing, improving and/or treating tumors, which may comprise administering the fusion polypeptide described in the present application, the immunoconjugate described in the present application to a subject in need, The nucleic acid molecule described herein, the vector described herein, the cell described herein, and/or the composition described herein.

According to the method described in the application, the tumor includes solid tumors and hematological tumors.

According to the method described in the application, the tumor is selected from the group consisting of colon tumor, breast tumor, lung tumor, stomach tumor, melanoma, head and neck tumor, lymphoma, nasopharyngeal tumor, cervical tumor, esophagus tumor, kidney tumor , squamous cell carcinoma of the skin, endometrial tumors, liver tumors, bladder tumors, urothelial tumors and skin tumors.

Those skilled in the art can easily perceive other aspects and advantages of the present application from the following detailed description. In the following detailed description, only exemplary embodiments of the present application are shown and described. As those skilled in the art will appreciate, the content of the present application enables those skilled in the art to make changes to the specific embodiments which are disclosed without departing from the spirit and scope of the invention to which this application relates. Correspondingly, the drawings and descriptions in the specification of the present application are only exemplary rather than restrictive.

### Description of drawings

The particular features of the invention to which this application relates are set forth in the appended claims. The features and advantages of the invention to which this application relates can be better understood with reference to the exemplary embodiments described in detail hereinafter and the accompanying drawings. A brief description of the accompanying drawings is as follows:

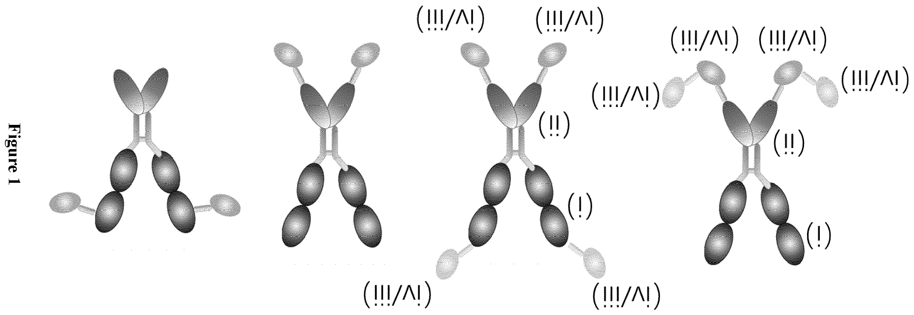

Figure 1 shows an exemplary structure diagram of the fusion polypeptide described in the present application. The present application provides exemplary examples that may include an antigen recognition region (i) derived from any anti-PD-L1 and/or anti-PD-1 antibody in the first domain, an immunoglobulin Fc region (ii), any one or more subunits in the second domain (iii) and/or (iv) the third domain (shown here is the second domain (iii) and/or the third domain (iv) with one subunit, although two or more of the same subunit linked in series can be considered, and can be extended to any subunit in (iv) mixed in combination and linked in series); the first domain binds to the second domain through the immunoglobulin Fc region, wherein the domains may be linked via a linker peptide or covalently, similarly the subunits of the third domain, the second domain may be linked via a linker peptide or covalently.
Figure 2 shows the results of SDS-PAGE detection of the expression of the fusion polypeptide complex of the present application.
Figures 3A-3F show the results of SEC-HPLC detection of the expression of the fusion polypeptide complex of the present application.
Figure 4 shows the results of detecting the binding of the fusion polypeptide complex of the present application to human PDL1 protein by ELISA.
Figure 5 shows the results of detecting the binding of the fusion polypeptide complex of the present application to human CTLA4 protein by ELISA.
Figure 6 shows the results of detecting the binding of the fusion polypeptide complex of the present application to human FGL1 protein by ELISA.
Figure 7 shows the results of detecting the combination of the fusion polypeptide complex of the present application with Raji cells expressing MHCII protein by flow cytometry.
Figures 8A-8B show the results of the fusion polypeptide complex of the present application regulating the activation of Jurkat T cells.
Figure 9 shows the results of the fusion polypeptide complex of the present application releasing the immunosuppressive effect mediated by CTLA4.
Figures 10A-10E show the expression results of co-stimulatory molecules up-regulated by the fusion polypeptide complex of the present application to regulate the expression of antigen-presenting cells.
Figure 11 shows the results of the fusion polypeptide complex of the present application regulating the morphology and differentiation of antigen-presenting cells.
Figures 12A-12B show the results of the fusion polypeptide complex of the present application regulating the secretion of CCL4 by antigen presentation cells.
Figures 13A-13B show the results of the fusion polypeptide complex of the present application regulating the phagocytosis of E.coli by antigen-presenting cells.
Figures 14A-14B show the results of activation of primary human T cells by the fusion polypeptide complex of the present application.
Figure 15 shows the results of the tumor inhibition curve of the fusion polypeptide complex of the present application in the MC38/hPD-L1 tumor model.
Figures 16A-16B show the results of the tumor inhibition curve of the fusion polypeptide complex of the present application in the EMT6/hPD-L1 tumor model.
Figure 17 shows the results of the regulation of Jurkat T cell activation by the fusion polypeptide complex of the present application.
Figures 18A-18B show the results of the fusion polypeptide complex of the present application regulating the secretion of CCL4 byantigen presentation cells.

### Embodiments

The implementation of the invention of the present application will be described in the following specific examples, and those skilled in the art can easily understand other advantages and effects of the invention of the present application from the content disclosed in this specification.

### Definition of Terms

In the present application, the term "regulation of T cell immune response" generally refers to the regulation of lymphatic T cell action by the dual and/or multifunctional fusion polypeptide disclosed in this application. For example, it can be extended to any other related T cell regulators that affect the expression of T cell NFAT transcription factors, the secretion of cytokines such as IL-2, IFN-γ, TNFα and Granzyme B, cell proliferation and effects such as cell killing of target cells including tumor cells, The T cells can be expanded to T cell lines and/or primary T cells including but not limited to CD4 T cells and CD4 T cell subsets Th1, Th2, Th9, Th17, TFH and/or Treg cells, etc.; also includes CD8 T cells include but are not limited to tumor tissue infiltrating CD8 T cells, effector CD8 T cells, immune memory CD8 T cells, etc.

In the present application, the term "stimulating antigen-presenting cells" generally refers to the regulation of antigen-presenting cell activation by the dual and/or multifunctional fusion polypeptides disclosed in this application. For example, it can also be extended to any other antigen-presenting cell regulator that affects the expression of antigen-presenting cells after treating the antigen-presenting cells, including but not limited to the costimulatory molecule CD40 or/and CD80 or/and CD83 or/and CD86. , activation and differentiation and maturation of antigen-presenting cells, antigen phagocytosis and presentation, production of chemokines CCL4, CCL22, CCL17, etc., expression of cytokines IL-6, IL-12, TNFa, IL-1β, etc., recruitment of T cells Migrate to secondary lymphoid organs and enhance T cell immune responses and other effects; the antigen-presenting cells can be expanded to but are not limited to B lymphocyte lines and primary B lymphocytes, monocyte lines and primary monocytes , macrophage cell lines and primary macrophages, dendritic cell (DC) cell lines and primary DC cells, etc.

In the present application, the term "relieving the inhibition of T cells by PD-L1/PD-1"generally refers to the dual and/or multifunctional fusion polypeptides disclosed in this application that bind to the PDL1 protein and block the binding of PD-L1 to PD-1, thereby improving the inhibition of T cells by PD-L1/PD-1, including but not limited to expressing the NFAT transcription factor on T cells, secreting cytokines such as IL-2, IFN-γ, TNFα and Granzyme B, and having effects on cell proliferation and cell killing of target cells.

In this application, the term "anti-tumor activity" generally refers to any biological activity that reduces or prevents the proliferation or viability of tumor cells in vivo and/or in vitro. In one embodiment, the anti-tumor activity is the anti-tumor effect possessed by the dual and/or multifunctional fusion polypeptide described in the present application.

In this application, the term "CD80" generally refers to a class of molecules that activate cells. For example, CD80 in this application includes its full length, variants, and/or functionally active fragments. For example, CD80 can refer to a polypeptide or fragment thereof that has at least about 85% amino acid identity with the protein encoded by the NCBI accession number Gene ID: 941 gene and has binding activity to CD28 (protein encoded by NCBI accession number Gene ID: 940 gene) and/or CTLA4 (protein encoded by NCBI accession number Gene ID: 1493 gene). Provided below is exemplary human CD80amino acid sequence (SEQ ID NO: 1); term "CD80 extracellular region" generally refers to a polypeptide or fragment thereof that has amino acid sequence of CD80 protein extracellular region or amino acid sequence that has at least about 85% amino acid identity, and has binding activity to CD28 and/or CTLA4; provided below is exemplary CD80 extracellular region amino acid sequence (SEQ ID NO: 2). The number of tandem combinations disclosed in this application is not limited to the number shown in the example, and any tandem combination including this functional domain can be regarded as within the scope of this application.

In this application, the term "anti-PD-L1 antibody" generally refers to an antibody that selectively binds and has the activity of blocking PD-L1 polypeptide. For example, Chinese Patent No. CN102245640(B), U.S. Patent Application Publication No. 7,943,743, U.S. Patent Application Publication No. 8,779,108 and U.S. Patent Application Publication No. 7,943,743, which are incorporated herein by reference; including but not limited to anti-PD-L1 antibodies such as Aspen Durvalumab (MEDI4736) developed by AstraZeneca, sugemalimab (CS1001) developed by CStone Pharmaceuticals, JS003 developed by Junshi Pharmaceuticals, Envafolimab (KN035) developed by Corning Jirui, Atezolizumab (MPDL3280A) developed by Roche and Germany Avelumab (MSB0010718C) jointly developed by Merck KGaA/Pfizer, the following provides an exemplary anti-PD-L1 antibody sugemalimab (CS1001) heavy chain polypeptide amino acid sequence (SEQ ID NO: 120) and light chain polypeptide amino acid sequence Sequence (SEQ ID NO: 121), JS003 heavy chain polypeptide amino acid sequence (SEQ ID NO: 3) and light chain polypeptide amino acid sequence (SEQ ID NO: 162), Durvalumab (MEDI4736) heavy chain polypeptide amino acid sequence (SEQ ID NO: 4) and light chain polypeptide amino acid sequence (SEQ ID NO: 163), atezolizumab (MPDL3280A) heavy chain polypeptide amino acid sequence (SEQ ID NO: 5) and light chain polypeptide amino acid sequence (SEQ ID NO: 164), Avelumab (MSB0010718C) The amino acid sequence of the heavy chain polypeptide (SEQ ID NO: 6), the amino acid sequence of the light chain polypeptide (SEQ ID NO: 165) and the amino acid sequence of Envafolimab (KN035) (SEQ ID NO: 7).

In this application, the term "anti-PD-1 antibody" generally refers to an antibody that selectively binds and has the activity of blocking PD-1 polypeptide. For example, U.S. Patent Application Publication No. 8,354,509 and U.S. Patent Application Publication No. 7,488,802, which are incorporated herein by reference; including but not limited to anti-PD-1 antibodies such as Permbrolizumab (MK-3475) developed by Merck and Bristol- For Nivolumab (BMS-936558) developed by BMS, the amino acid sequence of the heavy chain polypeptide (SEQ ID NO: 8) and light chain polypeptide amino acid sequence of an exemplary anti-PD-1 antibody Permbrolizumab (MK-3475) are provided below (SEQ ID NO: 166) and Nivolumab (BMS-936558) heavy chain polypeptide amino acid sequence (SEQ ID NO: 9) and light chain polypeptide amino acid sequence (SEQ ID NO: 167).

In this application, the term "LAG3" generally refers to a class of proteins or polypeptides. For example, LAG3 in this application includes its full length, variants, and/or functionally active fragments. For example, LAG3 may refer to a polypeptide or its fragment that has at least about 85% amino acid identity with the protein encoded by the NCBI accession number Gene ID: 3902 gene of LAG3 (Lymphocyte activation gene 3): and has binding activity to a complex protein MHCII (or HLA-DR) assembled from alpha subunit (the protein encoded by the NCBI accession number Gene ID: 3122 gene) and the β subunit (the protein encoded by the NCBI accession number Gene ID: 3123 gene, or the NCBI accession number Gene ID: 3125 gene) and/or FGL1 (Protein encoded by NCBI accession number Gene ID: 2267 gene); provided below is an exemplary human LAG3 amino acid sequence (SEQ ID NO: 10); the term "LAG3 extracellular region" usually refers to a polypeptide or fragment thereof that has the amino acid sequence of the extracellular region of the full-length LAG3 protein or the amino acid sequence that has at least about 85% amino acid identity, and has binding activity to MHCII and/or FGL1; provided below is an exemplary human LAG3 extracellular region amino acid sequence (SEQ ID NO: 11) and a tandem human LAG3 extracellular region amino acid sequence (SEQ ID NO: 12) covalently linked by a linker peptide; the number of tandems disclosed in this application is not limited to the number shown in the example, any tandem combination comprising this functional domain all can be regarded as the category of this application.

In this application, the term "LAG3-IgD1" generally refers to a polypeptide or fragment thereof that has an amino acid sequence of the Ig-like V-type domain in the extracellular region of human LAG3 (or an amino acid sequence has at least about 85% amino acid identity). Provided below is exemplary human LAG3-IgD1 amino acid sequence (SEQ ID NO: 13); term "LAG3-IgD2" generally refers to a polypeptide or fragment thereof that has an amino acid sequence of Ig-like C2-type 1 domain of human LAG3 extracellular region (or an amino acid sequence has at least about 85%amino acid identity); provided below is exemplary human LAG3-IgD2 amino acid sequence (SEQ ID NO: 14); term "LAG3-IgD3" generally refers to amino acid sequence of human LAG3 extracellular region Ig-like C2-type 2 domain (or an amino acid sequence has at least about 85% amino acid identity); provided below is exemplary human LAG3-IgD3amino acid sequence (SEQ ID NO: 15); term "LAG3-IgD4" generally refers to amino acid sequence of human extracellular region LAG3 Ig-like C2-type 3 (or an amino acid sequence has at least about 85% amino acid identity); provided below is exemplary human LAG3-IgD4amino acid sequence (SEQ ID NO: 16); term "LAG3-IgD1/IgD2" generally refers to a polypeptide or fragment thereof that has amino acid sequence of human LAG3-IgD1 and human LAG3-IgD2 (or an amino acid sequence has at least about 85%amino acid identity), and has binding activity to MHCII and/or FGL1; provided below is exemplary human LAG3-IgD1/IgD2 amino acid sequence (SEQ ID NO: 17); The number of tandem combinations disclosed in this application is not limited to the number shown in the example, any tandem combination comprising LAG3-IgD1/IgD2 functional domain can be deemed as within the scope of this application; term "LAG3-IgD1/IgD2/IgD3" generally refers to a polypeptide or fragment thereof that has amino acid sequence of human LAG3-IgD1, human LAG3-IgD2 and human LAG3-IgD3 (or an amino acid sequence has at least about 85% amino acid identity) and has binding activity to MHCII and/or FGL1; provided below is exemplary human LAG3-IgD1/IgD2/IgD3 amino acid sequence (SEQ ID NO: 18); The number of tandem combinations disclosed in this application is not limited to the number shown in the example, any tandem combinationcan comprising LAG3-IgD1/IgD2/IgD3 functional domain can be deemed as within the scope of this application; term "LAG3-IgD1/IgD2/IgD3/IgD4" generally refers to polypeptide or fragment thereof that has amino acid sequence of human LAG3-IgD 1, human LAG3-IgD2, human LAG3-IgD3 and human LAG3-IgD4 (or an amino acid sequence has at least about 85% amino acid identity), and has MHCII and/or FGL1 binding activity; provided below is human LAG3-IgD1/IgD2/IgD3/IgD4 amino acid sequence (SEQ ID NO: 19); The number of tandem combinations disclosed in this application is not limited to the number shown in the example, any tandem combination containing functional domain of LAG3-IgD1/IgD2/IgD3/IgD4 can be deemed as within the scope of this application.

In this application, the term "fusion polypeptide" generally refers to a polypeptide obtained by fusion of two or more proteins or polypeptides. Fusion polypeptides can be artificially produced by recombinant DNA techniques. For example, the genes or nucleic acid molecules encoding the two or more proteins or polypeptides can be linked to each other to form a fusion gene or a fused nucleic acid molecule which can encode the fusion polypeptide. Translation of the fusion gene may result in a single polypeptide, which may have the properties of at least one, or even each, of the two or more proteins or polypeptides prior to fusion.

In this application, the term "antibody-associated antigen-binding fragment" generally refers to an antibody comprising an amino acid fragment responsible for specific binding to an antigen. It can be a fragment that determines the key difference in an antibody molecule, and can also be called an antigen-binding domain, or an "epitope" or "antigenic determinant"; an antigen-binding domain is usually composed of an antibody heavy chain variable region (VH ) and antibody light chain variable region (VL), however, it does not necessarily include both, and the antigen-binding domain of the antibody disclosed in this application is not limited to the traditional domain consisting of VH and VL, but also includes any Antigen-binding domains contained in other types of antibodies such as but not limited to recombinant antibodies, single domain antibodies, heavy chain antibodies, chimeric antibodies, bispecific antibodies and other unconventional antibodies and combinations thereof.

Furthermore, the anti-PD-1 antibody and/or anti-PD-L1 antibody disclosed in this application are not limited to traditional natural antibodies, and should include any other antibodies with anti-PD-1 antibody and/or anti-PD-L1 properties such as but not limited to recombinant antibodies, single domain antibodies, heavy chain antibodies, chimeric antibodies, bispecific antibodies and other unconventional antibodies and combinations thereof.

In this application, the term "Fab" generally refers to an antibody fragment consisting of VL, VH, CL and CH1 domains.

In this application, the term "Fab'" generally refers to an antibody fragment that has several additional residues at the carboxy-terminus of the CH1 domain compared to the Fab fragment. For example, a Fab' may include one or more cysteines from the antibody hinge region.

In this application, the term "F(ab)₂" generally refers to an antigen-binding fragment obtained from a pair of Fab fragments linked by cysteines.

In this application, the term "dAb fragment" generally refers to an antibody fragment consisting of a VH domain (Ward et al., Nature 341:544546 (1989)).

In this application, the term "complementarity determining region CDR" usually refers to the three hypervariable regions (HVR) of the light chain variable region (VL) and the heavy chain variable region (VH). It forms a precise complementarity with the antigenic determinant, so the hypervariable region is also called complementarity-determining region.

In this application, the term "Fv fragment" generally refers to an antibody fragment consisting of the VL and VH domains of a single arm of an antibody.

In this application, the term "scFv" generally refers to a molecule formed by linking the variable region of the heavy chain and the variable region of the light chain of an antibody through a short peptide linker (linker), also known as a single-chain antibody.

The protein, polypeptide and/or amino acid sequence involved in this application should also be understood to include at least the following scope: variants or homologues having the same or similar functions as the protein or polypeptide.

In the present application, the variant may be that one or more amino acids are substituted, deleted or added to the amino acid sequence of the protein or the polypeptide (for example, an antibody or fragment thereof that specifically binds the protein). For example, the functional variant may comprise proteins or polypeptides with amino acid changes by at least 1, such as 1-30, 1-20 or 1-10, further such as 1, 2, 3, 4 or 5 amino acid substitutions, deletion and/or insertion. The functional variant may substantially maintain the biological properties of the protein or polypeptide prior to alteration (eg, substitution, deletion or addition). For example, the functional variant may retain at least 60%, 70%, 80%, 90%, or 100% of the biological activity (eg, antigen binding ability) of the protein or polypeptide prior to the alteration. For example, the substitutions may be conservative substitutions.

In the present application, the homologue may be a protein or polypeptide having at least about 85% (for example, having at least about 85%, about 90%, about 91%, about 92%, about 93%, about 94%, about 95%, about 96%, about 97%, about 98%, about 99% or more) sequence homology with the amino acid sequence of the protein and/or the polypeptide.

In this application, the homology generally refers to the similarity or association between two or more sequences. "Percent sequence homology" can be calculated by comparing the two sequences to be aligned in a comparison window and determining the presence of identical nucleic acid bases (e.g., A, T, C, G, I) in both sequences. ) or the same amino acid residue (e.g., Ala, Pro, Ser, Thr, Gly, Val, Leu, Ile, Phe, Tyr, Trp, Lys, Arg, His, Asp, Glu, Asn, Gln, Cys, and Met) Number of positions To obtain the number of matching positions, divide the number of matching positions by the total number of positions in the comparison window (i.e., window size), and multiply the result by 100 to produce percent sequence homology. Alignment to determine percent sequence identity can be accomplished in various ways known in the art, for example, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared or over a region of sequence of interest. The homology can also be determined by the following methods: FASTA and BLAST.

Usually, in a polypeptide chain, the amino group is connected to another carboxyl group in the polypeptide chain to make it a chain, but at the two ends of the protein, the remaining amino acid residues that do not form a peptide bond are respectively carrying the free end of the polypeptide chain with an amino group and the end of the polypeptide chain with a carboxyl group. In this application, the term "N-terminal" generally refers to the end of a polypeptide chain whose amino acid residues bear a free amino group. In this application, the term "C-terminal" generally refers to the end of the polypeptide chain whose amino acid residue bears a free carboxyl group.

In this application, the term "nucleic acid molecule" generally refers to an isolated form of nucleotides, deoxyribonucleotides or ribonucleotides or analogs thereof of any length isolated from their natural environment or artificially synthesized.

In this application, the term "immunoconjugate" generally refers to a polypeptide molecule conjugated to one or more heterologous molecules, including but not limited to cytotoxins.

In the present invention, the term "vector" refers to a nucleic acid delivery tool into which a polynucleotide encoding a protein can be inserted and the protein can be expressed.

In the present application, the term "immunoglobulin Fc domain" generally refers to the Fc fragment of one Fc fragment and two identical Fab fragments formed by papain hydrolysis of traditional antibody IgG. The Fc domain can include antibody heavy chain CH2, CH3 and hinge region fragments. The traditional Fc fragment has the function of binding to the Fc fragment receptor to mediate related biological effects, and site-specific mutation can change its binding ability to the corresponding target receptor, thus affecting its biological function, and the immunoglobulin Fc domain disclosed in this application should include but not limited to traditional Fc fragments and any other forms of Fc mutants. The following provides an exemplary human immunoglobulin IgG1 Fc domain (SEQ ID NO: 20) and Fc domain of human immunoglobulin IgG4 (SEQ ID NO: 21).

In this application, the term "linker peptide" generally refers to a linker molecule that links one or more polypeptides or domains thereof. For example, the linker peptide may have conformational flexibility and be a short peptide chain formed by a combination of amino acid Gly(G) and Ser(S) residues, wherein the ratio of the number of amino acid Gly to the number of amino acid Ser may be ≥1. The linker peptide as disclosed in this application can be extended to any short peptide with this property. The following provides exemplary linker peptide amino acid sequences including but not limited to GGGGS (SEQ ID NO: 22), GGGGSGGGS (SEQ ID NO: 23), GGGGSGGGGSGGGGS (SEQ ID NO :24), GGGGSGGGSGGGGSGGGGS (SEQ ID NO:25) and GGGGSGGGS (SEQ ID NO:26).

In this application, the term "dual and/or multifunctional fusion polypeptide" generally refers to a polypeptide or protein fused from one or more sources of polypeptide or its domains. Anti-PD-L1 antibody and/or anti-PD-1 antibody via the linker peptide is covalently linked to that at least comprising the CD80 extracellular domain, and/or that at least comprising the LAG3 extracellular domain, and/or that at least comprising one functional domain of LAG3 extracellular domain to form the functional fusion polypeptide, wherein the disclosed two or more LAG3 extracellular domain or/and functional domain comprising at least one LAG3 extracellular domain and LAG3 extracellular domain or/and functional domain comprising at least one LAG3 extracellular domain is linked in series to form two or more LAG3 extracellular domain; or/and functional domain comprising at least one LAG3 extracellular domainis linked via the linker peptide; the dual and/or multifunctional fusion polypeptide disclosed in this application includes but are not limited to the anti-PD-L1 antibody and/or the anti-PD-1 antibody; functional fusion polypeptide or combination thereof wherein any form of anti-PD-L1 antibody and/or anti-PD-1 antibody via a linker peptide is covalently linked to at least one subunit functional domain comprising CD80 extracellular domain, and/or LAG3 extracellular domain, and/or LAG3 extracellular domain should be deemed as within the scope of the present application.

In this application, the term "comprising" generally means the inclusion of explicitly specified features, but not the exclusion of other elements.

In this application, the term "about" generally refers to a range of 0.5%-10% above or below the specified value, such as 0.5%, 1%, 1.5%, 2%, 2.5%, above or below the specified value. 3%, 3.5%, 4%, 4.5%, 5%, 5.5%, 6%, 6.5%, 7%, 7.5%, 8%, 8.5%, 9%, 9.5%, or 10%.

### Detailed description of the invention

In one aspect, the application provides a fusion polypeptide, the fusion polypeptide may comprise a first domain and a second domain, the first domain may comprise HCDR3 of an antibody heavy chain, and the antibody heavy chain may comprise the amino acid sequence shown in SEQ ID NO: 121, and the second domain can activate immune response. For example, the fusion polypeptide may comprise one or more of the first domains and one or more of the second domains.

For example, the first domain may comprise HCDR2 of an antibody heavy chain, wherein the antibody heavy chain may comprise the amino acid sequence shown in SEQ ID NO:121.

For example, the first domain may comprise HCDR1 of an antibody heavy chain, wherein the antibody heavy chain may comprise the amino acid sequence shown in SEQ ID NO:121.

For example, the first domain may comprise the heavy chain variable region VH of an antibody heavy chain, wherein the antibody heavy chain may comprise the amino acid sequence shown in SEQ ID NO: 121.

For example, the first domain may comprise a heavy chain, wherein the antibody heavy chain may comprise the amino acid sequence shown in SEQ ID NO: 121.

For example, the first domain may comprise LCDR3 of an antibody light chain, wherein the antibody light chain may comprise the amino acid sequence shown in SEQ ID NO: 122.

For example, the first domain may comprise LCDR2 of an antibody light chain, wherein the antibody light chain may comprise the amino acid sequence shown in SEQ ID NO: 122.

For example, the first domain may comprise LCDR1 of an antibody light chain, wherein the antibody light chain may comprise the amino acid sequence shown in SEQ ID NO: 122.

For example, the first domain may comprise the light chain variable region VL of an antibody light chain, wherein the antibody light chain may comprise the amino acid sequence shown in SEQ ID NO: 122.

For example, the first domain may comprise a light chain, wherein the antibody light chain may comprise the amino acid sequence shown in SEQ ID NO: 122.

For example, the first domain may comprise HCDR3, HCDR2 and HCDR1 of an antibody heavy chain, wherein the antibody heavy chain may comprise the amino acid sequence shown in SEQ ID NO: 121. For example, the antibody can be Sugemalimab.

For example, the first domain may comprise LCDR3, LCDR2 and LCDR1 of an **antibody heavy chain,** wherein the antibody light chain may comprise the amino acid sequence shown in SEQ ID NO: 122. For example, the antibody can be Sugemalimab.

For example, the first domain may comprise HCDR3, HCDR2 and HCDR1 of an antibody heavy chain, wherein the antibody heavy chain may comprise an amino acid sequence as shown in SEQ ID NO: 121, and the first domain may comprise LCDR3, LCDR2 and LCDR1 of an **antibody heavy chain,** wherein the antibody light chain may comprise the amino acid sequence shown in SEQ ID NO: 122. For example, the antibody can be Sugemalimab.

For example, the first domain may comprise the heavy chain variable region VH of an antibody heavy chain, wherein the antibody heavy chain may comprise the amino acid sequence shown in SEQ ID NO: 121, and the first domain may comprise the light chain variable region VL of an antibody light chain, wherein the antibody light chain may comprise the amino acid sequence shown in SEQ ID NO: 122.

For example, the first domain may comprise a heavy chain, which may comprise the amino acid sequence shown in SEQ ID NO: 121, and the first domain may comprise a light chain, which may comprise a light chain such as Amino acid sequence shown in SEQ ID NO: 122.

For example, the first domain may comprise an antibody or antigen-binding fragment thereof.

For example, the antibody may be selected from the group consisting of recombinant antibodies, single domain antibodies, heavy chain antibodies, chimeric antibodies and bispecific antibodies.

For example, the antigen-binding fragment may be selected from one or more of the group consisting of: Fab, Fab', Fv fragment, F(ab')₂, F(ab)₂, scFv, di-scFv, VHH and dAb.

For example, said second domain may be selected from the group consisting of CD80 or a functionally active fragment thereof, and LAG3 or a functionally active fragment thereof.

For example, the second domain may be selected from the group consisting of human-derived CD80 or a functionally active fragment thereof, mouse-derived CD80 or a functionally active fragment thereof, human-derived LAG3 or a functionally active fragment thereof and mouse-derived LAG3 or a functionally active fragment thereof.

For example, the second domain can bind to CD28 and CTLA4.

For example, the second domain may comprise the extracellular domain of CD80 or a functionally active fragment thereof.

For example, the second domain may comprise the amino acid sequence shown in SEQ ID NO:2.

For example, the second domain is capable of binding to MHCII molecules on antigen presenting cells.

For example, the second domain may comprise the extracellular domain of LAG3 or a functionally active fragment thereof.

For example, the second domain may comprise the amino acid sequence shown in SEQ ID NO:11.

For example, said second domain may comprise IgD1, IgD2, IgD3 and/or IgD4 of LAG3 or a functionally active fragment thereof.

For example, the second domain may comprise an amino acid sequence selected from the group consisting of SEQ ID NO: 13, SEQ ID NO: 14, SEQ ID NO: 15, SEQ ID NO: 16, SEQ ID NO: 17, SEQ ID NO: 18, SEQ ID NO: 19, and SEQ ID NO: 12.

For example, the first domain and the second domain may be directly or indirectly linked.

For example, the first domain may comprise an antibody heavy chain, and the antibody heavy chain of the first domain may be directly or indirectly linked to the second domain.

For example, the first domain may comprise an antibody light chain, and the antibody light chain of the first domain may be directly or indirectly linked to the second domain.

For example, the N-terminus of the first domain and the second domain may be linked directly or indirectly.

For example, the C-terminus of the first domain and the second domain may be linked directly or indirectly.

For example, the first domain may comprise an antibody heavy chain, and the antibody heavy chain of the first domain may be directly or indirectly linked to the N-terminus of the second domain.

For example, the first domain may comprise an antibody heavy chain, and the antibody heavy chain of the first domain may be directly or indirectly linked to the C-terminus of the second domain.

For example, the first domain may comprise an antibody light chain, and the antibody light chain of the first domain may be directly or indirectly linked to the N-terminus of the second domain.

For example, the first domain may comprise an antibody light chain, and the antibody light chain of the first domain may be directly or indirectly linked to the C-terminus of the second domain.

For example, said indirect link can comprise link through a linker.

For example, the linker may comprise an amino acid sequence selected from the group consisting of SEQ ID NO:22, SEQ ID NO:23, SEQ ID NO:24, SEQ ID NO:25, and SEQ ID NO:26.

For example, the fusion polypeptide may comprise an amino acid sequence selected from the group consisting of SEQ ID NO: 27-42, SEQ ID NO: 130, SEQ ID NO: 132, SEQ ID NO: 135, SEQ ID NO: 137, SEQ ID NO: 139, SEQ ID NO: 147, SEQ ID NO: 149, SEQ ID NO: 153, SEQ ID NO: 155, SEQ ID NO: 159, SEQ ID NO: 161, SEQ ID NO: 168, SEQ ID NO: 170, SEQ ID NO: 172, SEQ ID NO: 174, and SEQ ID NO: 176.

In another aspect, the present application provides a fusion polypeptide, which may comprise a first domain and a second domain, the first domain can block PD-1/PD-L1 signaling, and the second domain may comprise LAG3 or a functionally active fragment thereof. For example, the fusion polypeptide may comprise one or more of the first domains and one or more of the second domains.

For example, the first domain may comprise HCDR3 of an antibody heavy chain, which may comprise an amino acid sequence selected from the group consisting of: SEQ ID NO: 3-9, and SEQ ID NO: 121.

For example, the first domain may comprise HCDR2 of an antibody heavy chain, which may comprise an amino acid sequence selected from the group consisting of SEQ ID NO: 3-9, and SEQ ID NO: 121.

For example, the first domain may comprise HCDR1 of an antibody heavy chain, which may comprise an amino acid sequence selected from the group consisting of: SEQ ID NO: 3-9, and SEQ ID NO: 121.

For example, the first domain may comprise the heavy chain variable region VH of an antibody heavy chain, wherein the antibody heavy chain may comprise an amino acid sequence selected from the group consisting of SEQ ID NO: 3-9, and SEQ ID NO: 121.

For example, the first domain may comprise an antibody heavy chain, which may comprise an amino acid sequence selected from the group consisting of SEQ ID NO: 3-9, and SEQ ID NO: 121.

For example, the first domain may comprise LCDR3 of an antibody light chain, wherein the antibody light chain may comprise an amino acid sequence selected from the group consisting of: SEQ ID NO: 162-167, and SEQ ID NO: 122.

For example, the first domain may comprise LCDR2 of an antibody light chain, wherein the antibody light chain may comprise an amino acid sequence selected from the group consisting of: SEQ ID NO: 162-167, and SEQ ID NO: 122.

For example, the first domain may comprise LCDR1 of an antibody light chain, wherein the antibody light chain may comprise an amino acid sequence selected from the group consisting of: SEQ ID NO: 162-167, and SEQ ID NO: 122.

For example, the first domain may comprise the light chain variable region VL of an antibody light chain, wherein the antibody light chain may comprise an amino acid sequence selected from the group consisting of: SEQ ID NO: 162-167, and SEQ ID NO: 122.

For example, the first domain may comprise an antibody light chain, wherein the antibody light chain may comprise an amino acid sequence selected from the group consisting of: SEQ ID NO: 162-167, and SEQ ID NO: 122.

For example, the first domain may comprise HCDR3, HCDR2 and HCDR1 of an antibody heavy chain, wherein the antibody heavy chain may comprise the amino acid sequence shown in SEQ ID NO: 121. For example, the antibody can be Sugemalimab.

For example, the first domain may comprise LCDR3, LCDR2 and LCDR1 of an antibody **heavy chain,** wherein the antibody light chain may comprise the amino acid sequence shown in SEQ ID NO: 122. For example, the antibody can be Sugemalimab.

For example, the first domain may comprise HCDR3, HCDR2 and HCDR1 of an antibody heavy chain, wherein the antibody heavy chain may comprise the amino acid sequence shown in SEQ ID NO: 121, and the first domain may comprise LCDR3, LCDR2 and LCDR1 of an antibody **heavy chain,** wherein the antibody light chain may comprise the amino acid sequence shown in SEQ ID NO: 122. For example, the antibody can be Sugemalimab.

For example, the first domain may comprise heavy chain variable region VH of antibody heavy chain, wherein the antibody heavy chain may comprise amino acid sequence shown in SEQ ID NO: 121, and the first domain may comprise light chain variable region VL of antibody light chain, wherein the antibody light chain may comprise amino acid sequence shown in SEQ ID NO: 122. For example, the antibody can be Sugemalimab.

For example, the first domain may comprise HCDR3, HCDR2 and HCDR1 of an antibody heavy chain, wherein the antibody heavy chain may comprise the amino acid sequence shown in SEQ ID NO:3. For example, the antibody can be JS003.

For example, the first domain may comprise LCDR3, LCDR2 and LCDR1 of an antibody heavy chain, wherein the antibody light chain may comprise the amino acid sequence shown in SEQ ID NO: 162. For example, the antibody can be JS003.

For example, the first domain may comprise HCDR3, HCDR2 and HCDR1 of an antibody heavy chain, wherein the antibody heavy chain may comprise the amino acid sequence shown in SEQ ID NO: 3, and the first domain may comprise LCDR3, LCDR2 and LCDR1 of an antibody **heavy chain,** wherein the antibody light chain may comprise the amino acid sequence shown in SEQ ID NO: 162. For example, the antibody can be JS003.

For example, the first domain may comprise the heavy chain variable region VH of an antibody heavy chai, wherein the antibody heavy chain may comprise the amino acid sequence as shown in SEQ ID NO: 3, and the first domain may comprise light chain variable region VL of antibody light chain, wherein the antibody light chain may comprise the amino acid sequence shown in SEQ ID NO: 162. For example, the antibody can be JS003.

For example, the first domain may comprise HCDR3, HCDR2 and HCDR1 of an antibody heavy chain, wherein the antibody heavy chain may comprise the amino acid sequence shown in SEQ ID NO:4. For example, the antibody can be Durvalumab.

For example, the first domain may comprise LCDR3, LCDR2 and LCDR1 of an antibody heavy chain, wherein the antibody light chain may comprise the amino acid sequence shown in SEQ ID NO: 163. For example, the antibody can be Durvalumab.

For example, the first domain may comprise HCDR3, HCDR2 and HCDR1 of an antibody heavy chain, wherein the antibody heavy chain may comprise an amino acid sequence as shown in SEQ ID NO: 4, and the first domain may comprise LCDR3, LCDR2 and LCDR1 of an **antibody heavy chain, wherein the light chain** of the antibody may comprise the amino acid sequence shown in SEQ ID NO: 163. For example, the antibody can be Durvalumab.

For example, the first domain may comprise the heavy chain variable region VH of an antibody heavy chain, wherein the antibody heavy chain may comprise the amino acid sequence shown in SEQ ID NO: 4, and the first domain may comprise the light chain variable region VL of an antibody light chain, wherein the antibody light chain may comprise the amino acid sequence shown in SEQ ID NO: 163. For example, the antibody can be Durvalumab.

For example, the first domain may comprise HCDR3, HCDR2 and HCDR1 of an antibody heavy chain, wherein the antibody heavy chain may comprise the amino acid sequence shown in SEQ ID NO:5. For example, the antibody can be atezolizumab.

For example, the first domain may comprise LCDR3, LCDR2 and LCDR1 of an antibody heavy chain, wherein the antibody light chain may comprise the amino acid sequence shown in SEQ ID NO: 164. For example, the antibody can be atezolizumab.

For example, the first domain may comprise HCDR3, HCDR2 and HCDR1 of an antibody heavy chain, wherein the antibody heavy chain may comprise the amino acid sequence shown in SEQ ID NO: 5, and the first domain may comprise LCDR3, LCDR2 and LCDR1 of an antibody **heavy chain,** wherein the light chain of the antibody may comprise the amino acid sequence shown in SEQ ID NO: 164. For example, the antibody can be atezolizumab.

For example, the first domain may comprise the heavy chain variable region VH of an antibody heavy chain, wherein the antibody heavy chain may comprise the amino acid sequence shown in SEQ ID NO: 5, and the first domain may comprise the light chain variable region VL of an antibody light chain, wherein the antibody light chain may comprise the amino acid sequence shown in SEQ ID NO: 164. For example, the antibody can be atezolizumab.

For example, the first domain may comprise HCDR3, HCDR2 and HCDR1 of an antibody heavy chain, wherein the antibody heavy chain may comprise the amino acid sequence shown in SEQ ID NO:6. For example, the antibody can be Avelumab.

For example, the first domain may comprise LCDR3, LCDR2 and LCDR1 of an antibody **heavy chain,** wherein the antibody light chain may comprise the amino acid sequence shown in SEQ ID NO: 165. For example, the antibody can be Avelumab.

For example, the first domain may comprise HCDR3, HCDR2 and HCDR1 of an antibody heavy chain, wherein the antibody heavy chain may comprise the amino acid sequence shown in SEQ ID NO: 6, and the first domain may comprise an anti- LCDR3, LCDR2 and LCDR1 of the heavy chain, wherein the light chain of the antibody may comprise the amino acid sequence shown in SEQ ID NO: 165. For example, the antibody can be Avelumab.

For example, the first domain may comprise the heavy chain variable region VH of an antibody heavy chain, wherein the antibody heavy chain may comprise the amino acid sequence shown in SEQ ID NO: 6, and the first domain may comprise the light chain variable region VL of an antibody light chain, wherein the antibody light chain may comprise the amino acid sequence shown in SEQ ID NO: 165. For example, the antibody can be Avelumab.

For example, the first domain may comprise HCDR3, HCDR2 and HCDR1 of an antibody, wherein the antibody may comprise the amino acid sequence shown in SEQ ID NO:7. For example, the antibody can be Envafolimab.

For example, the first domain may comprise HCDR3, HCDR2 and HCDR1 of an antibody heavy chain, wherein the antibody heavy chain may comprise the amino acid sequence shown in SEQ ID NO:8. For example, the antibody can be Permbrolizumab.

For example, the first domain may comprise LCDR3, LCDR2 and LCDR1 of an antibody heavy chain, wherein the antibody light chain may comprise the amino acid sequence shown in SEQ ID NO: 166. For example, the antibody can be Permbrolizumab.

For example, the first domain may comprise HCDR3, HCDR2 and HCDR1 of an antibody heavy chain, wherein the antibody heavy chain may comprise an amino acid sequence as shown in SEQ ID NO: 8, and the first domain may comprise LCDR3, LCDR2 and LCDR1 of an antibody **heavy chain,** wherein the light chain of the antibody may comprise the amino acid sequence shown in SEQ ID NO: 166. For example, the antibody can be Permbrolizumab.

For example, the first domain may comprise the heavy chain variable region VH of an antibody heavy chain, the antibody heavy chain may comprise the amino acid sequence shown in SEQ ID NO: 8, and the first domain may comprise the light chain variable region VL of the light chain, the antibody light chain may comprise the amino acid sequence shown in SEQ ID NO: 166. For example, the antibody can be Permbrolizumab.

For example, the first domain may comprise HCDR3, HCDR2 and HCDR1 of an antibody heavy chain, wherein the antibody heavy chain may comprise the amino acid sequence shown in SEQ ID NO:9. For example, the antibody can be Nivolumab.

For example, the first domain may comprise LCDR3, LCDR2 and LCDR1 of an antibody heavy chain, wherein **the antibody light chain** may comprise the amino acid sequence shown in SEQ ID NO: 167. For example, the antibody can be Nivolumab.

For example, the first domain may comprise HCDR3, HCDR2 and HCDR1 of an antibody heavy chain, wherein the antibody heavy chain may comprise an amino acid sequence as shown in SEQ ID NO: 9, and the first domain may comprise an LCDR3, LCDR2 and LCDR1 of the heavy chain, wherein **the light chain** of the antibody may comprise the amino acid sequence shown in SEQ ID NO: 167. For example, the antibody can be Nivolumab.

For example, the first domain may comprise the heavy chain variable region VH of an antibody heavy chain, wherein the antibody heavy chain may comprise the amino acid sequence shown in SEQ ID NO: 9, and the first domain may comprise the light chain variable region VL of an antibody light chain, wherein the antibody light chain may comprise the amino acid sequence shown in SEQ ID NO: 167. For example, the antibody can be Nivolumab.

For example, the first domain can bind to PD-L1 and/or PD-1.

For example, the first domain may comprise an antibody or antigen-binding fragment thereof.

For example, the antibody may be selected from the group consisting of recombinant antibodies, single domain antibodies, heavy chain antibodies, chimeric antibodies and bispecific antibodies.

For example, the antigen-binding fragment may be selected from one or more of the group consisting of Fab, Fab', Fv fragment, F(ab')2, F(ab)2, scFv, di-scFv, VHH and dAb.

For example, the second domain may be selected from the group consisting of human-derived LAG3 or a functionally active fragment thereof and mouse-derived LAG3 or a functionally active fragment thereof.

For example, the second domain can bind an MHCII molecule on an antigen presenting cell.

For example, the second domain may comprise the extracellular domain of LAG3 or a functionally active fragment thereof.

For example, the second domain may comprise the amino acid sequence shown in SEQ ID NO:11.

For example, said second domain may comprise IgD1, IgD2, IgD3 and/or IgD4 of LAG3 or a functionally active fragment thereof.

For example, the second domain may comprise IgD1, IgD1-IgD2, IgD1-IgD2-IgD3, and/or IgD1-IgD2-IgD3-IgD4 of LAG3 or a functionally active fragment thereof.

For example, the second domain may comprise an amino acid sequence selected from the group consisting of SEQ ID NO: 13, SEQ ID NO: 14, SEQ ID NO: 15, SEQ ID NO: 16, SEQ ID NO: 17, SEQ ID NO: 18, SEQ ID NO: 19, and SEQ ID NO: 12.

For example, the first domain and the second domain may be directly or indirectly linked.

For example, the first domain may comprise an antibody heavy chain, and the antibody heavy chain of the first domain may be directly or indirectly linked to the second domain.

For example, the first domain may comprise an antibody light chain, and the antibody light chain of the first domain may be directly or indirectly linked to the second domain.

For example, the N-terminus of the first domain and the second domain may be linked directly or indirectly.

For example, the C-terminus of the first domain and the second domain may be linked directly or indirectly.

For example, the first domain may comprise an antibody light chain, and the antibody light chain of the first domain may be directly or indirectly linked to the N-terminus of the second domain.

For example, the first domain may comprise an antibody light chain, and the antibody light chain of the first domain may be directly or indirectly linked to the C-terminus of the second domain.

For example, the first domain may comprise an antibody heavy chain, and the antibody heavy chain of the first domain may be directly or indirectly linked to the N-terminus of the second domain.

For example, the first domain may comprise an antibody heavy chain, and the antibody heavy chain of the first domain may be directly or indirectly linked to the C-terminus of the second domain.

For example, said indirect link can comprise link through a linker.

For example, the linker may comprise an amino acid sequence selected from the group consisting of SEQ ID NO:22, SEQ ID NO:23, SEQ ID NO:24, SEQ ID NO:25, and SEQ ID NO:26.

For example, the fusion polypeptide may comprise an amino acid sequence selected from the group consisting of: SEQ ID NO: 28-30, SEQ ID NO: 32, SEQ ID NO: 34-42, SEQ ID NO: 44-46, SEQ ID NO: 44-46, NO: 48, SEQ ID NO: 50-58, SEQ ID NO: 60-62, SEQ ID NO: 64, SEQ ID NO: 66-74, SEQ ID NO: 76-78, SEQ ID NO: 80, SEQ ID NO: 82-90, SEQ ID NO: 92-94, SEQ ID NO: 96-104, SEQ ID NO: 106-108, SEQ ID NO: 110, SEQ ID NO: 112-120, SEQ ID NO: 135, SEQ ID NO: 137, SEQ ID NO: 139, SEQ ID NO: 147, SEQ ID NO: 149, SEQ ID NO: 153, SEQ ID NO: 155, SEQ ID NO: 161, SEQ ID NO: 168, SEQ ID NO: 170, SEQ ID NO: 172, SEQ ID NO: 174, and SEQ ID NO: 176.

On the other hand, the application provides a fusion polypeptide, the fusion polypeptide may comprise a first domain, a second domain and a third domain, the first domain can block PD-1/PD-L1 signal, the second domain may comprise LAG3 or a functionally active fragment thereof, and the third domain is capable of activating an immune response. For example, the fusion polypeptide may comprise one or more of the first domains, one or more of the second domains and one or more of the third domains.

For example, the first domain may comprise HCDR3 of an antibody heavy chain, wherein the antibody heavy chain may comprise an amino acid sequence selected from the group consisting of: SEQ ID NO: 3-9, and SEQ ID NO: 121.

For example, the first domain may comprise HCDR2 of an antibody heavy chain, which may comprise an amino acid sequence selected from the group consisting of: SEQ ID NO: 3-9, and SEQ ID NO: 121.

For example, the first domain may comprise HCDR1 of an antibody heavy chain, which may comprise an amino acid sequence selected from the group consisting of: SEQ ID NO: 3-9, and SEQ ID NO: 121.

For example, the first domain may comprise the heavy chain variable region VH of an antibody heavy chain, wherein the antibody heavy chain may comprise an amino acid sequence selected from the group consisting of SEQ ID NO: 3-9, and SEQ ID NO : 121.

For example, the first domain may comprise an antibody heavy chain, wherein the antibody heavy chain may comprise an amino acid sequence selected from the group consisting of SEQ ID NO: 3-9, and SEQ ID NO: 121.

For example, the first domain may comprise LCDR3 of an antibody light chain, wherein the antibody light chain may comprise an amino acid sequence selected from the group consisting of: SEQ ID NO: 162-167, and SEQ ID NO: 122.

For example, the first domain may comprise LCDR2 of an antibody light chain, wherein the antibody light chain may comprise an amino acid sequence selected from the group consisting of: SEQ ID NO: 162-167, and SEQ ID NO: 122.

For example, the first domain may comprise LCDR1 of an antibody light chain, wherein the antibody light chain may comprise an amino acid sequence selected from the group consisting of: SEQ ID NO: 162-167, and SEQ ID NO: 122.

For example, the first domain may comprise the light chain variable region VL of an antibody light chain, wherein the antibody light chain may comprise an amino acid sequence selected from the group consisting of: SEQ ID NO: 162-167, and SEQ ID NO : 122.

For example, the first domain may comprise an antibody light chain, which may comprise an amino acid sequence selected from the group consisting of: SEQ ID NO: 162-167, and SEQ ID NO: 122.

For example, the first domain may comprise HCDR3, HCDR2 and HCDR1 of an antibody heavy chain, wherein the antibody heavy chain may comprise the amino acid sequence shown in SEQ ID NO: 121. For example, the antibody can be Sugemalimab.

For example, the first domain may comprise LCDR3, LCDR2 and LCDR1 of an antibody heavy chain, wherein the antibody light chain may comprise the amino acid sequence shown in SEQ ID NO: 122. For example, the antibody can be Sugemalimab.

For example, the first domain may comprise HCDR3, HCDR2 and HCDR1 of an antibody heavy chain, wherein the antibody heavy chain may comprise an amino acid sequence as shown in SEQ ID NO: 121, and the first domain may comprise an LCDR3, LCDR2 and LCDR1 of the heavy chain, wherein **the light chain** of the antibody may comprise the amino acid sequence shown in SEQ ID NO: 122. For example, the antibody can be Sugemalimab.

For example, the first domain may comprise the heavy chain variable region VH of an antibody heavy chain, wherein the antibody heavy chain may comprise the amino acid sequence shown in SEQ ID NO: 121, and the first domain may comprise the light chain variable region VL of an antibody light chain, wherein the antibody light chain may comprise the amino acid sequence shown in SEQ ID NO: 122. For example, the antibody can be Sugemalimab.

For example, the first domain may comprise HCDR3, HCDR2 and HCDR1 of an antibody heavy chain, wherein the antibody heavy chain may comprise the amino acid sequence shown in SEQ ID NO:3. For example, the antibody can be JS003.

For example, the first domain may comprise LCDR3, LCDR2 and LCDR1 of an antibody heavy chain, wherein the antibody light chain may comprise the amino acid sequence shown in SEQ ID NO: 162. For example, the antibody can be JS003.

For example, the first domain may comprise HCDR3, HCDR2 and HCDR1 of an antibody heavy chain, wherein the antibody heavy chain may comprise an amino acid sequence as shown in SEQ ID NO: 3, and the first domain may comprise an LCDR3, LCDR2 and LCDR1 of the heavy chain, wherein **the light chain** of the antibody may comprise the amino acid sequence shown in SEQ ID NO: 162. For example, the antibody can be JS003.

For example, the first domain may comprise the heavy chain variable region VH of an antibody heavy chain, wherein the antibody heavy chain may comprise the amino acid sequence shown in SEQ ID NO: 3, and the first domain may comprise the light chain variable region VL of an antibody light chain, wherein the antibody light chain may comprise the amino acid sequence shown in SEQ ID NO: 162. For example, the antibody can be JS003.

For example, the first domain may comprise HCDR3, HCDR2 and HCDR1 of an antibody heavy chain, wherein the antibody heavy chain may comprise the amino acid sequence shown in SEQ ID NO:4. For example, the antibody can be Durvalumab.

For example, the first domain may comprise LCDR3, LCDR2 and LCDR1 of an antibody heavy chain, wherein the antibody light chain may comprise the amino acid sequence shown in SEQ ID NO: 163. For example, the antibody can be Durvalumab.

For example, the first domain may comprise HCDR3, HCDR2 and HCDR1 of an antibody heavy chain, wherein the antibody heavy chain may comprise an amino acid sequence as shown in SEQ ID NO: 4, and the first domain may comprise an LCDR3, LCDR2 and LCDR1 of the heavy chain, wherein **the light chain** of the antibody may comprise the amino acid sequence shown in SEQ ID NO: 163. For example, the antibody can be Durvalumab.

For example, the first domain may comprise the heavy chain variable region VH of an antibody heavy chain, wherein the antibody heavy chain may comprise the amino acid sequence shown in SEQ ID NO: 4, and the first domain may comprise the light chain variable region VL of an antibody light chain, wherein the antibody light chain may comprise the amino acid sequence shown in SEQ ID NO: 163. For example, the antibody can be Durvalumab.

For example, the first domain may comprise HCDR3, HCDR2 and HCDR1 of an antibody heavy chain, wherein the antibody heavy chain may comprise the amino acid sequence shown in SEQ ID NO:5. For example, the antibody can be atezolizumab.

For example, the first domain may comprise LCDR3, LCDR2 and LCDR1 of an antibody heavy chain, wherein the antibody light chain may comprise the amino acid sequence shown in SEQ ID NO: 164. For example, the antibody can be atezolizumab.

For example, the first domain may comprise HCDR3, HCDR2 and HCDR1 of an antibody heavy chain, wherein the antibody heavy chain may comprise an amino acid sequence as shown in SEQ ID NO: 5, and the first domain may comprise an LCDR3, LCDR2 and LCDR1 of the heavy chain, wherein **the light chain** of the antibody may comprise the amino acid sequence shown in SEQ ID NO: 164. For example, the antibody can be atezolizumab.

For example, the first domain may comprise the heavy chain variable region VH of an antibody heavy chain, wherein the antibody heavy chain may comprise the amino acid sequence shown in SEQ ID NO: 5, and the first domain may comprise the light chain variable region VL of an antibody light chain, wherein the antibody light chain may comprise the amino acid sequence shown in SEQ ID NO: 164. For example, the antibody can be atezolizumab.

For example, the first domain may comprise HCDR3, HCDR2 and HCDR1 of an antibody heavy chain, wherein the antibody heavy chain may comprise the amino acid sequence shown in SEQ ID NO:6. For example, the antibody can be Avelumab.

For example, the first domain may comprise LCDR3, LCDR2 and LCDR1 of an antibody heavy chain, wherein the antibody light chain may comprise the amino acid sequence shown in SEQ ID NO: 165. For example, the antibody can be Avelumab.

For example, the first domain may comprise HCDR3, HCDR2 and HCDR1 of an antibody heavy chain, wherein the antibody heavy chain may comprise an amino acid sequence as shown in SEQ ID NO: 6, and the first domain may comprise an LCDR3, LCDR2 and LCDR1 of the heavy chain, wherein **the light chain** of the antibody may comprise the amino acid sequence shown in SEQ ID NO: 165. For example, the antibody can be Avelumab.

For example, the first domain may comprise the heavy chain variable region VH of an antibody heavy chain, wherein the antibody heavy chain may comprise the amino acid sequence shown in SEQ ID NO: 6, and the first domain may comprise the light chain variable region VL of an antibody light chain, wherein the antibody light chain may comprise the amino acid sequence shown in SEQ ID NO: 165. For example, the antibody can be Avelumab.

For example, the first domain may comprise HCDR3, HCDR2 and HCDR1 of an antibody, wherein the antibody may comprise the amino acid sequence shown in SEQ ID NO:7. For example, the antibody can be Envafolimab.

For example, the first domain may comprise HCDR3, HCDR2 and HCDR1 of an antibody heavy chain, wherein the antibody heavy chain may comprise the amino acid sequence shown in SEQ ID NO:8. For example, the antibody can be Permbrolizumab.

For example, the first domain may comprise LCDR3, LCDR2 and LCDR1 of an antibody heavy chain, wherein the antibody light chain may comprise the amino acid sequence shown in SEQ ID NO: 166. For example, the antibody can be Permbrolizumab.

For example, the first domain may comprise HCDR3, HCDR2 and HCDR1 of an antibody heavy chain, wherein the antibody heavy chain may comprise an amino acid sequence as shown in SEQ ID NO: 8, and the first domain may comprise an LCDR3, LCDR2 and LCDR1 of the heavy chain, wherein **the light chain** of the antibody may comprise the amino acid sequence shown in SEQ ID NO: 166. For example, the antibody can be Permbrolizumab.

For example, the first domain may comprise the heavy chain variable region VH of an antibody heavy chain, wherein the antibody heavy chain may comprise the amino acid sequence shown in SEQ ID NO: 8, and the first domain may comprise the light chain variable region VL of an antibody light chain, wherein the antibody light chain may comprise the amino acid sequence shown in SEQ ID NO: 166. For example, the antibody can be Permbrolizumab.

For example, the first domain may comprise HCDR3, HCDR2 and HCDR1 of an antibody heavy chain, wherein the antibody heavy chain may comprise the amino acid sequence shown in SEQ ID NO:9. For example, the antibody can be Nivolumab.

For example, the first domain may comprise LCDR3, LCDR2 and LCDR1 of an antibody heavy chain, wherein the antibody light chain may comprise the amino acid sequence shown in SEQ ID NO: 167. For example, the antibody can be Nivolumab.

For example, the first domain may comprise HCDR3, HCDR2 and HCDR1 of an antibody heavy chain, wherein the antibody heavy chain may comprise an amino acid sequence as shown in SEQ ID NO: 9, and the first domain may comprise an LCDR3, LCDR2 and LCDR1 of the heavy chain, wherein **the light chain** of the antibody may comprise the amino acid sequence shown in SEQ ID NO: 167. For example, the antibody can be Nivolumab.

For example, the first domain may comprise the heavy chain variable region VH of an antibody heavy chain, wherein the antibody heavy chain may comprise the amino acid sequence shown in SEQ ID NO: 9, and the first domain may comprise the light chain variable region VL of an antibody light chain, wherein the antibody light chain may comprise the amino acid sequence shown in SEQ ID NO: 167. For example, the antibody can be Nivolumab.

For example, the first domain can bind to PD-L1 and/or PD-1.

For example, the first domain may comprise an antibody or antigen-binding fragment thereof.

For example, the antibody may be selected from the group consisting of recombinant antibodies, single domain antibodies, heavy chain antibodies, chimeric antibodies and bispecific antibodies.

For example, the antigen-binding fragment may be selected from one or more of the group consisting of: Fab, Fab', Fv fragment, F(ab')2, F(ab)2, scFv, di-scFv, VHH and dAb.

For example, the second domain may be selected from the group consisting of human-derived LAG3 or a functionally active fragment thereof and mouse-derived LAG3 or a functionally active fragment thereof.

For example, the second domain can bind an MHCII molecule on an antigen presenting cell.

For example, the second domain may comprise the extracellular domain of LAG3 or a functionally active fragment thereof.

For example, the second domain may comprise the amino acid sequence shown in SEQ ID NO:11.

For example, said second domain may comprise IgD1, IgD2, IgD3 and/or IgD4 of LAG3 or a functionally active fragment thereof.

For example, the second domain may comprise IgD1, IgD1-IgD2, IgD1-IgD2-IgD3, and/or IgD1-IgD2-IgD3-IgD4 of LAG3 or a functionally active fragment thereof.

For example, the second domain may comprise an amino acid sequence selected from the group consisting of: SEQ ID NO: 13, SEQ ID NO: 14, SEQ ID NO: 15, SEQ ID NO: 16, SEQ ID NO: 17, SEQ ID NO: 18, SEQ ID NO: 19, and SEQ ID NO: 12.

For example, the third domain may comprise CD80 or a functionally active fragment thereof.

For example, the third domain may be selected from the group consisting of human-derived CD80 or a functionally active fragment thereof and mouse-derived CD80 or a functionally active fragment thereof.

For example, the third domain can bind CD28.

For example, the third domain may comprise the extracellular domain of CD80 or a functionally active fragment thereof.

For example, the third domain may comprise the amino acid sequence shown in SEQ ID NO:2.

For example, the first domain is directly or indirectly linked to the second domain.

For example, the first domain may comprise an antibody heavy chain, the antibody heavy chain of the first domain being directly or indirectly linked to the second domain.

For example, the first domain may comprise an antibody light chain, and the antibody light chain of the first domain may be directly or indirectly linked to the second domain.

For example, the first domain and the third domain may be directly or indirectly linked.

For example, the first domain may comprise an antibody heavy chain, and the antibody heavy chain of the first domain may be directly or indirectly linked to the third domain.

For example, the first domain may comprise an antibody light chain, and the antibody light chain of the first domain may be directly or indirectly linked to the third domain.

For example, the second domain and the third domain may be directly or indirectly linked.

For example, the N-terminus of the first domain and the second domain may be linked directly or indirectly.

For example, the C-terminus of the first domain and the second domain may be linked directly or indirectly.

For example, the first domain may comprise an antibody light chain, and the antibody light chain of the first domain may be directly or indirectly linked to the N-terminus of the second domain.

For example, the first domain may comprise an antibody light chain, and the antibody light chain of the first domain may be directly or indirectly linked to the C-terminus of the second domain.

For example, the first domain may comprise an antibody heavy chain, and the antibody heavy chain of the first domain may be directly or indirectly linked to the N-terminus of the second domain.

For example, the first domain may comprise an antibody heavy chain, and the antibody heavy chain of the first domain may be directly or indirectly linked to the C-terminus of the second domain.

For example, the N-terminus of the first domain and the third domain may be directly or indirectly linked.

For example, the C-terminus of the first domain and the third domain may be directly or indirectly linked.

For example, the first domain may comprise an antibody light chain, and the antibody light chain of the first domain may be directly or indirectly linked to the N-terminus of the third domain.

For example, the first domain may comprise an antibody light chain, and the antibody light chain of the first domain may be directly or indirectly linked to the C-terminus of the third domain.

For example, the first domain may comprise an antibody heavy chain, and the antibody heavy chain of the first domain may be directly or indirectly linked to the N-terminus of the third domain.

For example, the first domain may comprise an antibody heavy chain, and the antibody heavy chain of the first domain may be directly or indirectly linked to the C-terminus of the third domain.

For example, the N-terminus of the second domain and the third domain may be directly or indirectly linked.

For example, the C-terminus of the second domain and the third domain may be directly or indirectly linked.

For example, the N-terminus of the first domain and the second domain may be directly or indirectly linked, and the N-terminus of the second domain and the third domain may be directly or indirectly linked.

For example, the N-terminus of the first domain and the third domain may be directly or indirectly linked, and the third domain and the N-terminus of the second domain may be directly or indirectly linked.

For example, the first domain and the C-terminus of the second domain may be directly or indirectly linked, and the first domain and the N-terminus of the third domain may be directly or indirectly linked.

For example, the first domain and the C-terminus of the third domain may be directly or indirectly linked, and the first domain and the N-terminus of the second domain may be directly or indirectly linked.

For example, said indirect link can comprise linking through a linker.

For example, the linker may comprise an amino acid sequence selected from the group consisting of SEQ ID NO:22, SEQ ID NO:23, SEQ ID NO:24, SEQ ID NO:25, and SEQ ID NO:26.

For example, the fusion polypeptide may comprise an amino acid sequence selected from the group consisting of: SEQ ID NO: 35-42, SEQ ID NO: 51-58, SEQ ID NO: 67-74, SEQ ID NO: 83-90, SEQ ID NO: 97-104, SEQ ID NO: 113-120, SEQ ID NO: 147, SEQ ID NO: 149, SEQ ID NO: 153, SEQ ID NO: 155, SEQ ID NO: 168, SEQ ID NO: 170, SEQ ID NO:172, SEQ ID NO:174, and SEQ ID NO:176.

In one aspect, the application discloses dual and/or multifunctional fusion polypeptides comprising double chain fusion polypeptides. As shown in Figure 1, the fusion polypeptide may include the first domain that specifically binds to the first cell targetcomposed of (i) antigen binding domain, (ii)Fc domain (present or absent) and/or (iii) second domain that specifically binds to the second cell target and/or (iv) the third domain that specifically binds to the third cell target, wherein the second domain binding to the second cell target through first domain polypeptide C-terminal, or through first domain polypeptide N-terminal via linker peptide is covalently linked to the first domain, wherein the third cell target bind to the third domain through first domain polypeptide C-terminal, or through second domain C-terminal, or through first domain polypeptide N-terminal via linker peptide is covalently linked to the corresponding domain; wherein it should be noted that the first domain disclosed in this application may be extended to some anti-PD-L1 and/or anti-PD-1 antibody without Fc domain including but not limited to single domain antibodies, recombinant antibodies and single chain antibodies, etc. The case that C-terminal of heavy chain polypeptide or light chain polypeptide of the first domain via linker peptide is covalently linked to the second domain and/or the third domain, should be regarded as the scope of the claims of this application; similarily, The case that N-terminal of heavy chain polypeptide or light chain polypeptide of the first domain via linker peptide is covalently linked to the second domain and/or the third domain should also be regarded as the scope of the claims of this application; thus the dual and/or multifunctional fusion polypeptide is capable of simultaneously binding to the first cell target, the second cell target and/or the third cell target.

In another aspect, (a) antigen-binding fragment included in the first binding domain of the dual/or multifunctional fusion protein of this application may be derived from any anti- PD-L1 and/or anti-PD-1 antibody, including any antibody that can block or reduce the binding of PD-L1 to its receptor and thereby relieve the immunosuppressive effect mediated by PD-L1, including currently published anti-PD-L1 and/or anti-PD-1 and anti-PD-L1 antibodies and/or anti-PD-1 antibodies developed in the future.

In another aspect, dual/or multifunctional fusion protein of the present application includes second domain comprising the extracellular domains of one and/or more ligands, which at least comprise one or more subnits of (b)CD80 extracellular domain, or (c) polypeptide composed of CD80 extracellular domain IgV region and IgC2 region, or (d)LAG3 extracellular domain, or (e) polypeptide composed of IgD1, IgD2, IgD3 and IgD4 in LAG3 extracellular domain. For example, LAG3 can be a type 1 transmembrane protein at immune checkpoints and can be mainly expressed on activated NK cells and T cells. LAG3 is expressed on tumor-exhausted CD8+ T cells and can be an effective target for tumor immunotherapy. For example, the function of LAG3 can have two sides. On the one hand, LAG3 can play a negative regulatory role in T cell proliferation and activation. LAG3 and its ligand histocompatibility complex II (MHCII) or ligand fibrinogen-like protein 1 ( FGL1) can participate in inhibiting the activation and function of T cells after binding; on the other hand, LAG3 can participate in inducing the activation of antigen-presenting cells by binding to MHCII molecules on antigen-presenting cells, such as up-regulating the expression of CD80/CD83/CD86, enhancing antigen presentation and secretion of chemokines to recruit T cells are involved in assisting T cell activation. Antibodies that antagonize LAG3 can directly relieve the effect of LAGS-mediated T cell inhibition, but at the same time block the activation of antigen-presenting cells mediated by LAG3, so they cannot fully enhance the LAG3-related immune activation effect. The LAG3 extracellular domain chimeric protein provided in this application can simultaneously take into account the effects of relieving T cell immune suppression and activating antigen-presenting cells, and may have potential tumor treatment prospects.

In another aspect, the dual and/or multifunctional fusion polypeptide of the present application includes the third binding domain including an extracellular region of one and/or multiple ligands, which at least includes one or more subunits of (b) the extracellular domain of CD80 domain, or (c) a polypeptide consisting of the IgV region and IgC2 region of the CD80 extracellular domain, or (d) the LAG3 extracellular domain, or (e) a polypeptide consisting of IgD1, IgD2, IgD3 and IgD4 in the LAG3 extracellular domain. For example, CD80 and CD86 can be members of the B7 molecule family of proteins that costimulate activated T cells. Mature CD80 and CD86 molecules can be composed of an extracellular domain (ECD), a transmembrane domain, and an intracellular domain respectively, wherein the extracellular domain (ECD) can be the key region for these molecules to bind to the corresponding receptors on T cells. The extracellular domains of CD80 and CD86 both contain immunoglobulin-like V (IgV) regions and immunoglobulin-like C2 ( IgC2) region, and the IgV domain can be the key domain directly involved in its receptor binding respectively; the IgV of CD80 and CD86 extracellular domains can combine with CD28 to participate in inducing T cell activation, proliferation and effector functions. CTLA4 can bind to the IgV region of CD80 and CD86 to participate in the regulation of immunosuppression.

In one aspect, the application provides a specific polypeptide complex that constitutes a double and/or multifunctional fusion polypeptide, and the specific polypeptide complex includes a first polypeptide and a second polypeptide that form the polypeptide complex, and it should be clear that in some particular antibodies the second polypeptide may be absent.

In another aspect, the second polypeptide of the polypeptide complex described herein is the first binding domain light chain.

In another aspect, the first polypeptide of the polypeptide complex described in the present application includes, in sequence from the N-terminus of the polypeptide to the C-terminus, a third binding domain and/or a second binding domain, a linker peptide, and a first binding domain heavy chain, The linker peptide is covalently connected to the third binding domain and/or the second binding domain.

In another aspect, the first polypeptide of the polypeptide complex described in the present application includes sequentially from the N-terminus to the C-terminus of the polypeptide, the first binding domain heavy chain, the linker peptide, the second binding domain, the linker peptide and the third binding domain covalently linked.

In another aspect, the polypeptide complex described in the present application only includes the structure of the first polypeptide, and its structure includes in sequence from the N-terminal to the C-terminal of the polypeptide, the first domain heavy chain, the linker peptide, the second domain, and the linker The peptide and the third domain are covalently connected, or the heavy chain of the first binding domain, the linker peptide, the second binding domain, the linker peptide and the third binding domain are covalently linked.

In another aspect, the second polypeptide of the polypeptide complex described in the present application in sequence from the N-terminal to the C-terminal of the polypeptide includes the third domain and/or the second domain, linker peptide, the first domain light chain covalently linked.

In one aspect, the application provides polynucleotide sequences encoding the polypeptide complexes described herein.

In one aspect, the present application provides an assembleable vector comprising the polynucleotide described herein.

In one aspect, the present application provides a host cell comprising the polynucleotide described in the present application for encoding the polypeptide complex or the vector described in the present application.

In one aspect, the present application provides a method for expressing the polypeptide complex, and the expression method includes the host cell used for expressing the antibody-polypeptide complex described in the present application.

In one aspect, the application provides a method for producing the polypeptide complex: the method includes transferring the polynucleotide encoding the antibody-polypeptide complex described in the application into a host cell, expressing the first polypeptide, and/or the second polypeptide, wherein the first polypeptide and the second polypeptide, and the first polypeptide itself can form a stable dimer, and the stable dimer may contain natural bonds or/and at least one non-natural interchain bonds to maintaine the stable dimerization, said first polypeptide and second polypeptide are assembled into a stable polymer in a host cell, and form said Stable complexes of third cellular targets.

The following provides examples wherein an exemplary anti-PD-L1 antibody sugemalimab is linked to a linker peptide through the Fc domain, and the linker peptide is covalently linked to the CD80 extracellular domain to form the bifunctional fusion polypeptide first polypeptide amino acid sequence (SEQ ID NO: 27), sugemalimab is linked to a linker peptide through the Fc domain, and the linker peptide is covalently linked to the extracellular domain of LAG3 to form the bifunctional fusion polypeptide first polypeptide amino acid sequence (SEQ ID NO: 28), sugemalimab is linked to the linker peptide through the Fc domain, and the linker peptide is covalently linked to the IgD1/IgD2 domain of LAG3 extracellular domain to form the bifunctional fusion polypeptide first polypeptide amino acid sequence (SEQ ID NO: 29), sugemalimab is linked to a linker peptide through the Fc domain, and the linker peptide is covalently linked to the IgD1/IgD2/IgD3 domain of the extracellular domain of LAG3 to form the fusion polypeptide first polypeptide amino acid sequence (SEQ ID NO: 30), sugemalimab is linked to the linker peptide through the N-terminal of the heavy chain of the antigen-binding region, and the linker peptide is covalently linked to the extracellular domain of CD80 to form the amino acid sequence of the first polypeptide of the bifunctional fusion polypeptide (SEQ ID NO: 31), sugemalimab is linked to the linker peptide through the N-terminal of the heavy chain of the antigen-binding region, and the linker peptide is covalently linked to the LAG3 extracellular domain to form the amino acid sequence of the first polypeptide of the bifunctional fusion polypeptide (SEQ ID NO: 32), sugemalimab is linked to the linker peptide through the light chain N-terminal of the antigen-binding region, and the linker peptide is covalently linked to the extracellular domain of CD80 to form the second polypeptide amino acid sequence of the bifunctional fusion polypeptide fusion polypeptide (SEQ ID NO: 33), sugemalimab is linked to the linker peptide through the light chain N-terminal of the antigen-binding region, and the linker peptide is covalently linked to the extracellular domain of LAG3 to form the amino acid sequence of the second polypeptide of the bifunctional fusion polypeptide (SEQ ID NO: 34), sugemalimab via the Fc domain is linked to the linker peptide covalently linked to the extracellular domain of CD80, and the extracellular domain of CD80 is linked to the linker peptide linked in series to the IgD 1/IgD2 domain of the extracellular domain of LAG3 to form the first polypeptide amino acid sequence (SEQ ID NO: 35) of the multifunctional fusion polypeptide, sugemalimab via the Fc domain is linked to the linker peptide covalently linked to the CD80 extracellular domain, the CD80 extracellular domain is linked to the linker peptide linked in series to the IgD1/IgD2/IgD3 domain of the extracellular domain of LAG3 to form the multifunctional fusion polypeptide first polypeptide amino acid sequence (SEQ ID NO: 36), sugemalimab via the Fc domain is linked to the linker peptide covalently linked to extracellular domain of CD80, which is linked to the linker peptide linked in series to the LAG3 extracellular domain to form the multifunctional fusion polypeptide first polypeptide amino acid sequence (SEQ ID NO: 37), sugemalimab via the Fc domain is linked to the linker peptide covalently linked to the IgD1/IgD2 domain of LAG3 extracellular domain, which is linked to the linker peptide linked in series to the CD80 extracellular domain to form the multifunctional fusion polypeptide first polypeptide amino acid sequence (SEQ ID NO: 38), sugemalimab via the Fc domain is linked to the linker peptide covalently linked to IgD1/IgD2/IgD3 domain of the LAG3 extracellular domain, which is linked to the linker peptide linked in series to CD80 extracellular domain to form the multifunctional fusion polypeptide first polypeptide amino acid sequence (SEQ ID NO: 39), sugemalimab via Fc domain is linked to the linker peptide covalently linked to LAG3 extracellular domain, which is linked to linker peptide linked in series to CD80 extracellular domain to form the multifunctional fusion polypeptide first polypeptide amino acid sequence (SEQ ID NO: 40), sugemalimab via the N-terminal of the heavy chain of the antigen-binding region is linked to linker peptide, and the linker peptide is covalently linked to the extracellular domain of LAG3, and sugemalimab via the Fc domain is linked to the linker peptide covalently linked to the extracellular domain of CD80 to form the first polypeptide amino acid sequence of the multifunctional fusion polypeptide (SEQ ID NO: 41), sugemalimab via the N-terminal of the heavy chain of the antigen-binding region is linked to the linker peptide, and the linker peptide is covalently linked to the extracellular domain of CD80, and sugemalimab via the Fc domain is linked to linker peptide, and the linker peptide is covalently linked to LAG3 extracellular domain to form multifunctional fusion polypeptide first polypeptide amino acid sequence (SEQ ID NO: 42). The first polypeptide is assembled with the second polypeptide composed of sugemalimab light chain polypeptide (SEQ ID NO: 122) to form the double/or multifunctional fusion protein; the second polypeptide is assembled with the first polypeptide (SEQ ID NO: 121) composed of sugemalimab heavy chain polypeptide into the bi/or multifunctional fusion protein.

The following provides examples wherein an exemplary anti-PD-L1 antibody Durvalumab through the Fc domain linker peptide covalently linked to the CD80 extracellular domain bifunctional fusion polypeptide ammonia first polypeptide amino acid sequence (SEQ ID NO: 43), Durvalumab through The amino acid sequence of the first polypeptide (SEQ ID NO: 44) of the bifunctional fusion polypeptide covalently linked to the extracellular domain of LAG3 by the Fc domain linker peptide, Durvalumab is linked to linker peptide through the Fc domain, and the linker peptide is covalently linked to the IgD1 /IgD2 domain of LAG3 extracellular domain to form the bifunctional fusion polypeptide first polypeptide amino acid sequence (SEQ ID NO: 45), Durvalumab is linked to linker peptide through the Fc domain, and the linker peptide is covalently linked to the IgD1/IgD2/IgD3 domain of the LAG3 extracellular domain to form the functional fusion polypeptide first polypeptide amino acid sequence (SEQ ID NO: 46), Durvalumab is linked to linker peptide through the the N-terminal connection of the heavy chain of the antigen-binding region, and the linker peptide is covalently linked to the CD80 extracellular domain form the first polypeptide amino acid sequence of the bifunctional fusion polypeptide (SEQ ID NO: 47), Durvalumab is linked to linker peptide through the N-terminal linker peptide of the heavy chain of the antigen-binding region, and the linker peptide is covalently linked to the LAG3 extracellular domain form the bifunctional fusion polypeptide first polypeptide amino acid sequence (SEQ ID NO: 48), Durvalumab through the light chain N-terminal of the antigen-binding region is linked to the linker peptide covalently linked to the CD80 extracellular domain to form the second polypeptide amino acid sequence of the fusion polypeptide (SEQ ID NO: 49), Durvalumab via the N-terminal of the light chain of the antigen-binding region is linked to linker peptide covalently linked to the extracellular domain of LAG3 to form the amino acid sequence of the second polypeptide of the bifunctional fusion polypeptide (SEQ ID NO: 50), Durvalumab via the Fc domain is linked to the peptide covalently linked to the CD80 extracellular domain, which is linked to the linker peptide linked in series to the IgD1/IgD2 domain of the LAG3 extracellular domain to form multifunctional fusion polypeptide first polypeptide amino acid sequence (SEQ ID NO: 51), Durvalumab via the Fc domain is linked to the linker peptide covalently linked to the CD80 extracellular domain, which is linked to the linker peptide linked in series to the IgD1/IgD2/IgD3 domain of the LAG3 extracellular domain to form the multifunctional fusion polypeptide first polypeptide amino acid sequence (SEQ ID NO: 52), Durvalumab via the Fc domain is linked to the linker peptide covalently linked to LAG3 extracellular domain, which is linked to the linker peptide linked in series to the CD80 extracellular domain to form the multifunctional fusion polypeptide first polypeptide amino acid sequence (SEQ ID NO: 53), Durvalumab via the Fc domain is linked to the linker peptide covalently linked to the IgD1/IgD2 domain of LAG3 extracellular domain, which is linked to the linker peptide linked in series to the CD80 extracellular domain to form the multifunctional fusion polypeptide first polypeptide amino acid sequence (SEQ ID NO: 54), Durvalumab via the Fc domain is linked to the linker peptide covalently linked to IgD1/IgD2/IgD3 domain of the LAG3 extracellular domain, which is linked to the linker peptide linked in series to CD80 extracellular domain to form the multifunctional fusion polypeptide first polypeptide amino acid sequence (SEQ ID NO: 55), Durvalumab via the Fc domain is linked to the linker peptide covalently linked to CD80 extracellular domain, which is linked to the linker peptide linked in series to LAG3 extracellular domain to form the first polypeptide amino acid sequence of the multifunctional fusion polypeptide (SEQ ID NO: 56), Durvalumab via the N-terminal of the heavy chain of the antigen-binding region is linked to a linker peptide, and the linker peptide is covalently linked to the extracellular domain of LAG3, and Durvalumab via the Fc domain is linked to a linker peptide, and the linker peptide is linked to the extracellular domain of CD80 to form the first polypeptide amino acid sequence of the multifunctional fusion polypeptide (SEQ ID NO: 57), Durvalumab is linked to a linker peptide through the N-terminal linker peptide of the heavy chain of the antigen-binding region, and the linker peptide is covalently linked to the extracellular domain of CD80, and Durvalumab via the Fc Domain is linked to a linker peptide, and the linker peptide is linked to LAG3 extracellular domain to form multifunctional fusion polypeptide first polypeptide amino acid sequence (SEQ ID NO: 58). The first polypeptide and the second polypeptide (SEQ ID NO: 163) composed of the Durvalumab light chain polypeptide are assembled into the double/or multifunctional fusion protein; the second polypeptide and the first polypeptide composed of Durvalumab heavy chain polypeptide SEQ ID NO: 4) are assembled into the dual/or multifunctional fusion protein.

The following provides examples wherein an exemplary anti-PD-L1 antibody Atezolizumab is linked to linker peptide through Fc domain, and the linker peptide is , and the linker peptide is covalently linked to CD80 extracellular domain to form bifunctional fusion polypeptide first polypeptide amino acid sequence (SEQ ID NO: 59), Atezolizumab via Fc domain is linked to the linker peptide covalently linked to LAG3 extracellular domain to form bifunctional fusion polypeptide first polypeptide amino acid sequence (SEQ ID NO: 60), Atezolizumab via Fc domain is linked to linker peptide covalently linked to IgD1/IgD2domain of LAG3 extracellular domain to form bifunctional fusion polypeptide first polypeptide amino acid sequence (SEQ ID NO: 61), Atezolizumab via Fc domain is linked to linker peptide covalently linked to IgD1/IgD2/IgD3 domain of LAG3 extracellular domain to form bifunctional fusion polypeptide first polypeptide amino acid sequence (SEQ ID NO: 62), Atezolizumab is linked to linker peptide through antigen binding domain heavy chain N-terminal, and the linker peptide is covalently linked CD80 extracellular domain to form bifunctional fusion polypeptide first polypeptide amino acid sequence (SEQ ID NO: 63), Atezolizumab is linked to linker peptide through antigen binding domain heavy chain N-terminal, and the linker peptide is covalently linked to LAG3 extracellular domain to form bifunctional fusion polypeptide first polypeptide amino acid sequence (SEQ ID NO: 64), Atezolizumab is linked to linker peptide through antigen binding domain light chain N-terminal, and the linker peptide is covalently linked to CD80 extracellular domain to form bifunctional fusion polypeptide second polypeptide amino acid sequence (SEQ ID NO: 65), Atezolizumab is linked to linker peptide through antigen binding domain light chain N-terminal, and the linker peptide is covalently linked to LAG3 extracellular domain to form bifunctional fusion polypeptidesecond polypeptide amino acid sequence (SEQ ID NO: 66), Atezolizumab is linked to linker peptide through Fc domain, and the linker peptide is covalently linked to CD80 extracellular domain, and the CD80 extracellular domain is linked in series via linker peptide to IgD1/IgD2 domain of LAG3 extracellular domain to form multifunctional fusion polypeptide first polypeptide amino acid sequence (SEQ ID NO: 67), Atezolizumab is linked to linker peptide through Fc domain, and the linker peptide is covalently linked to CD80 extracellular domain, and the CD80 extracellular domain is linked in series via linker peptide to IgD1/IgD2/IgD3 domain of LAG3 extracellular domain to form multifunctional fusion polypeptide first polypeptide amino acid sequence (SEQ ID NO: 68), Atezolizumab is linked to linker peptide through Fc domain, and the linker peptide is covalently linked to CD80 extracellular domain, and the CD80 extracellular domain is linked in series via linker peptide to LAG3 extracellular domain to form multifunctional fusion polypeptide first polypeptide amino acid sequence (SEQ ID NO: 69), Atezolizumab is linked to linker peptide through Fc domain, and the linker peptide is covalently linked to IgD1/IgD2 domain of LAG3 extracellular domain, which is linked to linker peptide linked in series to CD80 extracellular domain to form multifunctional fusion polypeptide first polypeptide amino acid sequence (SEQ ID NO: 70), Atezolizumab is linked to linker peptide through Fc domain, and the linker peptide is covalently linked to IgD1/IgD2/IgD3 domain of LAG3 extracellular domain, which is linked to linker peptide linked in series to CD80 extracellular domain to form multifunctional fusion polypeptide first polypeptide amino acid sequence (SEQ ID NO: 71), Atezolizumab is linked to linker peptide through Fc domain, and the linker peptide is covalently linked to LAG3 extracellular domain, and the LAG3 extracellular domain is linked in series via linker peptide to CD80 extracellular domain to form multifunctional fusion polypeptide first polypeptide amino acid sequence (SEQ ID NO: 72), Atezolizumab via antigen binding domain heavy chain N-terminal is linked to linker peptide covalently linked to LAG3 extracellular domain, and Atezolizumab via Fc domain is linked to CD80 extracellular domain via linker peptide to form multifunctional fusion polypeptide first polypeptide amino acid sequence (SEQ ID NO: 73), Atezolizumab via antigen binding domain heavy chain N-terminal is linked to the linker peptide covalently linked to CD80 extracellular domain, and Atezolizumab via Fc domain is linked to the linker peptide linked in series to LAG3 extracellular domain to form multifunctional fusion polypeptide first polypeptide amino acid sequence (SEQ ID NO: 74). The first polypeptide is assembled with the second polypeptide (SEQ ID NO: 164) composed of the light chain polypeptide of Atezolizumab to form the double/or multifunctional fusion protein; the second polypeptide is assembled with the first polypeptide (SEQ ID NO: 5) composed of the heavy chain polypeptide of Atezolizumab to form the dual/or multifunctional fusion protein.

The following provides examples wherein an exemplary anti-PD-L1 antibody Avelumab Fc domain is covalently linked to the CD80 extracellular domain through the linker peptide to form bifunctional fusion polypeptide first polypeptide amino acid sequence (SEQ ID NO: 75), Avelumab Fc domain is covalently linked to the extracellular domain of LAG3 by the linker peptide to form first polypeptide amino acid sequence (SEQ ID NO: 76) of the bifunctional fusion polypeptide, Avelumab Fc domain is covalently linked to the IgD1/IgD2 domain of the LAG3 extracellular domain through the linker peptide to form the bifunctional fusion polypeptide first polypeptide amino acid sequence (SEQ ID NO: 77), Avelumab Fc domain is covalently linked to the IgD1/IgD2/IgD3 domain of the extracellular domain of LAG3 through the linker peptide to form the bifunctional fusion polypeptide first polypeptide amino acid sequence (SEQ ID NO: 78), the N-terminal of the heavy chain of the antigen-binding region of Avelumab via linker peptide is covalently linked to the extracellular domain of CD80 to form the amino acid sequence of the first polypeptide of the bifunctional fusion polypeptide (SEQ ID NO: 79), Avelumab via the N-terminal of the heavy chain of the antigen-binding region is linked to the linker peptide covalently linked to the the extracellular domain of LAG3 to form the bifunctional fusion polypeptide first polypeptide amino acid sequence (SEQ ID NO: 80), Avelumab is covalently linked to the linker peptide through the N-terminal of the light chain of the antigen-binding region, and the linker peptide is covalently linked to the CD80 extracellular domain to form the bifunctional fusion polypeptide second polypeptide amino acid sequence (SEQ ID NO: 81), Avelumab is linked to the linker peptide by the N-terminal of the light chain of the antigen-binding region, and the linker peptide is covalently linked to the extracellular domain of LAG3 to form the amino acid sequence of the second polypeptide (SEQ ID NO: 82) of the bifunctional fusion polypeptide, Avelumab is linked to the linker peptide through the Fc domain and the linker peptide is covalently linked to the extracellular domain of CD80, and the extracellular domain of CD80 is linked to the IgD1/IgD2 domain of the LAG3 extracellular domain in series via the linker peptide to form the multifunctional fusion polypeptide first polypeptide amino acid sequence (SEQ ID NO: 83), Avelumab through the Fc domain linker peptide is linked to the linker peptide covalently linked to the CD80 extracellular domain, which is linked to the linker peptide linked in series to the IgD1/IgD2/IgD3 domain of the LAG3 extracellular domain to form the multifunctional fusion polypeptide first polypeptide amino acid sequence (SEQ ID NO: 84), Avelumab via the Fc domain is linked to the linker peptide covalently linked to the CD80 extracellular domain, which is linked to the linker peptide linked in series to the LAG3 extracellular domain to form the first polypeptide amino acid sequence of the multifunctional fusion polypeptide(SEQ ID NO: 85), Avelumab through the Fc domain is linked to the linker peptide covalently linked to the IgD1/IgD2 domain of LAG3 extracellular domain, which is linked to the linker peptide linked in series to the CD80 extracellular domain to form the first polypeptide amino acid sequence of the multifunctional fusion polypeptide (SEQ ID NO: 86), Avelumab through the Fc domain is linked to the linker peptide covalently linked to IgD1/IgD2/IgD3 domain of the LAG3 extracellular domain, which is linked to the linker peptide linked in series to the CD80 extracellular domain to form the multifunctional fusion polypeptide first polypeptide amino acid sequence (SEQ ID NO: 87), Avelumab is linked to linker peptidd through the Fc domain, and the linker peptide is covalently linked to LAG3 extracellular domain, and the LAG3 extracellular domain is linked in series to CD80 extracellular domain via linker peptide to form the multifunctional fusion polypeptide first polypeptide amino acid sequence (SEQ ID NO: 88), Avelumab via the N-terminal of the heavy chain of the antigen-binding domain is linked to the linker peptide covalently linked to the extracellular domain of LAG3, and Avelumab via the Fc domain is linked to the linker peptide covalently linked to the extracellular domain of CD80 to form the multifunctional fusion polypeptide first polypeptide amino acid sequence (SEQ ID NO: 89), Avelumab via the N-terminal of the heavy chain of the antigen-binding domain is linked to the linker peptide covalently linked to the extracellular domain of CD80, and Avelumab via the Fc domain is linked to the linker peptide covalently linked to LAG3 extracellular domain to form the multifunctional fusion polypeptide first polypeptide amino acid sequence (SEQ ID NO: 90). The first polypeptide is assembled with the second polypeptide (SEQ ID NO: 165) composed of the Avelumab light chain polypeptide to form the double/or multifunctional fusion protein; the second polypeptide is assembled with the first polypeptide (SEQ ID NO: 6) composed of the Avelumab heavy chain polypeptide to form the dual/or multifunctional fusion protein.

The following provides examples wherein an exemplary anti-PD-L1 antibody Envafolimab is linked to the linker peptide through the Fc domain, and the linker peptide is covalently linked to the CD80 extracellular domain to form the bifunctional fusion polypeptide amino acid sequence (SEQ ID NO: 91), Envafolimab is linked to the linker peptide through the Fc domain, and the linker peptide is covalently linked to the extracellular domain of LAG3 to form the bifunctional fusion polypeptide amino acid sequence (SEQ ID NO: 92), Envafolimab is linked to the linker peptide through the Fc domain, and the linker peptide is covalently linked the IgD1/IgD2 domain of the extracellular structure of LAG3 to form the bifunctional fusion polypeptide amino acid sequence (SEQ ID NO: 93), Envafolimab is linked to linker peptide through the Fc domain, and the linker peptide is covalently linked to the IgD1/IgD2/IgD3 domain of the extracellular domain of LAG3 to form the amino acid sequence of the bifunctional fusion polypeptide (SEQ ID NO: 94), Envafolimab is linked to the linker peptide through the N-terminal of the antigen-binding region, and the linker peptide is covalently linked to the extracellular domain of CD80 to form the bifunctional fusion polypeptide amino acid sequence (SEQ ID NO: 95), Envafolimab via the N-terminal of the antigen-binding region is linked to the linker peptide covalently linked to the LAG3 extracellular domain to form the bifunctional fusion polypeptide amino acid sequence (SEQ ID NO: 96), Envafolimab through the Fc domain is linked to the linker peptide covalently linked to the extracellular domain of CD80, which is linked to the linker peptide linked in series to the IgD1/IgD2 domain of the LAG3 extracellular domain to form the multifunctional fusion polypeptide amino acid sequence (SEQ ID NO: 97), Envafolimab through the Fc domain is linked to the linker peptide covalently linked to the CD80 extracellular domain, which is linked to the linker peptide linked in series to IgD1/IgD2/IgD3 domains of LAG3 extracellular domain to form the multifunctional fusion polypeptide amino acid sequence (SEQ ID NO: 98), Envafolimab through Fc domain is linked to the linker peptide covalently linked to CD80 extracellular domain, which is linked to the linker peptide linked in series to LAG3 extracellular domain to form the first polypeptide amino acid sequence of multifunctional fusion polypeptide (SEQ ID NO: 99), Envafolimab covalently connects the LAG3 extracellular structure IgD1/IgD2 domain linker peptide in series with the CD80 extracellular domain amino acid sequence (SEQ ID NO: 100) through the Fc domain linker peptide, Envafolimab covalently connects the LAG3 extracellular structure IgD1/IgD2/IgD3 domain linker peptide amino acid sequence (SEQ ID NO: 101) to the multifunctional fusion polypeptide of the extracellular domain of LAG3 through the Fc domain linker peptide (SEQ ID NO: 101). The amino acid sequence of a multifunctional fusion polypeptide (SEQ ID NO: 102) that covalently connects the LAG3 extracellular domain linker peptide with the extracellular domain linker peptide in series (SEQ ID NO: 102), Envafolimab via the N-terminal of the antigen-binding region is linked to the linker peptide covalently linked to the LAG3 extracellular domain, and Envafolimab via the Fc domain is linked to the linker peptide that is linked to the CD80 extracellular domain to form the amino acid sequence of the multifunctional fusion polypeptide (SEQ ID NO: 103), Envafolimab via the N-terminal of the antigen-binding region is linked to the linker peptide covalently linked to the CD80 extracellular domain, and Envafolimab via Fc domain is linked to the linker peptide that is covalently linked the LAG3 extracellular domain to form the amino acid sequence of the multifunctional fusion polypeptide (SEQ ID NO: 104).

Provided below are examples wherein exemplary anti-PD-1 antibody Nivolumab is linked to linker peptide through Fc domain, and the linker peptide is covalently linked to CD80 extracellular domain to form bifunctional fusion polypeptide first polypeptide amino acid sequence (SEQ ID NO: 105), Nivolumab is linked to linker peptide through Fc domain, and the linker peptide is covalently linked to LAG3 extracellular domain to form bifunctional fusion polypeptide first polypeptide amino acid sequence (SEQ ID NO: 106), Nivolumab is linked to linker peptide through Fc domain, and the linker peptide is covalently linked to IgD1/IgD2 domain of LAG3 extracellular domainto form bifunctional fusion polypeptide first polypeptide amino acid sequence (SEQ ID NO: 107), Nivolumab is linked to linker peptide through Fc domain, and the linker peptide is covalently linked to IgD1/IgD2/IgD3 domain of LAG3 extracellular domain to form bifunctional fusion polypeptide first polypeptide amino acid sequence (SEQ ID NO: 108), Nivolumab is linked to linker peptide through antigen binding domain heavy chain N-terminal, and the linker peptide is covalently linked to CD80 extracellular domain to form bifunctional fusion polypeptide first polypeptide amino acid sequence (SEQ ID NO: 109), Nivolumab is linked to linker peptide through antigen binding domain heavy chain N-terminal, and the linker peptide is covalently linked to LAG3 extracellular domain to form bifunctional fusion polypeptide first polypeptide amino acid sequence (SEQ ID NO: 110), Nivolumab is linked to linker peptide through antigen binding domain light chain N-terminal, and the linker peptide is covalently linked to CD80 extracellular domain to form bifunctional fusion polypeptide second polypeptide amino acid sequence (SEQ ID NO: 111), Nivolumab is linked to linker peptide through antigen binding domain light chain N-terminal, and the linker peptide is covalently linked to LAG3 extracellular domain to form bifunctional fusion polypeptidesecond polypeptide amino acid sequence (SEQ ID NO: 112), Nivolumab is linked to linker peptide through Fc domain, and the linker peptide is covalently linked to CD80 extracellular domain, and the CD80 extracellular domain is linked in series via linker peptide to IgD1/IgD2 domain of LAG3 extracellular domain to form multifunctional fusion polypeptide first polypeptide amino acid sequence (SEQ ID NO: 113), Nivolumab is linked to linker peptide through Fc domain, and the linker peptide is covalently linked to CD80 extracellular domain, and the CD80 extracellular domain is linked in series via linker peptide to IgD1/IgD2/IgD3 domain of LAG3 extracellular domain to form multifunctional fusion polypeptide first polypeptide amino acid sequence (SEQ ID NO: 114), Nivolumab is linked to linker peptide through Fc domain, and the linker peptide is covalently linked to CD80 extracellular domain, and the CD80 extracellular domain is linked in series via linker peptide to LAG3 extracellular domain to form multifunctional fusion polypeptide first polypeptide amino acid sequence (SEQ ID NO: 115), Nivolumab is linked to linker peptide through Fc domain, and the linker peptide is covalently linked to IgD1/IgD2 domain of LAG3 extracellular domain, which is linked to the linker peptide linked in series to CD80 extracellular domain to form multifunctional fusion polypeptide first polypeptide amino acid sequence (SEQ ID NO: 116), Nivolumab is linked to linker peptide through Fc domain, and the linker peptide is covalently linked to IgD1/IgD2/IgD3 domain of LAG3 extracellular domain, which is linked to linker peptide linked in series to CD80 extracellular domain to form the multifunctional fusion polypeptide first polypeptide amino acid sequence (SEQ ID NO: 117), Nivolumab is linked to linker peptide through Fc domain, and the linker peptide is covalently linked to LAG3 extracellular domain, and the LAG3 extracellular domain is linked in series via linker peptide to CD80 extracellular domain to form multifunctional fusion polypeptide first polypeptide amino acid sequence (SEQ ID NO: 118), Nivolumab antigen binding domain heavy chain N-terminal is linked to linker peptide covalently linked to LAG3 extracellular domain, and Nivolumab Fc domain is linked to the linker peptide covalently linked to CD80 extracellular domain to form the multifunctional fusion polypeptide first polypeptide amino acid sequence (SEQ ID NO: 119), Nivolumab antigen binding domain heavy chain N-terminal is linked to linker peptide covalently linked to CD80 extracellular domain, and Nivolumab Fc domain is linked to linker peptide covalently linked to LAG3 extracellular domain to form the multifunctional fusion polypeptide first polypeptide amino acid sequence (SEQ ID NO: 120). The first polypeptide is assembled with the second polypeptide (SEQ ID NO: 167) composed of the Nivolumab light chain polypeptide to form the double/or multifunctional fusion protein; the second polypeptide is assembled with the first polypeptide (SEQ ID NO: 9) composed of the Nivolumab heavy chain polypeptide into the dual/or multifunctional fusion protein.

The term "amino acid identity" may refer to when amino acid sequences are compared and aligned (with the introduction of gaps, if necessary) for maximum correspondence. Values for percent identity can be determined using sequence comparison software, or algorithms, or by visual inspection, and for comparisons of amino acid identity of two or more or mixed tandem amino acid identities of the second binding domain, the percent identity of one subunit to any subunit of the second binding domain should be calculated by comparing it separately to any subunit of the second binding domain; there are different algorithms and software known in the art that can be used to obtain alignments of amino acid sequences such as, but not limited to, NCBI BLAST software.

For example, the bifunctional antibody AN_Bif_Abs_0016 can be composed of sugemalimab heavy chain antibody AN_Bif_Abs_0016 first polypeptide (SEQ ID NO: 121) and the sugemalimab light chain second polypeptide (SEQ ID NO: 122). **AN_Bif_Abs_0016** bifunctional antibody is assembled by introducing a vector comprising polynucleotide sequence (SEQ ID NO: 123) expressing the first polypeptide of AN_Bif_ Abs_0016 and a vector comprising polynucleotide sequence (SEQ ID NO: 124) expressing AN _Bif_ Abs_0016 second polypeptide into host cells for expression.

For example, the bifunctional antibody AN_Bif_Abs_0017 can be composed of first polypeptide of chimeric antibody AN_Bif_Abs_0017 (SEQ ID NO: 27) (sugemalimab heavy chain Fc domain is linked to the linker peptide (SEQ ID NO: 25) covalently linked to the extracellular region of CD80 to form the first polypeptide) and sugemalimab light chain second polypeptide (SEQ ID NO: 122) **AN_Bif_Abs_0017** bifunctional antibody is assmebled by introducing a vector comprising polynucleotide sequence (SEQ ID NO: 125) expressing AN_Bif_Abs_0017 first polypeptide and a vector comprising polynucleotide sequence (SEQ ID NO : 124) expressing AN_Bif_Abs_0017 second polypeptide into the host cell for expression.

For example, the bifunctional antibody AN_Bif_Abs_0025 can be composed of the first polypeptide of the chimeric antibody AN_Bif_Abs_0025 (SEQ ID NO: 126) (a SOJA control antibody heavy chain Fc domain is linked to the linker peptide (SEQ ID NO: 25) covalently linked to the extracellular region of CD80 to form the first polypeptide) and aSOJA light chain second polypeptide (SEQ ID NO: 127). **AN_Bif**_**Abs_0025** bifunctional antibody is assmebled by introducing a vector comprising polynucleotide sequence (SEQ ID NO: 128) expressing AN_Bif_Abs_0025 first polypeptide and a vector comprising polynucleotide sequence (SEQ ID NO: 129) expressing AN_Bif_Abs_0025 second polypeptide into host cells for expression.

For example, the bifunctional antibody AN_Bif_Abs_0042 can be composed of the first polypeptide of chimeric antibody AN_Bif_Abs_0042 (SEQ ID NO: 130) (sugemalimab via heavy chain Fc domain is linked to the linker peptide (SEQ ID NO: 24) covalently linked to the extracellular region of CD80 to form the first polypeptide) and sugemalimab light chain second polypeptide (SEQ ID NO: 122). **AN_Bif_Abs_0042** bifunctional antibody is assmebled by introducing a vector comprising polynucleotide sequence (SEQ ID NO: 131) expressing AN_Bif_Abs_0042 first polypeptide and a vector comprising polynucleotide sequence (SEQ ID NO : 124) expressing AN_Bif_Abs_0042 second polypeptide into host cell for expression.

For example, the bifunctional antibody AN_Bif_Abs_0043 can be composed of the first polypeptide of chimeric antibody AN_Bif_Abs_0043 (SEQ ID NO: 132) (sugemalimab via heavy chain Fc domain is linked to the linker peptide (SEQ ID NO: 23) covalently linked to the extracellular region of CD80 to form the first polypeptide) and sugemalimab The light chain second polypeptide (SEQ ID NO: 122). **AN_Bif_Abs_0043** bifunctional antibody is assmebled by introducing a vector comprising polynucleotide sequence (SEQ ID NO: 133) expressing AN_Bif_Abs_0043 first polypeptide and a vector comprising polynucleotide sequence (SEQ ID NO : 124) expressing AN_Bif_Abs_0043 second polypeptide into host cell for expression.

For example, the bifunctional antibody AN_Bif_Abs_0019 can be composed of the first polypeptide of chimeric antibody AN_Bif_Abs_0019 (SEQ ID NO: 28) (sugemalimab via heavy chain Fc domain is linked to the linker peptide (SEQ ID NO: 25) covalently linked to the extracellular region of LAG3 to form the first polypeptide) and sugemalimab light chain second polypeptide (SEQ ID NO: 122). **AN_Bif_Abs_0019** bifunctional antibody is assembled by introducing a vevtor expressing AN_Bif_Abs_0019 first polypeptide polynucleotide sequence (SEQ ID NO: 134) and a vector comprising expressing polynucleotide sequence (SEQ ID NO : 124) AN_Bif_ Abs_0019 second polypeptide into host cells for expression.

For example, the bifunctional antibody AN_Bif_Abs_0044 can be composed of the first polypeptide of chimeric antibody AN_Bif_Abs_0044 (SEQ ID NO: 135) (sugemalimab via heavy chain Fc domain is linked to the linker peptide (SEQ ID NO: 24) covalently linked to the extracellular region of LAG3 to form the first polypeptide) and sugemalimab light chain second polypeptide (SEQ ID NO: 122). **AN_Bif_Abs_0044** bifunctional antibody is assembled by introducing a vector comprising polynucleotide sequence (SEQ ID NO: 136) expressing AN_Bif_Abs_0044 first polypeptide and a vector comprising polynucleotide sequence (SEQ ID NO : 124) expressing AN_Bif_Abs_0044 second polypeptide into host cells for expression.

For example, bifunctional antibodyAN_Bif_Abs_0045 can be composed of chimeric antibody AN_Bif_Abs_0045 first polypeptide (SEQ ID NO: 137) (sugemalimab via heavy chain Fc domain is linked to the linker peptide (SEQ ID NO: 23) covalently linked to the extracellular region of LAG3 to form the first polypeptide) and sugemalimab light chain second polypeptide (SEQ ID NO: 122). **AN_Bif_Abs_0045** bifunctional antibody is assembled by introducing a vector comprising polynucleotide sequence (SEQ ID NO: 138) expressing AN_Bif_Abs_0045 first polypeptide and a vector comprising polynucleotide sequence (SEQ ID NO: 124) expressing AN_Bif_Abs_0045 second polypeptide into host cells for expression.

For example, the bifunctional antibody AN_Bif_Abs_0046 can be composed of the first polypeptide of chimeric antibody AN_Bif_Abs_0046 (SEQ ID NO: 139) (sugemalimab via heavy chain Fc domain is covalently linked to the extracellular region of LAG3 via linker peptide (SEQ ID NO: 22) to form the first polypeptide) and sugemalimab light chain second polypeptide (SEQ ID NO: 122). **AN_Bif_Abs_0046** bifunctional antibody is assembled by introducing a vevtor expressing AN_Bif_Abs_0046 first polypeptide polynucleotide sequence (SEQ ID NO: 140) and a vector comprising polynucleotide sequence (SEQ ID NO: 124) expressing AN_Bif_Abs_0046 second polypeptide into host cell for expression.

For example, the bifunctional antibody AN_Bif_Abs_0035 can be composed of the first polypeptide of the chimeric antibody AN_Bif_Abs_0035 (SEQ ID NO: 32) (sugemalimab via heavy chain antigen domain heavy chain N-terminal is linked to the linker peptide (SEQ ID NO: 25) covalently linked to the extracellular region of LAG3 to form the first polypeptide) and sugemalimab light chain second polypeptide (SEQ ID NO: 122). **AN_Bif_Abs_0035** bifunctional antibody is assembled by introducing a vector compring polynucleotide sequence (SEQ ID NO: 141) expressing AN_Bif_Abs_0035 first polypeptide and a vector comprising polynucleotide sequence (SEQ ID NO: 124) expressing AN_Bif_Abs_0035 second polypeptide into host cells for expression.

For example, the bifunctional antibody AN_Bif_Abs_0047 can be composed of the first polypeptide of the chimeric antibody AN_Bif_Abs_0047 (SEQ ID NO: 30) (sugemalimab via heavy chain Fc domain is linked to the linker peptide (SEQ ID NO: 25) covalently linked to the IgD1/IgD2/IgD3 domain of the extracellular region ofLAG3 to form the first polypeptide) and sugemalimab light chain second polypeptide (SEQ ID NO: 122). **AN_Bif_Abs_0047** bifunctional antibody is assembled by introducing a vector comprising polynucleotide sequence (SEQ ID NO: 142) expressing AN_Bif_Abs_0047 first polypeptide and a vector comprising polynucleotide sequence (SEQ ID NO: 124) expressing AN_Bif_ Abs_0047 second polypeptide into host cell for expression.

For example, the bifunctional antibody AN_Bif_Abs_0048 can be composed of the first polypeptide of chimeric antibody AN_Bif_Abs_0048 (SEQ ID NO: 29) (sugemalimab via heavy chain Fc domain is linked to the linker peptide (SEQ ID NO: 25) covalently linked to the IgD1/IgD2 domain of the extracellular region of LAG3 to form the first polypeptide) and sugemalimab light chain second polypeptide (SEQ ID NO: 122). **AN_Bif_Abs_0048** bifunctional antibody is assembled by introducing a vector comprising polynucleotide sequence (SEQ ID NO: 143) expressing AN_Bif_Abs_0048 first polypeptide and a vector comprising polynucleotide sequence (SEQ ID NO: 124) expressing AN_Bif_ Abs_0048 second polypeptide into host cell for expression.

For example, the multifunctional antibody AN_Bif_Abs_0036 can be composed of a chimeric antibody AN_Bif_Abs_0036 first polypeptide (SEQ ID NO: 41) (the heavy chain N-terminus of the sugemalimab heavy chain antigen domain is linked to the linker peptide (SEQ ID NO: 25) covalently linked to the LAG3 extracellular domain, and at the same time sugemalimab via the heavy chain C-terminus is linked to the linker peptide (SEQ. ID NO: 25) linked in series to the CD80 extracellular domain to form the first polypeptide) and sugemalimab light chain second polypeptide (SEQ ID NO: 122). **AN_Bif_Abs_0036** multifunctional antibody is assembled by introducing a vector comprising polynucleotide sequence (SEQ ID NO: 144) expressing AN_Bif_Abs_0036 first polypeptide and a vector comprising polynucleotide sequence (SEQ ID NO: 124) expressing AN_Bif_ Abs_0036 second polypeptide into host cells for expression.

For example, the multifunctional antibody AN_Bif_Abs_0037 can be composed of chimeric antibody AN_Bif_Abs_0037 first polypeptide (SEQ ID NO: 40) (sugemalimab via heavy chain Fc domain heavy chain C-terminal is linked to the linker peptide (SEQ ID NO: 25) covalently linked to the LAG3 extracellular domain, and the LAG3 extracellular domain via linker peptide (SEQ ID NO: 25) is linked in series to CD80 extracellular domain to form the first polypeptide) and sugemalimab light chain second polypeptide (SEQ ID NO: 122). **AN_Bif_Abs_0037** multifunctional antibody is assembled by introducing a vector comprising polynucleotide sequence (SEQ ID NO: 145) expressing AN_Bif_Abs_0037 first polypeptide and a vector comprising polynucleotide sequence (SEQ ID NO: 124) expressiing AN_Bif_Abs_0037 second polypeptide into host cells for expression.

For example, the multifunctional antibody AN_Bif_Abs_0038 can be composed of chimeric antibody AN_Bif_Abs_0038 first polypeptide (SEQ ID NO: 37) (sugemalimab via heavy chain Fc domain heavy chain C-terminal is linked to the linker peptide (SEQ ID NO: 25) covalently linked to the CD80 extracellular domain, which is linked to the linker peptide (SEQ ID NO: 25) linked in series to the LAG3 extracellular domain to form the first polypeptide) and sugemalimab light chain second polypeptide (SEQ ID NO: 122). **AN_Bif_Abs_0038** multifunctional antibody is assembled by introducing a vector comprising polynucleotide sequence (SEQ ID NO: 122). NO: 146) expressing the AN_Bif_Abs_0038 first polypeptide and a vector comprising polynucleotide sequence (SEQ ID NO: 124) expressing AN_Bif_Abs_0038 second polypeptide into host cells for expression.

For example, the multifunctional antibody AN_Bif_Abs_0049 can be composed of chimeric antibody AN_Bif_Abs_0049 first polypeptide (SEQ ID NO: 147) (sugemalimab via heavy chain Fc domain heavy chain C-terminal is linked to the linker peptide (SEQ ID NO: 23) covalently linked to CD80 extracellular domain, which is linked to the linker peptide (SEQ ID NO: 23) linked in series to LAG3 extracellular domain to form the first polypeptide) and sugemalimab light chain second polypeptide (SEQ ID NO: 122). **AN_Bif_Abs_0049** multifunctional antibody is assembled by introducing a vector comprising polynucleotide sequence (SEQ ID NO: 148) expressing AN_Bif_Abs_0049 first polypeptide and a vector comprising polynucleotide sequence (SEQ ID NO: 124) expressing AN_Bif_ Abs_0049 second polypeptide into host cells for expression.

For example, the multifunctional antibody AN_Bif_Abs_0050 can be composed of chimeric antibody AN_Bif_Abs_0050 first polypeptide (SEQ ID NO: 149) (sugemalimab via heavy chain Fc domain heavy chain C-terminal is linked to the linker peptide (SEQ ID NO: 23)covalently linked to CD80 extracellular domain, which is linked to the linker peptide (SEQ ID NO: 23) linked in series to IgD1/IgD2/IgD3/IgD4 domain of the extracellular region of LAG3 to form the first polypeptid) and sugemalimab light chain second polypeptide (SEQ ID NO: 122). **AN_Bif_Abs_0050** multifunctional antibody is assembled by introducing a vector comprising polynucleotide sequence (SEQ ID NO: 150) expressing AN_Bif_Abs_0050 first polypeptide and a vector comprising polynucleotide sequence (SEQ ID NO: 124) expressing AN_Bif_Abs_0050 second polypeptide into host cells for expression.

For example, the multifunctional antibody AN_Bif_Abs_0051 can be composed of chimeric antibody AN_Bif_Abs_0051 first polypeptide (SEQ ID NO: 36) (sugemalimab via heavy chain Fc domain heavy chain C-terminal is linked to the linker peptide (SEQ ID NO: 23) covalently linked to CD80 extracellular domain, which is linked to the linker peptide (SEQ ID NO: 23) linked in series to the IgD1/IgD2/IgD3 domain of extracellular region of LAG3 to form the first polypeptide) and sugemalimab light chain second polypeptide (SEQ ID NO: 122). **AN_Bif_Abs_0051** multifunctional antibody is assembled by introducing a vector comprising polynucleotide sequence (SEQ ID NO: 151) expressing AN_Bif_Abs_0051 first polypeptide and a vector comprising polynucleotide sequence (SEQ ID NO: 124) expressing AN_Bif_Abs_0051 second polypeptide into host cells for expression.

For example, the multifunctional antibody AN_Bif_Abs_0052 can be composed of chimeric antibody AN_Bif_Abs_0052 first polypeptide (SEQ ID NO: 35) (sugemalimab via heavy chain Fc domain heavy chain C-terminal is linked to the linker peptide (SEQ ID NO: 23) covalently linked to CD80 extracellular domain, which is linked to the linker peptide (SEQ ID NO: 23) linked in series to IgD1/IgD2 domain of the extracellular region ofLAG3 to form the first polypeptide) and sugemalimab light chain second polypeptide (SEQ ID NO: 122). **AN_Bif_Abs_0052** multifunctional antibody is assembled by introducing a vector comprising polynucleotide sequence (SEQ ID NO: 152) expressing AN_Bif_Abs_0052 first polypeptide and a vector comprising polynucleotide sequence (SEQ ID NO: 124) expressing AN_Bif_Abs_0052 second polypeptide into host cells for expression.

For example, the multifunctional antibody AN_Bif_Abs_0053 can be composed of chimeric antibody AN_Bif_Abs_0053 first polypeptide (SEQ ID NO: 153) (sugemalimab via heavy chain Fc domain heavy chain C-terminal is linked to the linker peptide (SEQ ID NO: 22) covalently linked to CD80 extracellular domain, which is linked to the linker peptide (SEQ ID NO: 22) linked in series to LAG3 extracellular domain to form the first polypeptide) and sugemalimab light chain second polypeptide (SEQ ID NO: 122). **AN_Bif_Abs_0053** multifunctional antibody is assembled by introducing a vector comprising polynucleotide sequence (SEQ ID NO: 154) expressing AN_Bif_Abs_0053 first polypeptide and a vector comprising polynucleotide sequence (SEQ ID NO: 124) expressing AN_Bif_ Abs_0053 second polypeptide into host cells for expression.

For example, the multifunctional antibody AN_Bif_Abs_0054 can be composed of chimeric antibody AN_Bif_Abs_0054 first polypeptide (SEQ ID NO: 155) (sugemalimab via heavy chain Fc domain heavy chain C-terminal is linked to the linker peptide (SEQ ID NO: 24) covalently linked to CD80 extracellular domain, which is linked to the linker peptide (SEQ ID NO: 24) linked in series to LAG3 extracellular domain to form the first polypeptide) and sugemalimab light chain second polypeptide (SEQ ID NO: 122). **AN_Bif_Abs_0054** multifunctional antibody is assembled by introducing a vector comprising polynucleotide sequence (SEQ ID NO: 124) expressing AN_Bif_Abs_0054 first polypeptide and a vector comprising polynucleotide sequence (SEQ ID NO: 156) expressing AN_Bif_ Abs_0054 second polypeptide into host cells for expression.

For example, bifunctional antibodyAN _Bif_ Abs_0030 can be composed of the chimeric antibody AN_Bif_Abs_0030 first polypeptide (SEQ ID NO: 157) (aSOJA control antibody heavy chain Fc domain is linked to the linker peptide (SEQ ID NO: 25) covalently linked to the extracellular region of LAG3 to form the first polypeptide) and aSOJA light chain second polypeptide (SEQ ID NO: 127). AN_Bif_Abs_0030 bifunctional antibody is assembled by introducing a vector comprising polynucleotide sequence (SEQ ID NO: 158) expressing AN_Bif_Abs_0030 first polypeptide and a vector comprising polynucleotide sequence (SEQ ID NO: 129) expressing AN_Bif_Abs_0030 second polypeptide into host cells for expression.

For example, the multifunctional antibody AN_Bif_Abs_0055 can be composed of chimeric antibody AN_Bif_Abs_0055 first polypeptide (SEQ ID NO: 168) (sugemalimab via heavy chain antigen domain heavy chain N-terminal is linked to the linker peptide (SEQ ID NO: 25)covalently linked to the IgD1/D2 domain of LAG3 extracellular domain, meanwhile sugemalimab via heavy chain C-terminal is linked to the linker peptide (SEQ ID NO: 25) linked in series to the CD80 extracellular domain to form the first polypeptide) and sugemalimab light chain second polypeptide (SEQ ID NO: 122). **AN_Bif_Abs_0055** multifunctional antibody is assembled by introducing a vector comprising polynucleotide sequence (SEQ ID NO: 169) expressing AN_Bif_Abs_0055 first polypeptide and a vector comprising polynucleotide sequence (SEQ ID NO: 124) expressing AN _Bif_ Abs_0055 second polypeptide into host cells for expression.

For example, the multifunctional antibody AN_Bif_Abs_0056 can be composed of chimeric antibody AN_Bif_Abs_0056 first polypeptide (SEQ ID NO: 170) (sugemalimab via heavy chain antigen domain heavy chain N-terminal is linked to the linker peptide (SEQ ID NO: 25) covalently linked to IgD1/D2/D3 domain of LAG3 extracellular domain, meanwhile sugemalimab via heavy chain C-terminal is linked to the linker peptide (SEQ ID NO: 25) linked in series to the CD80 extracellular domain to form the first polypeptide) and sugemalimab light chain second polypeptide (SEQ ID NO: 122). **AN_Bif_Abs_0056** multifunctional antibody is assembled by introducing a vector comprising polynucleotide sequence (SEQ ID NO: 171) expressing AN_Bif_Abs_0056 first polypeptide and a vector comprising polynucleotide sequence (SEQ ID NO: 124) expressing AN _Bif_ Abs_0056 second polypeptide into host cells for expression.

For example, the multifunctional antibody AN_Bif_Abs_0060 can be composed of a chimeric antibody AN_Bif_Abs_0060 first polypeptide (SEQ ID NO: 172) (the heavy chain N-terminus of the sugemalimab heavy chain antigen domain is linked to the linker peptide (SEQ ID NO: 24) covalently linked to the LAG3 extracellular domain, and at the same time sugemalimab via heavy chain N-terminus is linked to the linker peptide (SEQ. ID NO: 25) linked in series to the CD80 extracellular domain to form the first polypeptide) and sugemalimab light chain second polypeptide (SEQ ID NO: 122). AN_Bif_Abs_0060 multifunctional antibody is assembled by introducing a vevtor comprsing polynucleotide sequence (SEQ ID NO: 173) expressing AN_Bif_Abs_0060 first polypeptide and a vector comprsing polynucleotide sequence (SEQ ID NO: 124) expressing AN_Bif_ Abs_0060 second polypeptide into host cells for expression.

For example, the multifunctional antibody AN_Bif_Abs_0061 can be composed of a chimeric antibody AN_Bif_Abs_0061 first polypeptide (SEQ ID NO: 174) (sugemalimab via heavy chain antigen domain heavy chain N-terminal is linked to the linker peptide (SEQ ID NO: 23) covalently linked to the LAG3 extracellular domain, and at the same time sugemalimab via the heavy chain C-terminal is linked to the linker peptide (SEQ ID NO: 25) linked in series to the CD80 extracellular domain to form the first polypeptide) and sugemalimab light chain second polypeptide (SEQ ID NO: 122). **AN_Bif_Abs_0061** multifunctional antibody is assembled by introducing a vector comprising polynucleotide sequence (SEQ ID NO: 175) expressing the AN_Bif_Abs_0061 first polypeptide and a vector comprising polynucleotide sequence (SEQ ID NO: 124) expressiing AN_Bif_Abs_0061 second polypeptide into host cells for expression.

For example, the multifunctional antibody AN_Bif_Abs_0062 can be composed of chimeric antibody AN_Bif_Abs_0062 first polypeptide (SEQ ID NO: 176) (sugemalimab via heavy chain antigen domain heavy chain N-terminal is linked to the linker peptide (SEQ ID NO: 22)covalently linked to the LAG3 extracellular domain, meanwhile sugemalimab via heavy chain C-terminal is linked to the linker peptide (SEQ ID NO: 25) linked in series to the CD80 extracellular domain to form the first polypeptide) and the second polypeptide (SEQ ID NO: 122) (sugemalimab light chain). **AN_Bif_Abs_0062** multifunctional antibody is assembled by introducing a vector comprising polynucleotide sequence (SEQ ID NO: 177) expressing AN_Bif_Abs_0062 first polypeptide and a vector comprising polynucleotide sequence (SEQ ID NO: 124) expressing AN _Bif_ Abs_0062 second polypeptide into host cells for expression.

For example, the multifunctional antibody AN_Bif_Abs_0014 can be composed of the first polypeptide of the chimeric antibody AN_Bif_Abs_0014 (SEQ ID NO: 178) (the C-terminal of the heavy chain of sugemalimab is linked to the linker peptide (SEQ ID NO: 25) covalently linked to the ICOSLG extracellular domain by to form the first polypeptide) and the second polypeptide (SEQ ID NO: 122) (the light chain of sugemalimab). **AN_Bif_Abs_0014** multifunctional antibody is assembled by introducing a vector comprsing the polynucleotide sequence of (SEQ ID NO: 179) expressing the first polypeptide of AN_Bif_ Abs_0014 and a vector comprsing the polynucleotide sequence of (SEQ ID NO: 124) expressing the second polypeptide of AN_Bif_Abs_0014 into the host cell for expression.

For example, the multifunctional antibody AN_Bif_Abs_0006 can be composed of the first polypeptide of the chimeric antibody AN_Bif_Abs_0006 (SEQ ID NO: 75) (the C-terminal of the heavy chain of Avelumab is linked to the linker peptide (SEQ ID NO: 25) covalently linked to the CD80 extracellular domain to form the first polypeptide) and the light chain of Avelumab second polypeptide (SEQ ID NO: 165). **AN_Bif_Abs_0006** multifunctional antibody is assembled by introducing a vector comprising polynucleotide sequence (SEQ ID NO: 180) expressing AN_Bif_Abs_0006 first polypeptide and a vector comprising polynucleotide sequencee (SEQ ID NO: 188) expressing AN_Bif_Abs_0006 second polypeptide into the host cell for expression.

For example, the multifunctional antibody AN_Bif_Abs_0024 can be composed of the first polypeptide of the chimeric antibody AN_Bif_Abs_0024 (SEQ ID NO: 59) (the C-terminal of the heavy chain of Atezolizumab is linked to the linker peptide (SEQ ID NO: 25) covalently linked to the CD80 extracellular domain to form the first polypeptide) and the light chain of Atezolizumab second polypeptide (SEQ ID NO: 164). By incorporating the vector comprising the polynucleotide sequence of (SEQ ID NO: 181) expressing the first polypeptide of AN_Bif_Abs_0024 and the vector comprising the polynucleotide sequence of (SEQ ID NO: 189) expressing the second polypeptide of AN_Bif_ Abs_0024 into the host cell for expression to achieve assembly into **AN_Bif_Abs_0024** multifunctional antibody.

For example, the multifunctional antibody AN_Bif_Abs_0013 can be composed of the first polypeptide of the chimeric antibody AN_Bif_Abs_0013 (SEQ ID NO: 44) (the C-terminal of the heavy chain of Durvalumab is linked to the linker peptide (SEQ ID NO: 25) covalently linked to the extracellular domain of LAG3y to form the first polypeptide) and the Durvalumab light chain second polypeptide (SEQ ID NO: 163). **AN_Bif_Abs_0013** multifunctional antibody is assembled by introducing the vector comprising the polynucleotide sequence of (SEQ ID NO: 182) expressing the first polypeptide of AN_Bif_Abs_0013 and the vector comprising the polynucleotide sequence of (SEQ ID NO: 190) expressing the second polypeptide of AN_Bif_Abs_0013 into the host cell for expression.

For example, the multifunctional antibody AN_Bif_Abs_0079 can be composed of the first polypeptide of the chimeric antibody AN_Bif_Abs_0079 (SEQ ID NO: 91) (the C-terminal of the heavy chain of Envafolimab is linked to the linker peptide (SEQ ID NO: 25) covalently linked to the extracellular domain of LAG3 to form the first polypeptide); a vector comprising polynucleotide sequence (SEQ ID NO: 183) expressing AN_Bif_Abs_0079 first polypeptide is introduced into the host cell for expression to achieve assembly into **AN_Bif_Abs_0079** multifunctional antibody.

For example, the multifunctional antibody AN_Bif_Abs_0078 can be composed of the first polypeptide of the chimeric antibody AN_Bif_Abs_0078 (SEQ ID NO: 103) (the N-terminal of the heavy chain antigen domain of the Envafolimab is linked to the linker peptide (SEQ ID NO: 25) covalently linked to the extracellular domain of LAG3, and Envafolimab via the C-terminal of the heavy chain can be linked to the linker peptide (SEQ ID NO: 25) at the same time linked in series to the CD80 extracellular domain to form the first polypeptide); by introducing the vector comprising the polynucleotide sequence of (SEQ ID NO: 184) expressing the first polypeptide of AN_Bif_Abs_0078 into the host cells for expression to achieve assembly into **AN_Bif_Abs_0078** multifunctional antibody.

For example, the multifunctional antibody AN_Bif_Abs_0106 can be composed of the chimeric antibody AN_Bif_Abs_0106 first polypeptide (SEQ ID NO: 42) (sugemalimab via N-terminal of the heavy chain antigen domain is linked to the linker peptide (SEQ ID NO: 25) covalently linked to the CD80 extracellular domain, and sugemalimab via the C-terminal of the heavy chain at the same time linked to the linker peptide (SEQ ID NO: 25) linked in series to the LAG3 extracellular domain to form the first polypeptide) and sugemalimab light chain second polypeptide (SEQ ID NO: 122). **AN_Bif_Abs_0106** multifunctional antibody is assembled by introducing a vector comprising polynucleotide sequence (SEQ ID NO: 185) expressing AN_Bif_Abs_0106 first polypeptide and a vector comprising polynucleotide sequence (SEQ ID NO: 124) expressing AN_Bif_Abs_0106 second polypeptide into host cells for expression.

For example, the multifunctional antibody AN_Bif_Abs_0093 can be composed of the first polypeptide of the chimeric antibody AN_Bif_Abs_0093 (SEQ ID NO: 93) (the C-terminal of the heavy chain of Envafolimab is linked to the linker peptide (SEQ ID NO: 25) covalently linked to the LAG3-D1/D2 domain to form the first polypeptide); AN_Bif_Abs_0093 multifunctional antibody is assembled by introducing the vector comprsing polynucleotide sequence (SEQ ID NO: 186) expressing the first polypeptide of AN_Bif_Abs_0093 into the host cell for expression.

For example, the multifunctional antibody AN_Bif_Abs_0094 can be composed of the first polypeptide of the chimeric antibody AN_Bif_Abs_0094 (SEQ ID NO: 94) (the C-terminal of the heavy chain of Envafolimab is linked to the linker peptide (SEQ ID NO: 25) covalently linked to the LAG3-D1/D2/D3 domain to form the first polypeptide); The **AN_Bif_Abs_0094** multifunctional antibody is assembed by introducing the vector comprising polynucleotide sequence (SEQ ID NO: 187) expressing the first polypeptide of AN_Bif_Abs_0094 into the host cell for expression.

In some embodiments, the present application also provides a method for isolating a polypeptide complex to isolate the polypeptide complex.

In other aspects, the content disclosed in this application provides a method for releasing the inhibitory effect of PD-L1/PD-1 on T cells and at the same time stimulating the activation of antigen-presenting cells or/and T cells. The disclosed dual and/or multifunctional fusion polypeptides are used to stimulate the activation of T cells, but are not limited to the schemes used in the examples, and can be extended to those that have been published or developed in the future and can be used to evaluate antigen-presenting cells or / and T cell activation schemes.

On the other hand, the dual and/or multifunctional fusion polypeptide disclosed in this application has the effect of blocking the growth of tumors, including but not limited to colon tumors, breast tumors, lung tumors, gastric tumors, melanoma, head and neck tumors, lymphoma, nasopharyngeal tumors, cervical tumors, esophageal tumors, renal tumors, skin squamous cell carcinomas, endometrial tumors, liver tumors, bladder tumors, urothelial tumors, and/or skin tumors.

In another aspect, the present application provides an immunoconjugate, which may comprise the fusion polypeptide described in the present application.

In another aspect, the present application provides a nucleic acid molecule, which can encode the fusion polypeptide described in the present application.

In another aspect, the present application provides a vector, which may comprise the nucleic acid molecule described in the present application.

In another aspect, the present application provides a cell that can contain and/or express the fusion polypeptide described in the present application, the immunoconjugate described in the present application, the nucleic acid molecule described in the present application, and/or the the carrier.

In another aspect, the present application provides a composition, which may comprise the fusion polypeptide described in the present application, the immunoconjugate described in the present application, the nucleic acid molecule described in the present application, the carrier described in the present application, and /or the cells described in the present application, and optionally a pharmaceutically acceptable carrier.

On the other hand, the present application provides a method for preparing the fusion polypeptide described in the present application, which includes culturing the cells described in the present application under the conditions enabling the expression of the fusion polypeptide.

In another aspect, the present application provides the present application provides a method for blocking the interaction between PD-L1 protein and PD-1, which includes administering an effective amount of the fusion polypeptide described in the present application, the immunoconjugate described in the present application, the nucleic acid molecule described herein, the vector described herein, the cell described herein, and/or the composition described herein.

In another aspect, the present application provides a method for stimulating antigen-presenting cells and/or activating the action of T cells, which may include administering an effective amount of the fusion polypeptide described in the present application, the immunoconjugate described in the present application, he nucleic acid molecule described in the present application, the vector described in this application, the cell described in this application, and/or the composition described in this application.

According to the method described in this application, the stimulating antigen-presenting cells comprises being selected from the following group: increasing the expression of costimulatory molecules in the antigen-presenting cells, causing the morphological changes and maturation of the antigen-presenting cells, and causing the chemokines of the antigen-presenting cells. Increase secretion and enhance phagocytosis of antigen-presenting cells.

In another aspect, the present application provides a method for inhibiting the growth and/or proliferation of tumors or tumor cells, which may comprise administering an effective amount of the fusion polypeptide described in this application, the immunoconjugate described in this application, the nucleic acid molecule described in this application, the vector described in this application, the cells described in this application, and/or the composition described in this application.

In another aspect, the present application provides the use of the fusion polypeptide described in the present application, the immunoconjugate described in the present application, the nucleic acid molecule described in the present application, the carrier described in the present application, the cell described in the present application, and/or the composition described in this application in the preparation of medicaments, wherein the medicaments can be used to prevent, improve and/or treat tumors.

According to the use described in the present application, the tumor may include solid tumors and hematological tumors.

According to the purposes described in the present application, wherein the tumor can be selected from the following group: colon tumor, breast tumor, lung tumor, stomach tumor, melanoma, head and neck tumor, lymphoma, nasopharyngeal tumor, cervical tumor, esophagus tumor, kidney tumor tumors, endometrial tumors, liver tumors, bladder tumors, urothelial tumors and skin tumors. For example, skin tumors can include squamous cell carcinoma of the skin.

In another aspect, the present application provides the fusion polypeptide described in the present application, the immunoconjugate described in the present application, the nucleic acid molecule described in the present application, the carrier described in the present application, the cell described in the present application, and/or the composition described in this application, which can be used to prevent, improve and/or treat tumors.

In another aspect, the present application provides the fusion polypeptide described in the present application, the immunoconjugate described in the present application, the nucleic acid molecule described in the present application, the carrier described in the present application, the cell described in the present application, and/or the composition described in the application, which can be used to prevent, improve and/or treat tumors, wherein the tumors include solid tumors and hematological tumors.

In another aspect, the present application provides the fusion polypeptide described in the present application, the immunoconjugate described in the present application, the nucleic acid molecule described in the present application, the carrier described in the present application, the cell described in the present application, and/or the composition described in the application, it can be used for preventing, improving and/or treating tumor, wherein said tumor can be selected from following group: colon tumor, breast tumor, lung tumor, stomach tumor, melanoma, head and neck tumor, Lymphoma, nasopharyngeal tumor, cervical tumor, esophageal tumor, renal tumor, endometrial tumor, liver tumor, bladder tumor, urothelial tumor and skin tumor. For example, skin tumors can include squamous cell carcinoma of the skin.

In another aspect, the present application provides a method for preventing, improving and/or treating tumors, which may comprise administering the fusion polypeptide described in the present application, the immunoconjugate described in the present application to a subject in need, The nucleic acid molecule described herein, the vector described herein, the cell described herein, and/or the composition described herein.

According to the method described in the present application, the tumors may include solid tumors and hematological tumors.

According to the method described in the present application, wherein the tumor can be selected from the following group: colon tumor, breast tumor, lung tumor, stomach tumor, melanoma, head and neck tumor, lymphoma, nasopharyngeal tumor, cervical tumor, esophagus tumor, kidney tumor tumors, endometrial tumors, liver tumors, bladder tumors, urothelial tumors and skin tumors. For example, skin tumors can include squamous cell carcinoma of the skin.

Not intending to be limited by any theory, the following examples are only for explaining the products, preparation methods and uses of the present application, and are not intended to limit the scope of the present invention.

### Example

### Example 1

### Fusion Polypeptide Complex Expression and Purification

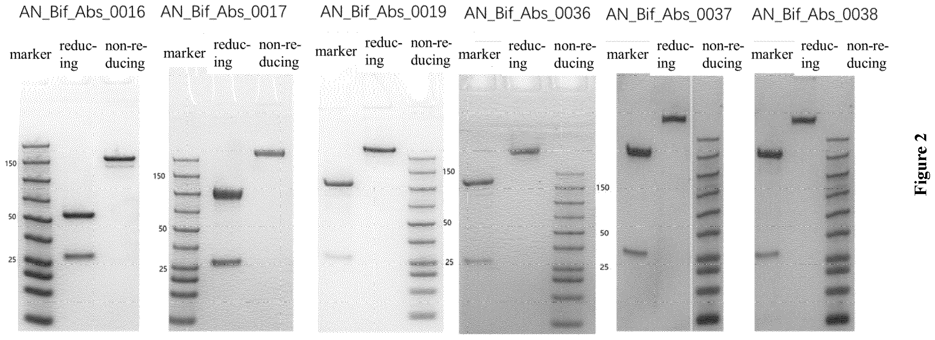

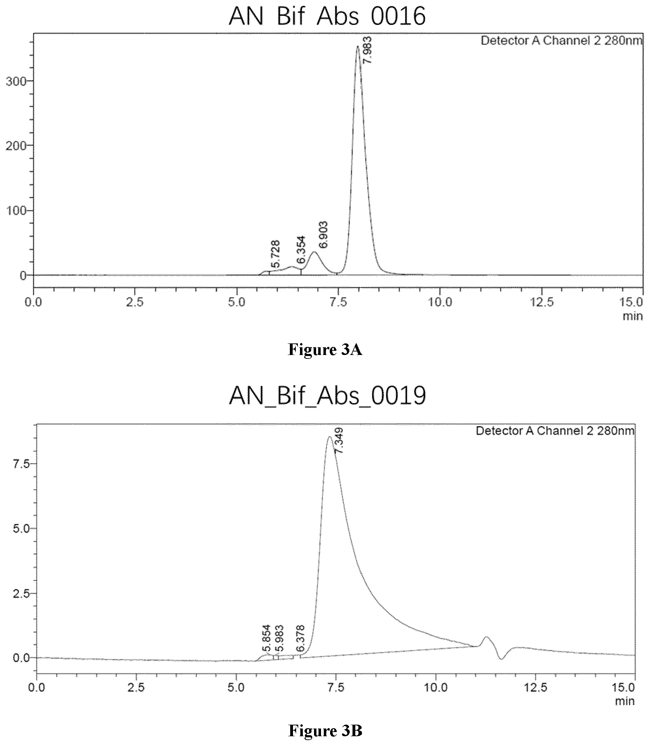

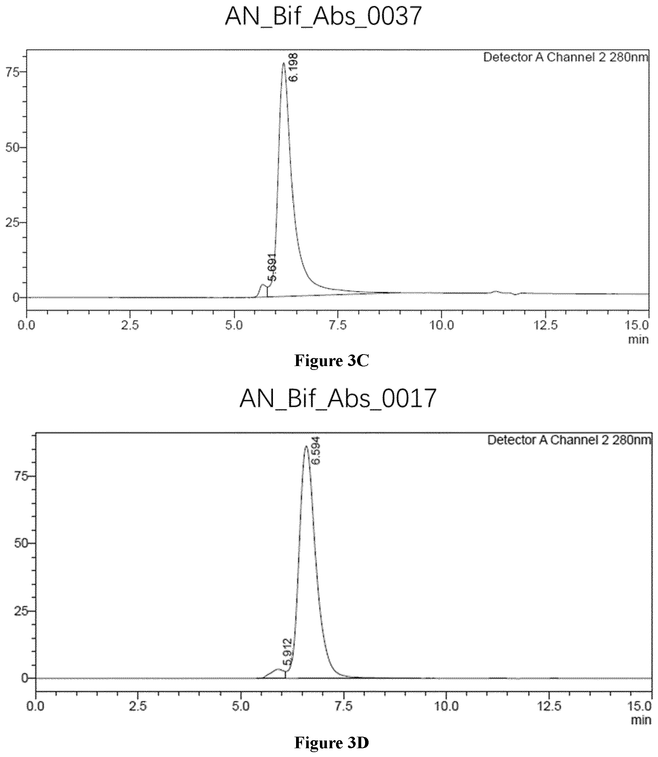

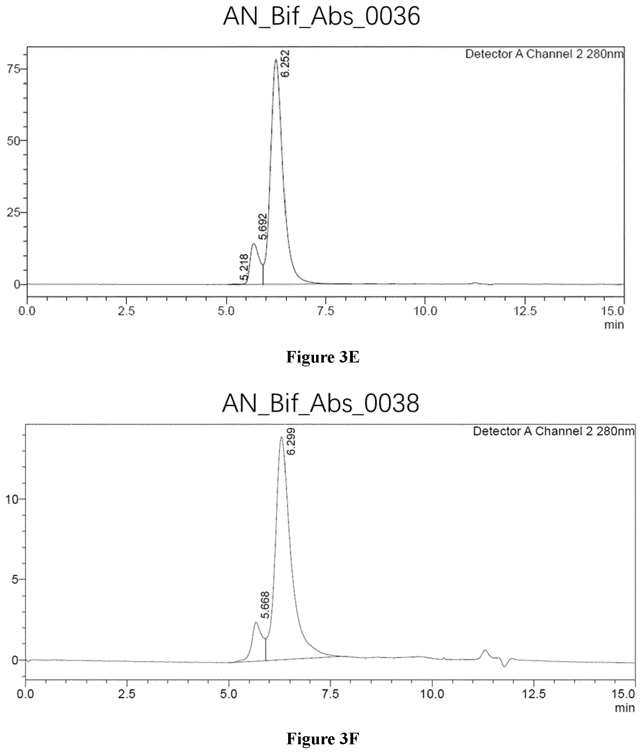

In order to further identify the antibodies obtained through screening, it is necessary to express the fusion polypeptide complex of the present application in mammalian cells. Therefore, an expression plasmid vector expressing the heavy chain of the multi/dual functional antibodies and an expression plasmid vector comprising the light chain were first constructed. About 24 hours before plasmid transfection, subculture Expi293 cells to make the cell density about 2.4×10⁶ cells/ml. When the cell density is 6×10⁶ cells/ml and the viability is >95%, take 25ug of the plasmid vector expressing the mixed heavy chain and light chain and transfect 25ml of Expi293 cells with Expifectamine 293, shake at 37°C, 130rpm, 8% CO₂ and culture for 7 days, centrifuge the cell culture product, take the supernatant, filter with 0.45 MCE filter, then use Capturem protein A Maxiprep column to purify and collect the target antibody, then use Vivaspin 20 Centrifugal Concentrator 50Kto carriy out centrifugal concentration, and measure A280 by NanoDrop 2000 Quantification, SDS-PAGE, SEC-HPLC to determine the purity of the antibody. Figure 2 shows the results of SDS-PAGE detection of the expression of the fusion polypeptide complex of the present application. Figures 3A-3F show the results of SEC-HPLC detection of the expression of the fusion polypeptide complex of the present application, and the results show that the fusion polypeptide complex of the present application can be expressed and purified.

### Example 2

### Binding Affinity Assay

In a specific embodiment, the present application provides that AN_Bif_Abs_0016, and/or AN_Bif_Abs_0017 and/or AN_Bif_Abs_0019, and/or LAG3-Ig, and/or AN_Bif_Abs_0036, and/or AN_Bif_Abs_0037, and/or AN_Bif_Abs_0038 bind to human PD-L1 protein and human CTLA4 protein; AN_Bif_Abs_0016, and/or AN_Bif_Abs_0019, and/or LAG3-Ig, and/or AN_Bif_Abs_0036, and/or AN_Bif_Abs_0037, and/or AN_Bif_Abs_0038 bind to human PD-L1 protein, human FGL1 protein and human MHCII.

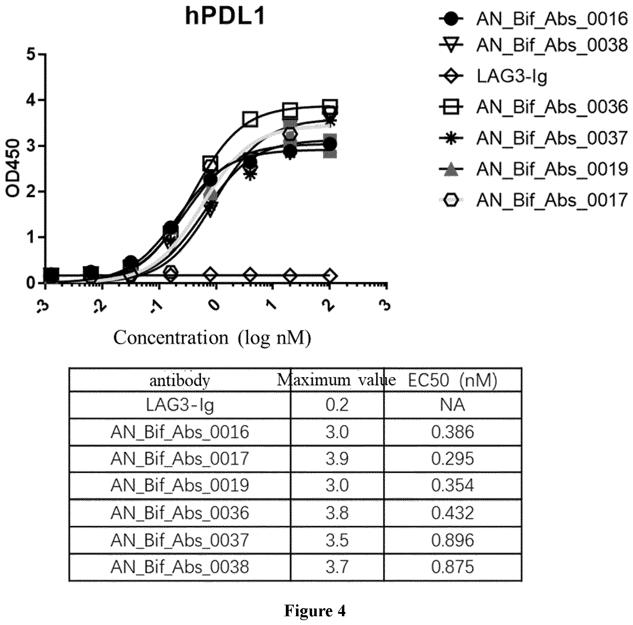

Specifically, human PD-L1 protein (5 µ g/mL in PBS; Aero Corporation, PD1-H5258) in a flat-bottom 96-well Immuno transparent microplate (MaxiSorp) (Thermo Scientific, 446469) was incubated at 4°C overnight. Then, after washing add 2% BSA(in PBS; VWR Life Science Corporation, 0332-1KG) to each well and culture at room temperature for 1 hour; after washing add serial dilution of AN_Bif_Abs_0016, AN_Bif_Abs_0017, and/or LAG3-Ig, and/or AN_Bif_Abs_0019, and/or AN_Bif_Abs_0036, and/or AN_Bif_Abs_0037, and/or AN_Bif_Abs_0038 to each well to the final test concentration, and incubate at room temperature for 2 hours; after washing add Goat Anti-Human Lambda-HRP(SouthernBiotech, 2060-05) to each well and incubate at room temperature for 30 minutes; after washing use Soluble TMB Kit(CoWin Biosciences,Inc., CW0050S) to detec tand show the results. The specific operation is carried out according to the manufacturer's instructions. Detect the binding affinity of candidate multi/ bifunctional fusion polypeptide complex to human PD-L1 protein. Figure 4 shows the results of detecting the binding of the fusion polypeptide complex of the present application to human PDL1 protein by ELISA.

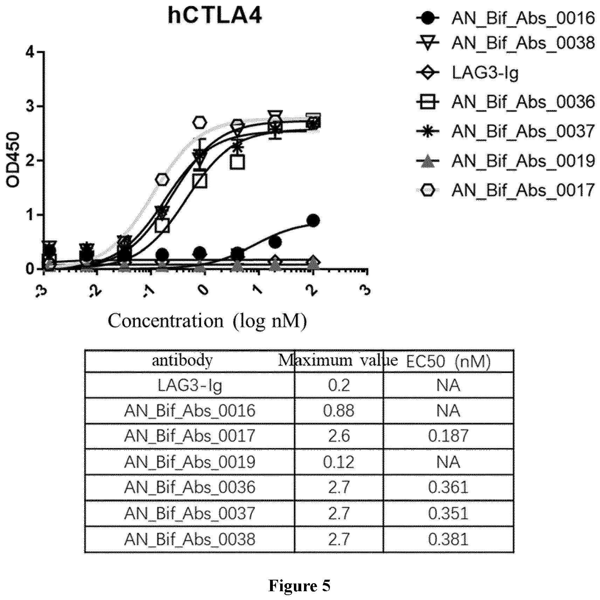

Similarily, human CTLA4 protein (5 µg/mL in PBS; Aero Corporation) in a flat-bottom 96-well Immuno transparent microplate(MaxiSorp)(Thermo Scientific, 446469) was incubated at 4°C overnight; the similar method was used to detect the binding affinity of AN_Bif_Abs_0016, and/or AN_Bif_Abs_0017 and/or AN_Bif_Abs_0019, and/or LAG3-Ig and/or AN_Bif_Abs_0036, and/or AN_Bif_Abs_0037, and/or AN_Bif_Abs_0038 to human CTLA4 protein. Figure 5 shows the results of detecting the binding of the fusion polypeptide complex of the present application to human CTLA4 protein by ELISA.

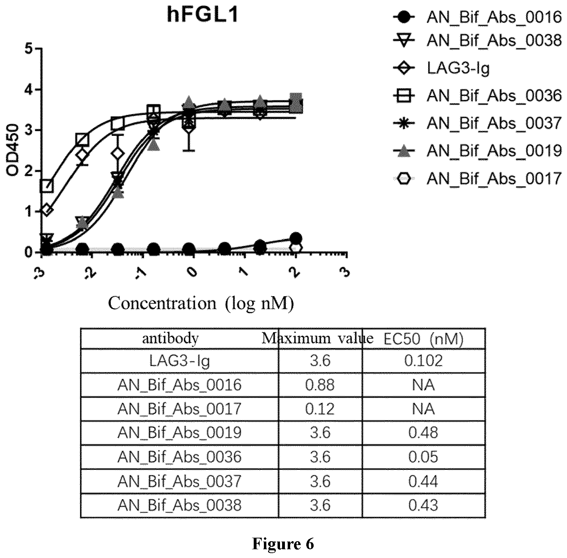

Similarily, human FGL1 protein (5 µg/mL in PBS; Aero Corporation) in a flat-bottom 96-well Immuno transparent microplate(MaxiSorp)(Thermo Scientific, 446469) was incubated at 4°C overnight; the similar method was used to detect the binding affinity of AN_Bif_Abs_0016, and/or AN_Bif_Abs_0017, and/or AN_Bif_Abs_0019, and/or LAG3-Ig, and/or AN_Bif_Abs_0036, and/or AN_Bif_Abs_0037, and/or AN_Bif_Abs_0038 to human FGL1 protein. Figure 6 shows the results of detecting the binding of the fusion polypeptide complex of the present application to human FGL1 protein by ELISA.

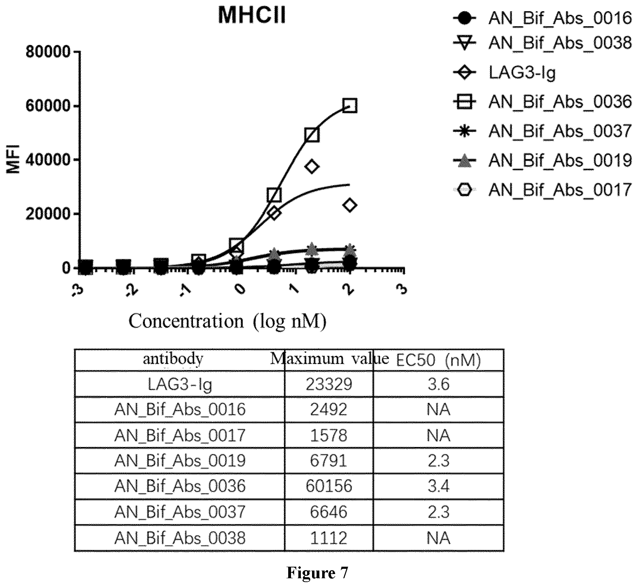

AN_Bif_Abs_0016, and/or AN_Bif_Abs_0017, and/or AN_Bif_Abs_0019, and/or LAG3-Ig, and/or AN_Bif_Abs_0036, and/or AN_Bif_Abs_0037, and/or AN_Bif_Abs_0038 serial dilutions were co-incubated with Raji cells expressing MHCII at room temperature 1h, after washing, Goat Anti-Human IgG-AF647 (SB Cat. No. 2040-31) was added to each well and incubated at 4°C for 30 min. After washing, the affinity of sugemalimab or AN_Bif_Abs_0019 to human MHCII protein was analyzed by flow cytometry. Figure 7 shows the results of detecting the binding of the fusion polypeptide complex of the present application with Raji cells expressing MHCII protein by flow cytometry. The results show that the fusion polypeptide complex of the present application can have the binding affinity of human PD-L1 protein, human CTLA4 protein, human FGL1 protein and/or human MHCII.

### Example 3

### T Cell Activation Immune Response Assay

In a specific embodiment, the present invention discloses that AN_Bif_ Abs_0016, and/or AN_Bif_Abs_0017, and/or AN_Bif_Abs_0025, and/or LAG3-Ig, and/or AN_Bif_Abs_0036, and/or AN_Bif_Abs_0037, and/or AN_Bif_Abs_0038, and/or AN_Bif_Abs_0014, and/or AN_Bif_Abs_0023, and/or AN_Bif_Abs_0024, and/or AN_Bif_Abs_0106, and/or AN_Bif_Abs_0005, and/or AN_Bif_Abs_0006, and/or AN_Bif_Abs_0031, and/or AN_Bif_Abs_0078, and/or AN_Bif_Ab s_0060, and/or AN_Bif_Abs_0061, and/or AN_Bif_Abs_0062 regulate the T cell immune response.

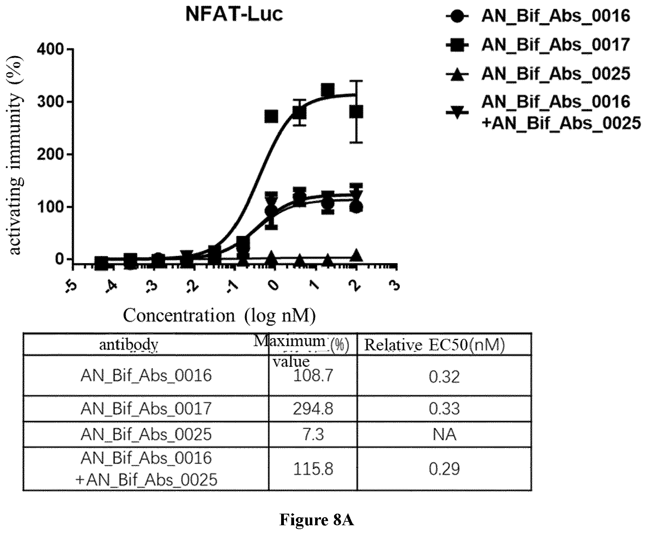

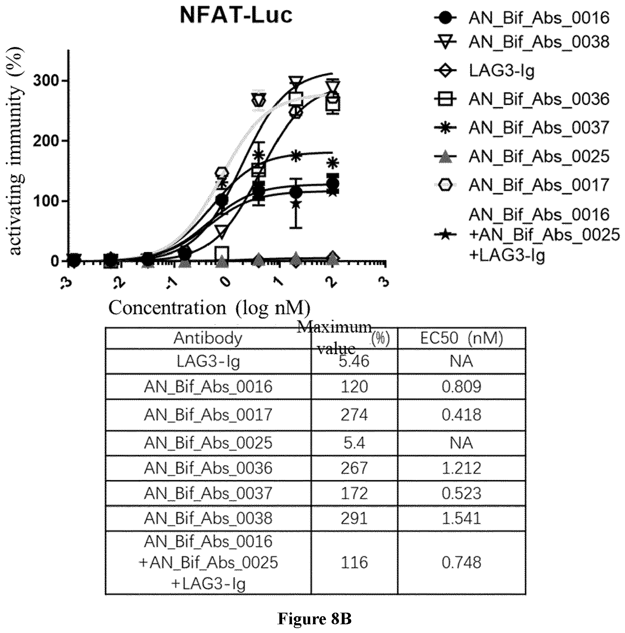

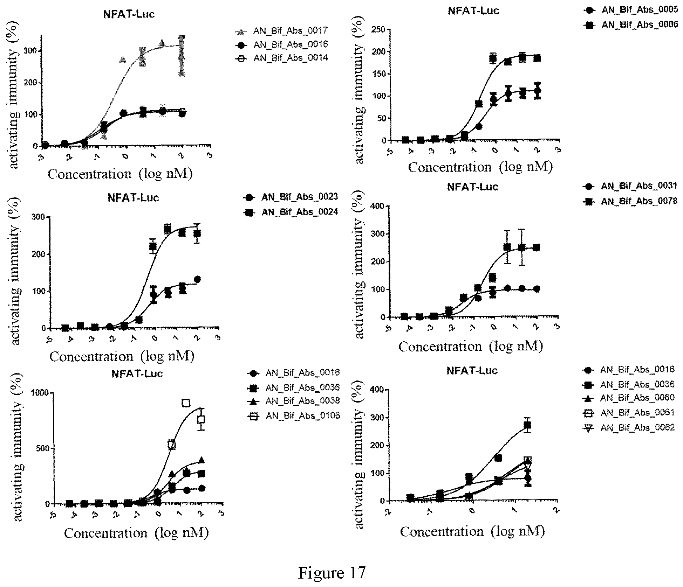

Specifically, on the first day, use 0.05% trypsin to digest, collect TCR activator PD-L1-CHO cells (BPS bioscience), count and plate; add fresh medium to mix the cells and adjust the cell density to 3.5*10⁵cells/ml. Add cells to a sterile 96-well flat-bottom plate (CORNING, 3599) at 100ul/well, a total of 35,000 cells/well; grow overnight in a 37°C incubator to 80%; remove the culture medium of TCR activator PD-L1- CHO cells from the 96-well flat-bottom plate the next day. 50ul fresh medium containing the corresponding concentration test substance (serially diluted) AN_Bif_Abs_0016, and/or AN_Bif_Abs_0017, and/or AN_Bif_Abs_0025, and/or LAG3-Ig, and/or AN_Bif_Abs_0036, and/or AN_Bif_Abs_0037, and/or AN_Bif_Abs_0038, and/or AN_Bif_Abs_0014, and/or AN_Bif_Abs_0023, and/or AN_Bif_Abs_0024, and/or AN_Bif_Abs_0106, and/or AN_Bif_Abs_0005, and/or AN_Bif_Abs_0006, and/or AN_Bif_Abs_0031, and/or AN_Bif_Abs_0078, and/or AN_Bif_Abs_0060, and/or AN_Bif_Abs_0061, and/or AN_Bif_Abs_0062 was added to each well. After 30 minutes of treatment, Jurkat-Lucia^{™} TCR-hPD-1 cells(Invivogen) were collected and counted; fresh medium was added to mix the cells evenly and adjust the cell density to 4*10⁵cells/ml; add 50ul Jurkat-Lucia^{™} TCR-hPD-1 cells to a final cell density of 2*10⁴ /well, and incubate in a 96-well flat-bottom plate in a 37°C incubator for 5-6 hours. After incubation, observe the cell morphology under a microscope and detect the chemiluminescence value with QUANTI-Luc^{™} (Invivogen; rep-qlc1), according to the manufacturer's instructions. Figures 8A-8B and Figure 17 show the results of the regulation of Jurkat T cell activation by the fusion polypeptide complex of the present application. The fusion polypeptide complex of the present application may have the ability to regulate the immune response of T cells.

### Example 4

### Determination of Relief of T Cell Immunosuppression

In a specific embodiment, the present invention discloses that AN_Bif_ Abs_0016, and/or AN_Bif_Abs_0017, AN_Bif_Abs_0025, and/or LAG3-Ig, and/or AN_Bif_Abs_0036, AN_Bif_Abs_0037, and/or AN_Bif_Abs_0038 relieve CTLA4 and PDL1-mediated T cell immunosuppression.

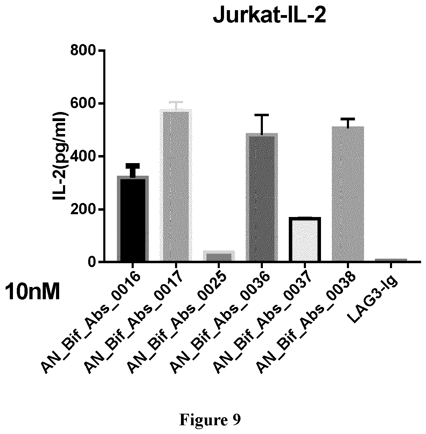

Specifically, collect Raji-APC-hPD-L1 cells (Invivogen), count and plate: add fresh medium to mix the cells and adjust the cell density to 0.5*10⁵ cells/ml. 50ul fresh medium containing the corresponding concentration test substance (serially diluted) AN _Bif_ Abs_0016, and/or AN_Bif_Abs_0017 and control AN_Bif_Abs_0025, and/or LAG3-Ig, and/or AN_Bif_Abs_0036, and/or AN_Bif_Abs_0037, and/or AN_Bif_Abs_0038 was added to each well. After treatment for 30min, PD-1 / NFAT Reporter cells (BPS bioscience) were collected and counted, and 1*10⁵ was added to each well; 1ug/ml CTLA4 protein (Acro company) and 1ug/ml anti-Human CD3 antibody were added (Invitrogen) After co-incubation for 24 h, the culture supernatant was collected, and the secretion of IL-2 in the supernatant was detected using an IL-2 ELISA detection kit (R&D, DY202), and the specific operation was performed according to the manufacturer's instructions. Fig. 9 shows the results of the fusion polypeptide complex of the present application releasing CTLA4-mediated immunosuppression. The fusion polypeptide complex of the present application may have the ability to relieve T cell immunosuppression.

### Example 5

### Antigen Presenting Cell Up-regulated Expression of Costimulatory Molecules

In a specific embodiment, the present invention discloses AN_Bif_Abs_0016, and/or LAG3-Ig, and/or AN_Bif_Abs_0019, and/or AN_Bif_Abs_0025, or LAG3-Ig (Aero, LA3-H5255), and/or AN_Bif_Abs_0036, and/or AN_Bif_Abs_0037, and/or AN_Bif_Abs_0038 induces the expression of co-stimulatory molecules in antigen-presenting cells.

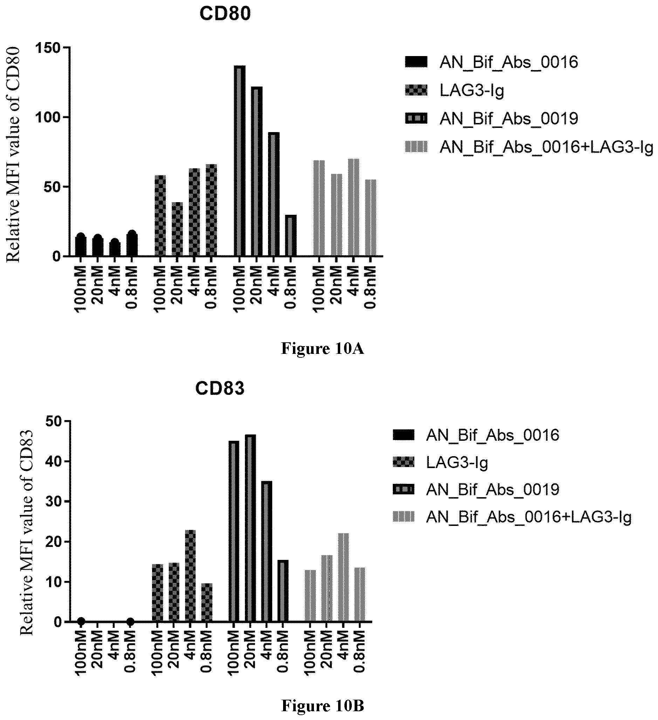

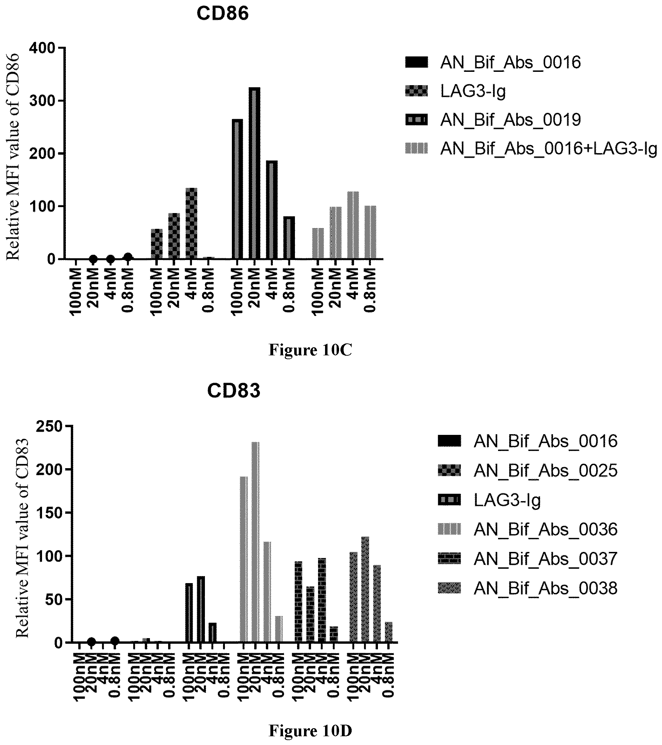

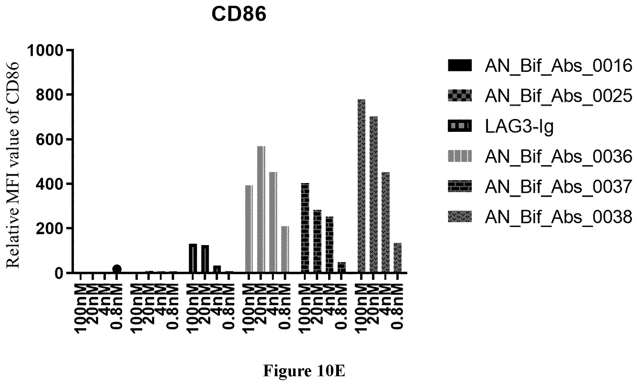

Specifically, collect THP-1 cells (ATCC), count and plate: add fresh medium to mix the cells and adjust the cell density to 1 * 10⁵cells/ml, and plate 100ul of cells per well. 100 ul fresh medium containing the corresponding concentration test substance (serially diluted) AN_BIF_ABS_0016, and/or an_bif_abs_0025, and/or an_bif_abs_0019, and the control Lag3-Ig, and/or an_bif_abs_0036, and/or an_bif_abs_ 0037, and/or an_bif_abs_0038 was added to each well. After culturing for 3 days, the cells were collected, and the levels of co-stimulatory molecules CD80, CD83 and CD86 expressed on the surface of THP-1 cells were detected by flow cytometry, and the specific operation was performed according to the instruction manual of flow cytometry. Figures 10A-10E show the results of the fusion polypeptide complex of the present application regulating the up-regulation of costimulatory molecule expression in antigen-presenting cells. The fusion polypeptide complex of the present application can have the ability to induce the expression of costimulatory molecules in antigen-presenting cells.

### Example 6

### Antigen Presenting Cell Maturation Morphological Change Assay

In a specific embodiment, the present invention discloses AN_Bif_Abs_0016, and/or AN_Bif_Abs_0017, and/or AN_Bif_Abs_0019, and/or LAG3-Ig (Aero, LA3-H5255), and/or AN_Bif_Abs_0036, and/or AN_Bif_Abs_0037, and/or AN_Bif_Abs_0038 induces antigen-presenting cells to differentiate and mature to undergo morphological changes.

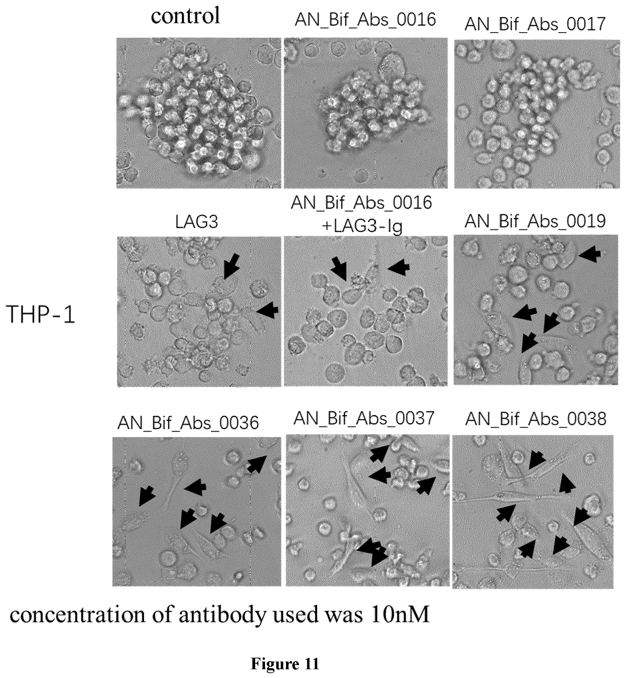

Specifically, collect THP-1 cells (ATCC), count and plate: add fresh medium to mix the cells and adjust the cell density to 1*10⁵cells/ml, and plate 100ul of cells per well. 100 ul fresh medium containing 10nM test substance AN_Bif_ Abs_0016, and/or AN_Bif_Ab s_0019 and cotronl LAG3-Ig, and/or AN_Bif_Abs_0037, and/or AN_Bif_Abs_0038 was added to each well. After culturing for 3 days, the cells were collected and the changes in THP-1 cell morphology were detected using an inverted microscope (Leica). The specific operation was performed according to the instruction manual of the inverted microscope. Figure 11 shows the results of the fusion polypeptide complex of the present application regulating the morphology and differentiation of antigen-presenting cells. The fusion polypeptide complex of the present application may have the ability to induce differentiation and maturation of antigen-presenting cells.

### Example 7

### Antigen Presenting Cell Secreted Chemokine Assay

In a specific embodiment, the present invention discloses AN_Bif_Abs_0016, and/or AN_Bif_Abs_0017, and/or AN_Bif_Abs_0019, and/or AN_Bif_Abs_0025, and/or LAG3-Ig (Aero, LA3-H5255), and/or AN_Bif_Abs_0036, and/or AN_Bif_Abs_0037, and/or AN_Bif_Abs_0038, and/or AN_Bif_Abs_0001, and/or AN_Bif_Abs_0013, and/or AN_Bif_Abs_0031, and/or **AN_Bif_Abs_0079,** and/or AN_Bif_Abs_0078, and/or **AN_Bif_Abs_00 79,** and/or AN_Bif_Abs_0092, and/or AN_Bif_Abs_0094 induce antigen presentation cells secrete the chemokine CCL4.

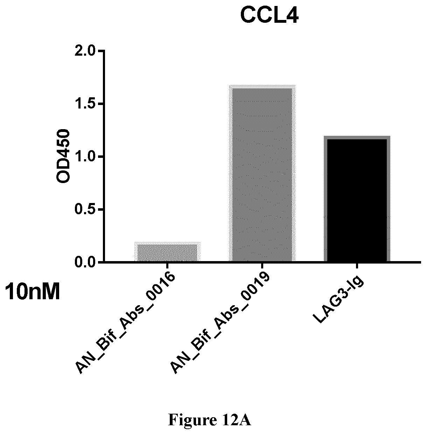

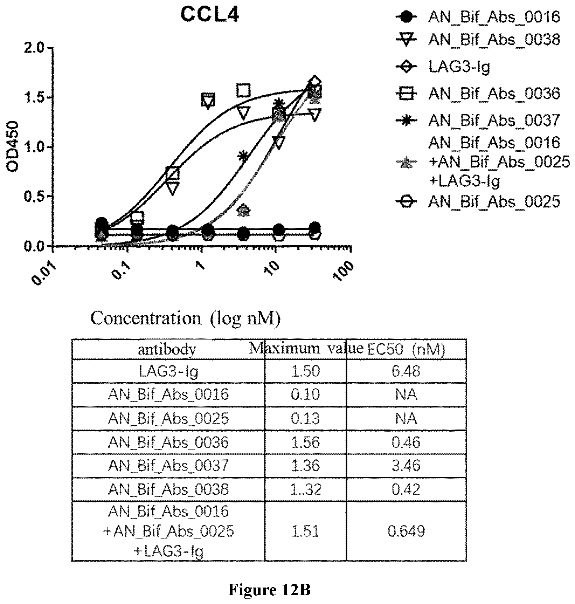

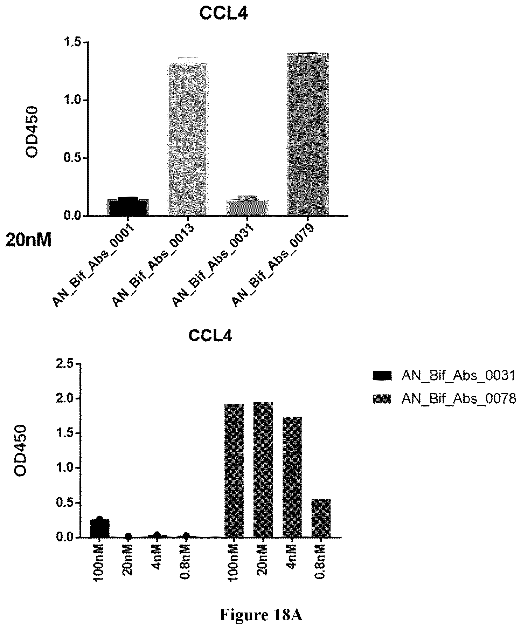

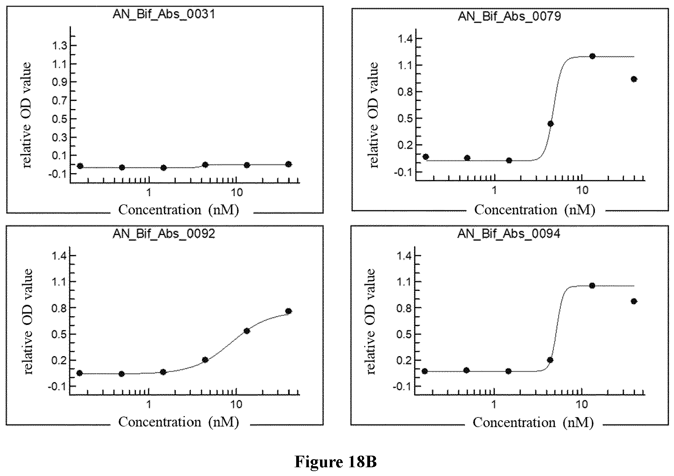

Specifically, collect THP-1 cells (ATCC), count and plate: add fresh medium to mix the cells and adjust the cell density to 1 * 10⁶cells/ml, and plate 100ul of cells per well. 100 ul fresh medium containing the corresponding concentration test substance AN_Bif_ Abs_0016, and/or AN_Bif_Abs_0025, and/or AN_Bif_Abs_0019 and control LAG3-Ig, and/or AN_Bif_Abs_0036, and/or AN_Bif_Abs_0037, and/or AN_Bif_Abs_003 8 was added to each well. The cell culture supernatant was collected after 6 hours, and the CCL4 expression level was detected using a CCL4 detection kit (PROTEINTECH). The specific operation was performed according to the manufacturer's instructions.) Figures 12A-12B and Figures 18A-18B show the results of the fusion polypeptide complex of the present application regulating antigen presentation and secretion of CCL4. The fusion polypeptide complex of the present application may have the ability to induce antigen-presenting cells to secrete the chemokine CCL4.

### Example 8

### Phagocytosis of E.coli by Antigen Presenting Cells

In a specific embodiment, the present invention discloses that AN_Bif_ Abs_0016, and/or AN_Bif_Abs_0025, and/or AN_Bif_Abs_0019, or LAG3-Ig (Aero, LA3-H5255), and/or AN_Bif_Abs_0036, and/or AN_Bif_Abs_0037, and/or AN_Bif_Abs_00 38 induce phagocytosis of E.coli by antigen-presenting cells.

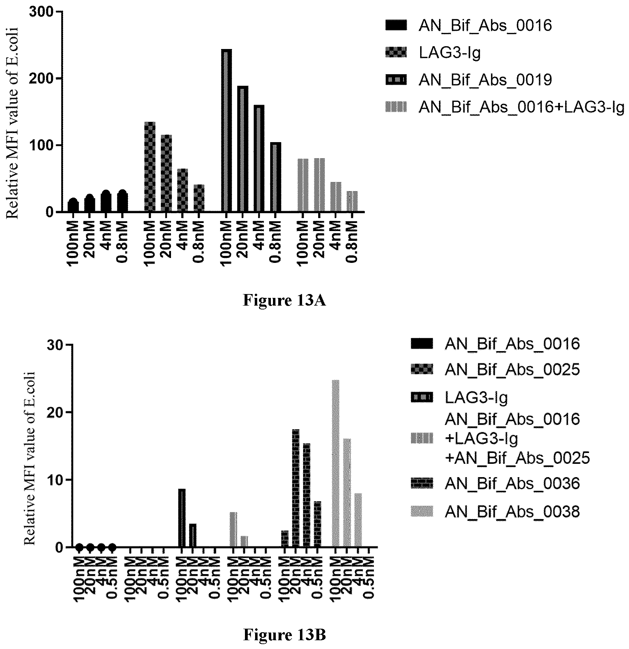

Specifically, collect THP-1 cells (ATCC), count and plate; add fresh medium to mix the cells and adjust the cell density to 1 * 10⁶cells/ml, and plate 100ul of cells per well. 100 ul fresh medium containing the corresponding concentration test substance AN_Bif_ Abs_0016, and/or AN_Bif_Abs_0025, and/or AN_Bif_Abs_0019 and control LAG3-Ig, and/or AN_Bif_Abs_0036, and/or AN_Bif_Abs_0037, and/or AN_Bif_ Abs_0038 was added to each well. After 3 days of culture, the cells were harvested, and an appropriate amount of pHrodo^{™} Deep Red E. coli BioParticles^{™} conjugate (Invitrogen) for detecting phagocytosis was added to the cells according to the manufacturer's instructions. Figures 13A-13B show the results of the fusion polypeptide complex of the present application regulating the phagocytosis of antigen-presenting cells E.coli, and the fusion polypeptide complex of the present application may have the ability to induce phagocytosis of antigen-presenting cells.

### Example 9

### Activation of Primary T cell

In a specific embodiment, the present invention discloses that AN_Bif_ Abs_0016, and/or AN_Bif_Abs_0017, and/or AN_Bif_Abs_0019, and/or_Bif_ Abs_0025, orLAG3-Ig (Aero, LA3-H5255), and/or AN_Bif_Abs_0036, and/or AN_Bif_Abs_00 37, and/or AN_Bif_Abs_0038 induce activation of human T cells.

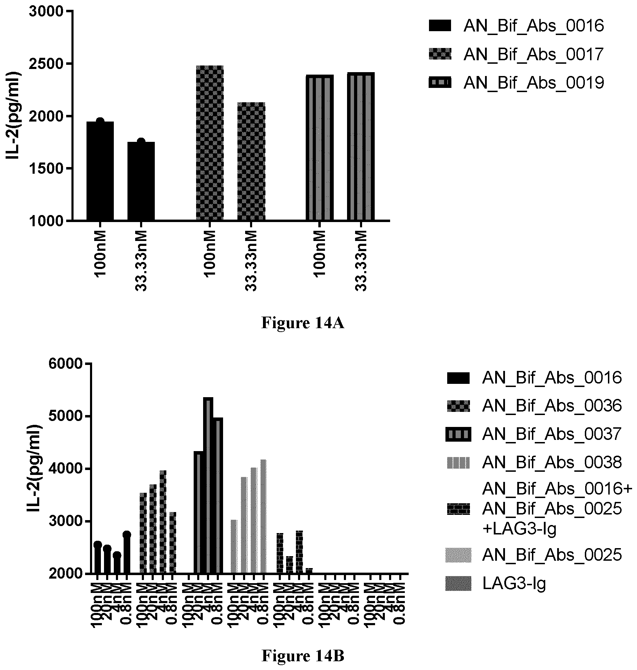

Specifically, collect purified T cells from human peripheral mononuclear cells, count and plate; add fresh medium to mix the cells and adjust the cell density to 2*10⁶cells/ml, mix with 2*10⁵cells/ml and plate 100ul cells in each well. 100 ul fresh medium containing the corresponding concentration test substance AN_Bif_Abs_0016, and/or AN_Bif_Abs_0025, and/or AN_Bif_Abs_0019, and/or control LAG3-Ig, and/or AN_Bif_Abs_0037, and/or AN_Bif_Abs_0038 was added to each well. After incubatiing for 1 hour, 40ng/ml SEB (Toxin BT202) was added. After culturing for 3 days, the culture supernatant was collected and the IL-2 ELISA detection kit (R&D, DY202) was used to detect the secretion of IL-2 in the supernatant. The operation was carried out according to the manufacturer's instructions. Figures 14A-14B show the results of activation of human primary T cells by the fusion polypeptide complex of the present application. The fusion polypeptide complex of the present application may have the ability to induce T cell activation.

### Example 10

### Determination of antitumor activity

In a specific embodiment, the present invention discloses the anti-tumor activity of aPDL1, aPDL1-mCD80, or aPDL1-mLAG3.

Specifically, to study the effects of bifunctional fusion polypeptide complexes in mice, in view of that the amino acid sequence identity of human CD80 extracellular domain and mouse CD80 extracellular domain is about 50%, the amino acid sequence identity of human LAG3 extracellular domain and mouse LAG3 extracellular domain is about 70%, use mouse CD80 extracellular domain to replace human CD80 extracellular domain to contstruct a bifunctional fusion polypeptide complex AN_Bif_ Abs_0002 in which the aPD-L1 antibody is covalently linked to the mouse CD80 extracellular domain; shown below are heavy chain amino acid sequence (SEQ ID NO: 159) and light chain amino acid sequence (SEQ ID NO: 163) thereof; construct a control fusion polypeptide complex AN_Bif_Abs_0003 in which aSOJA antibody is covalently linked to the mouse CD80 extracellular domain; shown below are heavy chain amino acid sequence (SEQ ID NO: 160) and light chain amino acid sequence (SEQ ID NO: 127) thereof; use mouse LAG3 extracellular domain to replace human LAG3 extracellular domain to construct a bifunctional fusion polypeptide complex AN_Bif_Abs_0004 in which the aPD-L1 antibody is covalently linked to the mouse LAG3 extracellular domain; shown below are heavy chain amino acid sequence (SEQ ID NO: 161) and light chain amino acid sequence (SEQ ID NO: 163).

Specifically, the mouse colon cancer cell line MC38 (MC38/hPD-L1), a genetically engineered cell expressing human PD-L1 and the mouse breast cancer cell line EMT6 (EMT6/hPD), a genetically engineered cell expressing human PD-L1 were used. -L1) cells were used to detect and evaluate the anti-tumor activity of the bifunctional fusion polypeptide complex.

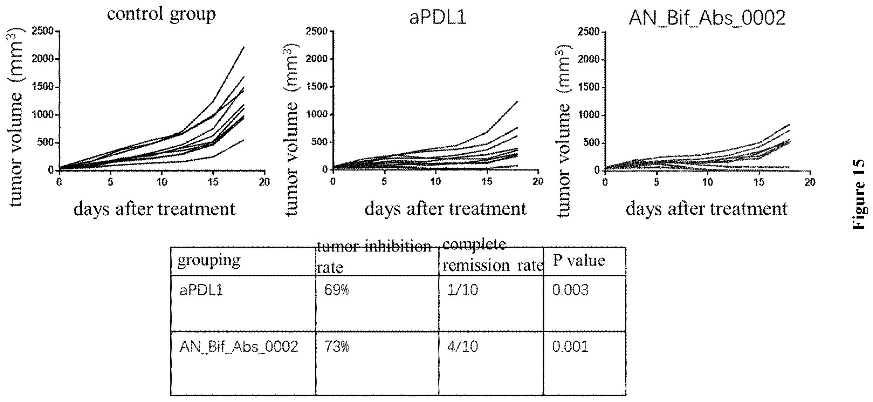

Specifically, on the first day, the MC38/ hPD-L1 cells were subcutaneously implanted into C57BL/6 mice, and the EMT6/ hPD-L1 cells were subcutaneously implanted into Balb/c mice, with eight mice in each group. When the average volume of the mouse subcutaneous tumor grows to 50mm3; the control article (PBS buffer), or the test article anti-aPD-L1 monoclonal antibody (1mg/kg), or AN_Bif_Abs_0002 (1.35mg/kg), intraperitoneal injection administration, administration The interval was twice a week, and the duration of dosing was two weeks; individual tumor volumes measured over time are illustrated in the graph; ip = intraperitoneal, sc = subcutaneous. Figure 15 shows the results of the tumor inhibition curve of the fusion polypeptide complex of the present application in the MC38/hPD-L1 tumor model.

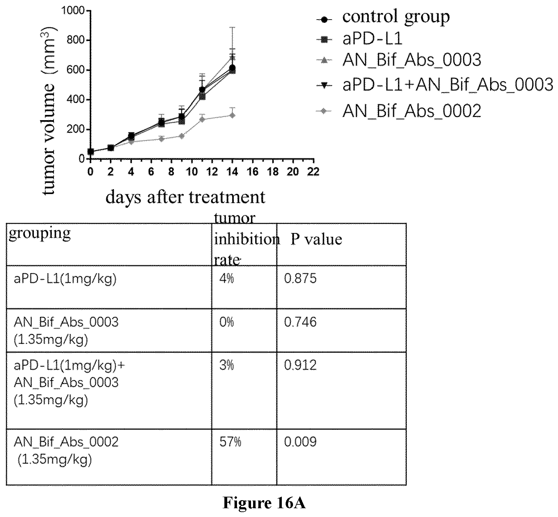

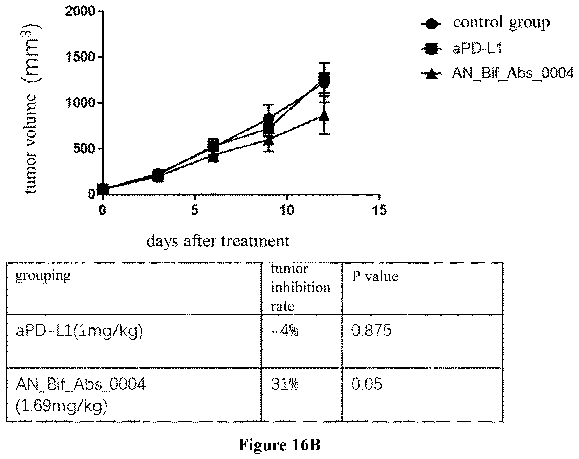

Similarly, EMT6/ hPD-L1 cells were subcutaneously implanted into Balb/c mice, eight mice per group, and when the average volume of subcutaneous tumors in the mice grew to 50mm³, the control article (PBS buffer), or the test article anti-aPD-L1 monoclonal antibody (1mg/kg), or AN_Bif_Abs_0002 (1.35mg/kg), or AN_Bif_Abs_0003 (1.35mg/kg), or aPD-L1 monoclonal antibody (1mg/kg) + AN_Bif_Abs_0003 (1.35mg/kg), or AN_Bif_Abs_0004 (1.69 mg/kg) was administered by intraperitoneal injection twice a week for two weeks; individual tumor volumes measured over time are illustrated in the figure; ip = intraperitoneal, sc = intraperitoneal subcutaneous. Figures 16A-16B show the results of the tumor inhibition curve of the fusion polypeptide complex of the present application in the EMT6/hPD-L1 tumor model. The fusion polypeptide complex of the present application may have anti-tumor activity.

The foregoing detailed description has been offered by way of explanation and example, not to limit the scope of the appended claims. Variations on the presently recited embodiments of this application will be apparent to those of ordinary skill in the art and remain within the scope of the appended claims and their equivalents.

## Claims

1. A fusion polypeptide, the fusion polypeptide comprises a first domain and a second domain, the first domain comprises HCDR3 of an antibody heavy chain, and the antibody heavy chain comprises the amino acid sequence shown in SEQ ID NO: 121, the second domain is capable of activating an immune response.

2. The fusion polypeptide according to claim 1, the first domain comprises HCDR2 of the heavy chain of the antibody, and the heavy chain of the antibody comprises the amino acid sequence shown in SEQ ID NO: 121.

3. The fusion polypeptide according to any one of claims 1-2, the first domain comprises HCDR1 of an antibody heavy chain, and the antibody heavy chain comprises the amino acid sequence shown in SEQ ID NO: 121.

4. The fusion polypeptide according to any one of claims 1-3, the first domain comprises the heavy chain variable region VH of an antibody heavy chain, and the antibody heavy chain comprises the amino acid sequence shown in SEQ ID NO: 121.

5. The fusion polypeptide according to any one of claims 1-4, the first domain comprises a heavy chain, and the heavy chain comprises the amino acid sequence shown in SEQ ID NO:121.

6. The fusion polypeptide according to any one of claims 1-5, the first domain comprises LCDR3 of an antibody light chain, and the antibody light chain comprises the amino acid sequence shown in SEQ ID NO: 122.

7. The fusion polypeptide according to any one of claims 1-6, the first domain comprises LCDR2 of an antibody light chain, and the antibody light chain comprises the amino acid sequence shown in SEQ ID NO: 122.

8. The fusion polypeptide according to any one of claims 1-7, the first domain comprises LCDR1 of an antibody light chain, and the antibody light chain comprises the amino acid sequence shown in SEQ ID NO: 122.

9. The fusion polypeptide according to any one of claims 1-8, the first domain comprises the light chain variable region VL of the antibody light chain, and the antibody light chain comprises the amino acid sequence shown in SEQ ID NO: 122.

10. The fusion polypeptide according to any one of claims 1-9, the first domain comprises a light chain comprising the amino acid sequence shown in SEQ ID NO:122.

11. The fusion polypeptide according to any one of claims 1-10, the first domain comprises an antibody or an antigen-binding fragment thereof.

12. The fusion polypeptide according to claim 11, said antibody is selected from the group consisting of recombinant antibody, single domain antibody, heavy chain antibody, chimeric antibody and bispecific antibody.

13. The fusion polypeptide according to any one of claims 11-12, the antigen-binding fragment is selected from one or more of the group consisting of: Fab, Fab', Fv fragment, F(ab')₂, F(ab)₂, scFv, di-scFv, VHH and dAb.

14. The fusion polypeptide according to any one of claims 1-13, the second domain is selected from the group consisting of CD80 or a functionally active fragment thereof, and LAG3 or a functionally active fragment thereof.

15. The fusion polypeptide according to any one of claims 1-14, the second domain is selected from the group consisting of human-derived CD80 or its functionally active fragment, mouse-derived CD80 or its functionally active fragment, human-derived LAG3 or its functionally active fragments and mouse-derived LAG3 or its functionally active fragments.

16. The fusion polypeptide according to any one of claims 1-15, wherein the second domain is capable of **binding** to CD28.

17. The fusion polypeptide according to any one of claims 1-16, wherein the second domain comprises an extracellular domain of CD80 or a functionally active fragment thereof.

18. The fusion polypeptide according to any one of claims 1-17, wherein the second domain comprises the amino acid sequence shown in SEQ ID NO:2.

19. The fusion polypeptide according to any one of claims 1-18, wherein the second domain is capable of binding to MHCII molecules on antigen-presenting cells.

20. The fusion polypeptide according to any one of claims 1-19, wherein the second domain comprises an extracellular domain of LAG3 or a functionally active fragment thereof.

21. The fusion polypeptide according to any one of claims 1-20, wherein the second domain comprises the amino acid sequence shown in SEQ ID NO:11.

22. The fusion polypeptide according to any one of claims 1-21, wherein the second domain comprises IgD1, IgD2, IgD3 and/or IgD4 of LAG3 or a functionally active fragment thereof.

23. The fusion polypeptide according to any one of claims 1-22, wherein the second domain comprises an amino acid sequence selected from the group consisting of: SEQ ID NO: 13, SEQ ID NO: 14, SEQ ID NO: 15, SEQ ID NO:16, SEQ ID NO:17, SEQ ID NO:18, SEQ ID NO:19, and SEQ ID NO:12.

24. The fusion polypeptide according to any one of claims 1-23, wherein the first domain is directly or indirectly linked to the second domain.

25. The fusion polypeptide according to any one of claims 1-24, wherein the first domain comprises an antibody heavy chain, and the antibody heavy chain of the first domain is directly or indirectly linked to the second domain.

26. The fusion polypeptide according to any one of claims 1-25, wherein the first domain comprises an antibody light chain, and the antibody light chain of the first domain is directly or indirectly linked to the second domain.

27. The fusion polypeptide according to any one of claims 1-26, wherein the first domain is directly or indirectly linked to the N-terminus of the second domain.

28. The fusion polypeptide according to any one of claims 1-27, wherein the first domain is directly or indirectly connected to the C-terminus of the second domain.

29. The fusion polypeptide according to any one of claims 24-28, wherein the indirect link comprises link via a linker.

30. The fusion polypeptide according to claim 29, wherein the linker comprises an amino acid sequence selected from the group consisting of: SEQ ID NO: 22, SEQ ID NO: 23, SEQ ID NO: 24, SEQ ID NO: 25, and SEQ ID NO: 26.

31. The fusion polypeptide according to any one of claims 1-30, wherein the fusion polypeptide comprising an amino acid sequence selected from the group consisting of:
SEQ ID NO: 27-42, SEQ ID NO: 130, SEQ ID NO: 132, SEQ ID NO: 135, SEQ ID NO: 137, SEQ ID NO: 139, SEQ ID NO: 147, SEQ ID NO: 149, SEQ ID NO: 153, SEQ ID NO: 155, SEQ ID NO: 159, SEQ ID NO: 161, SEQ ID NO: 168, SEQ ID NO: 170, SEQ ID NO: 172, SEQ ID NO: 174, and SEQ ID NO: 176.

32. A fusion polypeptide, wherein the fusion polypeptide comprises a first domain and a second domain, the first domain is capable of blocking PD-1/PD-L1 signaling, and the second domain comprises LAG3 or its functional activity fragment.

33. The fusion polypeptide according to claim 32, wherein the first domain comprises HCDR3 of an antibody heavy chain, and the antibody heavy chain comprises an amino acid sequence selected from the following groups: SEQ ID NO: 3-9, and SEQ ID NO :121.

34. The fusion polypeptide according to any one of claims 32-33, wherein the first domain comprises HCDR2 of an antibody heavy chain, and the antibody heavy chain comprises an amino acid sequence selected from the following group: SEQ ID NO: 3-9, and SEQ ID NO:121.

35. The fusion polypeptide according to any one of claims 32-34, wherein the first domain comprises HCDR1 of an antibody heavy chain, and the antibody heavy chain comprises an amino acid sequence selected from the following group: SEQ ID NO: 3-9, and SEQ ID NO:121.

36. The fusion polypeptide according to any one of claims 32-35, wherein the first domain comprises the heavy chain variable region VH of an antibody heavy chain, and the antibody heavy chain comprises an amino acid sequence selected from the following group: SEQ ID NO:3-9, and SEQ ID NO:121.

37. The fusion polypeptide according to any one of claims 32-36, wherein the first domain comprises an antibody heavy chain, and the antibody heavy chain comprises an amino acid sequence selected from the following groups: SEQ ID NO: 3-9, and SEQ ID NO:121.

38. The fusion polypeptide according to any one of claims 32-37, wherein the first domain comprises LCDR3 of an antibody light chain, and the antibody light chain comprises an amino acid sequence selected from the group consisting of: SEQ ID NO: 162- 167, and SEQ ID NO:122.

39. The fusion polypeptide according to any one of claims 32-38, wherein the first domain comprises LCDR2 of an antibody light chain, and the antibody light chain comprises an amino acid sequence selected from the group consisting of: SEQ ID NO: 162- 167, and SEQ ID NO:122.

40. The fusion polypeptide according to any one of claims 32-39, wherein the first domain comprises LCDR1 of an antibody light chain, and the antibody light chain comprises an amino acid sequence selected from the group consisting of: SEQ ID NO: 162- 167, and SEQ ID NO: 122.

41. The fusion polypeptide according to any one of claims 32-40, wherein the first domain comprises the light chain variable region VL of an antibody light chain, and the antibody light chain comprises an amino acid sequence selected from the group consisting of: SEQ ID NO:162-167, and SEQ ID NO: 122.

42. The fusion polypeptide according to any one of claims 32-41, wherein the first domain comprises an antibody light chain, and the antibody light chain comprises an amino acid sequence selected from the group consisting of: SEQ ID NO: 162-167, and SEQ ID NO: 122.

43. The fusion polypeptide according to any one of claims 32-42, wherein the first binding domain is capable of **binding to** PD-L1 and/or PD-1.

44. The fusion polypeptide according to any one of claims 32-43, wherein the first domain comprises an antibody or an antigen-binding fragment thereof.

45. The fusion polypeptide according to claim 44, wherein the antibody is selected from the group consisting of recombinant antibody, single domain antibody, heavy chain antibody, chimeric antibody and bispecific antibody.

46. The fusion polypeptide according to any one of claims 44-45, wherein the antigen-binding fragment is selected from one or more of the group consisting of: Fab, Fab', Fv fragment, F(ab')₂, F(ab)₂, scFv, di-scFv, VHH and dAb.

47. The fusion polypeptide according to any one of claims 32-46, wherein the second domain is selected from the group consisting of human-derived LAG3 or a functionally active fragment thereof and mouse-derived LAG3 or a functionally active fragment thereof.

48. The fusion polypeptide according to any one of claims 32-47, wherein the second domain is capable of binding to MHCII molecules on antigen-presenting cells.

49. The fusion polypeptide according to any one of claims 32-48, wherein the second domain comprises an extracellular domain of LAG3 or a functionally active fragment thereof.

50. The fusion polypeptide according to any one of claims 32-49, wherein the second domain comprises the amino acid sequence shown in SEQ ID NO: 11.

51. The fusion polypeptide according to any one of claims 32-50, wherein the second domain comprises IgD1, IgD2, IgD3 and/or IgD4 of LAG3 or a functionally active fragment thereof.

52. The fusion polypeptide according to any one of claims 32-51, wherein the second domain comprises an amino acid sequence selected from the group consisting of: SEQ ID NO: 13, SEQ ID NO: 14, SEQ ID NO: 15, SEQ ID NO: 16, SEQ ID NO: 17, SEQ ID NO:18, SEQ ID NO: 19, and SEQ ID NO: 12.

53. The fusion polypeptide according to any one of claims 32-52, wherein the first domain is directly or indirectly linked to the second domain.

54. The fusion polypeptide according to any one of claims 32-53, wherein the first domain comprises an antibody heavy chain, and the antibody heavy chain of the first domain is directly or indirectly linked to the second domain.

55. The fusion polypeptide according to any one of claims 32-54, wherein the first domain comprises an antibody light chain, and the antibody light chain of the first domain is directly or indirectly linked to the second domain.

56. The fusion polypeptide according to any one of claims 32-55, wherein the first domain is directly or indirectly linked to the N-terminus of the second domain.

57. The fusion polypeptide according to any one of claims 32-56, wherein the first domain is directly or indirectly linked to the C-terminus of the second domain.

58. The fusion polypeptide according to any one of claims 53-57, wherein the indirect link comprises link via a linker.

59. The fusion polypeptide according to claim 58, wherein the linker comprises an amino acid sequence selected from the group consisting of: SEQ ID NO: 22, SEQ ID NO: 23, SEQ ID NO: 24, SEQ ID NO: 25, and SEQ ID NO: 26.

60. The fusion polypeptide according to any one of claims 32-59, wherein said fusion polypeptide comprises an amino acid sequence selected from the group consisting of: SEQ ID NO: 28-30, SEQ ID NO: 32, SEQ ID NO: 34- 42, SEQ ID NO: 44-46, SEQ ID NO: 48, SEQ ID NO: 50-58, SEQ ID NO: 60-62, SEQ ID NO: 64, SEQ ID NO: 66-74, SEQ ID NO: 76-78, SEQ ID NO: 80, SEQ ID NO: 82-90, SEQ ID NO: 92-94, SEQ ID NO: 96-104, SEQ ID NO: 106-108, SEQ ID NO: 110, SEQ ID NO: 112-120, SEQ ID NO: 135, SEQ ID NO: 137, SEQ ID NO: 139, SEQ ID NO: 147, SEQ ID NO: 149, SEQ ID NO: 153, SEQ ID NO: 155, SEQ ID NO: 161, SEQ ID NO: 168, SEQ ID NO: 170, SEQ ID NO: 172, SEQ ID NO: 174, and SEQ ID NO: 176.

61. A fusion polypeptide, the fusion polypeptide comprises a first domain, a second domain and a third domain, wherein the first domain can block PD-1/PD-L1 signaling, the second domain comprises LAG3 or a functionally active fragment thereof, and the third domain is capable of activating an immune response.

62. The fusion polypeptide according to claim 61, wherein the first domain comprises HCDR3 of an antibody heavy chain, and the antibody heavy chain comprises an amino acid sequence selected from the following groups: SEQ ID NO: 3-9, and SEQ ID NO :121.

63. The fusion polypeptide according to any one of claims 61-62, wherein the first domain comprises HCDR2 of an antibody heavy chain, and the antibody heavy chain comprises an amino acid sequence selected from the following group: SEQ ID NO: 3-9, and SEQ ID NO:121.

64. The fusion polypeptide according to any one of claims 61-63, wherein the first domain comprises HCDR1 of an antibody heavy chain, and the antibody heavy chain comprises an amino acid sequence selected from the following group: SEQ ID NO: 3-9, and SEQ ID NO:121.

65. The fusion polypeptide according to any one of claims 61-64, wherein the first domain comprises the heavy chain variable region VH of an antibody heavy chain, and the antibody heavy chain comprises an amino acid sequence selected from the following group: SEQ ID NO:3-9, and SEQ ID NO:121.

66. The fusion polypeptide according to any one of claims 61-65, wherein the first domain comprises an antibody heavy chain, and the antibody heavy chain comprises an amino acid sequence selected from the following groups: SEQ ID NO: 3-9, and SEQ ID NO:121.

67. The fusion polypeptide according to any one of claims 61-66, wherein wherein the first domain comprises LCDR3 of an antibody light chain, and the antibody light chain comprises an amino acid sequence selected from the group consisting of: SEQ ID NO: 162- 167, and SEQ ID NO: 122.

68. The fusion polypeptide according to any one of claims 61-67, wherein the first domain comprises LCDR2 of an **antibody light chain,** and the antibody light chain comprises an amino acid sequence selected from the group consisting of:: SEQ ID NO: 162- 167, and SEQ ID NO: 122.

69. The fusion polypeptide according to any one of claims 61-68, wherein the first domain comprises LCDR1 of an antibody light chain, and the antibody light chain comprises an amino acid sequence selected from the group consisting of: SEQ ID NO: 162- 167, and SEQ ID NO: 122.

70. The fusion polypeptide according to any one of claims 61-69, wherein the first domain comprises the light chain variable region VL of an antibody light chain, and the antibody light chain comprises an amino acid sequence selected from the group consisting of: SEQ ID NO:162-167, and SEQ ID NO: 122.

71. The fusion polypeptide according to any one of claims 61-70, wherein the first domain comprises an antibody light chain, and the antibody light chain comprises an amino acid sequence selected from the group consisting of: SEQ ID NO: 162-167, and SEQ ID NO: 122.

72. The fusion polypeptide according to any one of claims 61-71, wherein the first domain is capable of binding PD-L1 and/or PD-1.

73. The fusion polypeptide according to any one of claims 61-72, wherein the first domain comprises an antibody or an antigen-binding fragment thereof.

74. The fusion polypeptide according to claim 73, wherein said antibody is selected from the group consisting of recombinant antibody, single domain antibody, heavy chain antibody, chimeric antibody and bispecific antibody.

75. The fusion polypeptide according to any one of claims 73-74, wherein the antigen-binding fragment is selected from one or more of the group consisting of: Fab, Fab', Fv fragment, F(ab')₂, F(ab)₂, scFv, di-scFv, VHH and dAb.

76. The fusion polypeptide according to any one of claims 61-75, wherein the second domain is selected from the group consisting of human-derived LAG3 or a functionally active fragment thereof and mouse-derived LAG3 or a functionally active fragment thereof.

77. The fusion polypeptide according to any one of claims 61-76, wherein the second domain is capable of binding to MHCII molecules on antigen-presenting cells.

78. The fusion polypeptide according to any one of claims 61-77, wherein the second domain comprises an extracellular domain of LAG3 or a functionally active fragment thereof.

79. The fusion polypeptide according to any one of claims 61-78, the second domain comprises the amino acid sequence shown in SEQ ID NO: 11.

80. The fusion polypeptide according to any one of claims 61-79, wherein the second domain comprises IgD1, IgD2, IgD3 and/or IgD4 of LAG3 or a functionally active fragment thereof.

81. The fusion polypeptide according to any one of claims 61-80, said second domain comprises an amino acid sequence selected from the group consisting of: SEQ ID NO: 13, SEQ ID NO: 14, SEQ ID NO: 15, SEQ ID NO: 16, SEQ ID NO: 17, SEQ ID NO: 18, SEQ ID NO: 19, and SEQ ID NO: 12.

82. The fusion polypeptide according to any one of claims 61-81, wherein the third domain comprises CD80 or a functionally active fragment thereof.

83. The fusion polypeptide according to any one of claims 61-82, wherein the third domain is selected from the group consisting of human-derived CD80 or its functionally active fragment and mouse-derived CD80 or its functionally active fragment.

84. The fusion polypeptide according to any one of claims 61-83, wherein the third domain is capable of binding to CD28.

85. The fusion polypeptide according to any one of claims 61-84, wherein the third domain comprises an extracellular domain of CD80 or a functionally active fragment thereof.

86. The fusion polypeptide according to any one of claims 61-85, wherein the third domain comprises the amino acid sequence shown in SEQ ID NO:2.

87. The fusion polypeptide according to any one of claims 61-86, wherein the first domain is directly or indirectly linked to the second domain.

88. The fusion polypeptide according to any one of claims 61-87, wherein the first domain comprises an antibody heavy chain, and the antibody heavy chain of the first domain is directly or indirectly linked to the second domain.

89. The fusion polypeptide according to any one of claims 61-88, wherein the first domain comprises an antibody light chain, and the antibody light chain of the first domain is directly or indirectly linked to the second domain.

90. The fusion polypeptide according to any one of claims 61-89, wherein the first domain is directly or indirectly linked to the third domain.

91. The fusion polypeptide according to any one of claims 61-90, wherein the first domain comprises an antibody heavy chain, and the antibody heavy chain of the first domain is directly or indirectly linked to the third domain.

92. The fusion polypeptide according to any one of claims 61-91, wherein the first domain comprises an antibody light chain, and the antibody light chain of the first domain is directly or indirectly linked to the third domain.

93. The fusion polypeptide according to any one of claims 61-92, wherein the second domain is directly or indirectly linked to the third domain.

94. The fusion polypeptide according to any one of claims 61-93, wherein the first domain is directly or indirectly linked to the N-terminus of the second domain.

95. The fusion polypeptide according to any one of claims 61-94, wherein the first domain is directly or indirectly linked to the C-terminus of the second domain.

96. The fusion polypeptide according to any one of claims 61-95, wherein the first domain is directly or indirectly linked to the N-terminus of the third domain.

97. The fusion polypeptide according to any one of claims 61-96, wherein the first domain is directly or indirectly linked to the C-terminus of the third domain.

98. The fusion polypeptide according to any one of claims 61-97, wherein the second domain is directly or indirectly linked to the N-terminus of the third domain.

99. The fusion polypeptide according to any one of claims 61-98, wherein the second domain is directly or indirectly linked to the C-terminus of the third domain.

100. The fusion polypeptide according to any one of claims 61-99, wherein the first domain is directly or indirectly linked to the N-terminus of the second domain, and the second domain is directly or indirectly linked to the N-terminal of the third domain.

101. The fusion polypeptide according to any one of claims 61-100, wherein the first domain is directly or indirectly linked to the N-terminus of the third domain, and the third domain is directly or indirectly linked to the N-terminal of the second domain.

102. The fusion polypeptide according to any one of claims 61-101, wherein the first domain is directly or indirectly connected to the C-terminus of the second domain, and the first domain is directly or indirectly linked to the N-terminus of the third domain.

103. The fusion polypeptide according to any one of claims 61-102, wherein the first domain is directly or indirectly linked to the C-terminus of the third domain, and the first domain is directly or indirectly linked to the N-terminus of the second domain.

104. The fusion polypeptide according to any one of claims 87-103, wherein the indirect link comprises link via a linker.

105. The fusion polypeptide according to claim 104, wherein the linker comprises an amino acid sequence selected from the group consisting of: SEQ ID NO: 22, SEQ ID NO: 23, SEQ ID NO: 24, SEQ ID NO: 25, and SEQ ID NO: 26.

106. The fusion polypeptide according to any one of claims 61-105, wherein said fusion polypeptide comprising an amino acid sequence selected from the following group consisting of: SEQ ID NO: 35-42, SEQ ID NO: 51-58, SEQ ID NO: 67-74, SEQ ID NO: 83-90, SEQ ID NO: 97-104, SEQ ID NO: 113-120, SEQ ID NO: 147, SEQ ID NO: 149, SEQ ID NO: 153, SEQ ID NO: 155, SEQ ID NO:168, SEQ ID NO:170, SEQ ID NO:172, SEQ ID NO:174, and SEQ ID NO:176.

107. An immunoconjugate comprising the fusion polypeptide of any one of claims 1-106.

108. A nucleic acid molecule encoding the fusion polypeptide of any one of claims 1-106.

109. A vector comprising the nucleic acid molecule of claim 108.

110. A cell comprising and/or expressing the fusion polypeptide of any one of claims 1-106, the immunoconjugate of claim 107, the nucleic acid molecule of claim 108, and/or the vector of claim 109.

111. A composition comprising the fusion polypeptide of any one of claims 1-106, the immunoconjugate of claim 107, the nucleic acid molecule of claim 108, and the vector of claim 109, and/or the cell of claim 110, and optionally a pharmaceutically acceptable carrier.

112. A method of preparing the fusion polypeptide according to any one of claims 1-106, comprising culturing the cell according to claim 110 under conditions enabling expression of the fusion polypeptide.

113. A method for blocking the interaction between PD-L1 protein and PD-1, which comprises administering an effective amount of the fusion polypeptideof any one of claims 1-106, the immunoconjugate of claim 107, and the nucleic acid molecule of claim 108, the vector of claim 109, the cell of claim 110, and/or the composition of claim 111.

114. A method for stimulating antigen-presenting cells and/or activating the action of T cells, comprising administering an effective amount of the fusion polypeptide of any one of claims 1-106, the immunoconjugate of claim 107, The nucleic acid molecule of claim 108, the vector of claim 109, the cell of claim 110, and/or the composition of claim 111.

115. The method according to claim 114, wherein the stimulating antigen-presenting cells is selected from the following group: increasing the expression of costimulatory molecules in the antigen-presenting cells, causing the morphological changes and maturation of the antigen-presenting cells, increasing secretion of the chemokines of the antigen-presenting cells, and enhancing phagocytosis of antigen-presenting cells.

116. A method of inhibiting the growth and/or proliferation of tumors or tumor cells, comprising administering an effective amount of the fusion polypeptide of any one of claims 1-106, the immunoconjugate of claim 107, the nucleic acid molecule of claim 108, the vector of claim 109, the cell of claim 110, and/or the composition of claim 111.

117. Use of the fusion polypeptide of any one of claims 1-106, the immunoconjugate of claim 107, the nucleic acid molecule of claim 108, the vector of claim 109, the cell of claim 110, and/or the composition of claim 111 in the preparation of a medicament, wherein the medicament is used to prevent, improve and/or treat tumors.

118. The use according to claim 117, wherein the tumor comprises solid tumors and hematological tumors.

119. The use according to any one of claims 117-118, wherein the tumor is selected from the group consisting of colon tumors, breast tumors, lung tumors, stomach tumors, melanoma, head and neck tumors, lymphoma, nasopharyngeal tumors, cervical tumors tumors, esophageal tumors, kidney tumors, skin squamous cell carcinoma, endometrial tumors, liver tumors, bladder tumors, urothelial tumors and skin tumors.
